# EUROPEAN PATENT APPLICATION

(11) **EP 1 967 513 A1**
(43) Date of publication of application: **10.09.2008**
(21) Application number: 07103803.8
(22) Date of filing: 08.03.2007
(51) Int. Cl.: C07C 309/46, C07C 309/53, C07C 323/37, C07C 229/74, C07D 213/74, C07D 239/42, C07D 241/20, A61K 31/136, A61P 7/02, A61P 9/10

(54) **Novel P2Y12 receptor antagonists**

(71) Applicant: Rheinische Friedrich-Wilhelms-Universität Bonn, 53113 Bonn (DE)
(72) Inventor: Müller, Christa E., Prof. Dr., 53113 Bonn (DE); Baqi, Younis, 53115 Bonn (DE)
(74) Representative: Viering, Jentschura & Partner

(57) **Abstract**

The present invention relates to compounds of Formula I, in which A and B are independently CH₂, O, S, NH, C=O, C=NH, C=S or C=N-OH; X is NH, O, S, C=O or CH₂ and R₁-R₅ are as defined in claim 1, which are P2Y₁₂ receptor antagonists and useful for treating, alleviating and/or preventing diseases and disorders related to P2Y₁₂ receptor function as well as pharmaceutical compositions comprising such compounds and methods for preparing such compounds. The present invention is further directed to the use of these compounds, alone or in combination with other therapeutic agents, for the alleviation, prevention and/or treatment of diseases and disorders, especially the use as antithrombotic agents for inhibiting platelet aggregation.

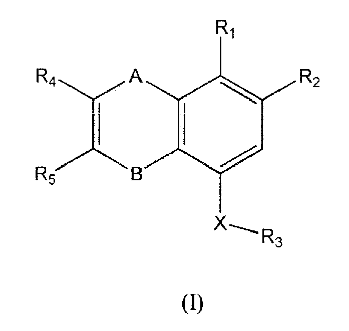

## Description

### Field of the Invention

The present invention lies on the field of pharmacology, drug design and organic chemistry and relates to novel P2Y₁₂ receptor antagonists useful for treating, alleviating and/or preventing diseases and disorders related to P2Y₁₂ receptor function as well as pharmaceutical compositions comprising such compounds and methods for preparing such compounds. The present invention is further directed to the use of these compounds, alone or in combination with other therapeutic agents, for the alleviation, prevention and/or treatment of diseases and disorders, especially the use as antithrombotic agents for inhibiting platelet aggregation.

### Background of the Invention

The P2 receptors are a major class of receptors in the human body (Guile, S. D. et al., Prog. Med. Chem., 2001, 38, 115; Lambrecht, G. et al., Curr. Pharm. Des, 2002, 8, 2371**;** Gao, Z. G., Jacobson, K. A. Curr. Top. Med. Chem., 2004, 4, 855; Burnstock, G. Curr. Top. Med. Chem., 2004, 4, 793-803). They are subdivided into two main groups: G protein-coupled or "metabotropic" P2 receptors, designated P2Y, and ligand-gated ion channels or "ionotropic" receptors, termed P2X (Khakh, B.S. et al., Pharmacol. Rev. 2001, 53, 107-118; Abbracchio, M.P. et al., Pharmacol. Rev. 2006, 58, 281-341; Jacobson, K. A.; Jarvis, M.F.; Williams, M. J. Med. Chem. 2002, 45, 4057-4093; Müller, C.E. Curr. Pharm. Design 2002, 8, 2353-69). Currently, there are seven subtypes in the P2X family and 8 subtypes in the P2Y family (P2Y_{1,2,4,6,11,12,13,14}).

Blood platelets express one P2X receptor subtype, P2X₁, activated by ATP, and two P2Y receptor subtypes, P2Y₁ and P2Y₁₂, both of which are activated by the nucleotide ADP, which induces platelet aggregation (Gachet C., Annu. Rev. Pharmacol. Toxicol. 2006, 46, 277-300).

The combined action of both P2Y receptor subtypes on thrombocytes is required for a full aggregation response following stimulation by ADP. P2Y₁ , coupled to the heterotrimeric GTP-binding protein G_{q} and phospholipase C_{β} is responsible for the mobilization of calcium ions from internal stores mediating platelet shape change and the initial wave of rapidly reversible platelet aggregation induced by ADP. P2Y₁₂, on the other hand, is coupled to inhibition of adenylate cyclase through G; protein and mediates a progressive and sustained aggregation not preceded by shape change. The latter receptor also plays an important role in the potentiation of platelet secretion induced by several agonists, and its congenital deficiency has been shown to result in a lifelong bleeding disorder (Leon, C. et al. Thromb. Haemost. 1999, 81: 775-781; Cattaneo, M., Gachet, C. Arterioscler. Thromb. Vasc. Biol. 1999, 19: 2281-2285).

Modulation of P2 receptors in platelets appears to be of paramount importance in regulating platelet function, and, as a consequence, in controlling thrombotic diseases, which are the most common cause of morbidity and mortality in the Western world. The P2Y₁₂ receptor shows a limited expression in the human body. It is expressed in very high density on blood platelets (E1-Tayeb, A., Griessmeier, K.J, Mueller, C.E. Bioorg, Med. Chem. Lett. 2005, 15: 5450-52). Apart from that, it shows a limited expression pattern (no or only low expression in peripheral tissues and cells, some expression in the brain) making it an ideal drug target for selective therapeutic intervention (Barnard E.A., Simon, J. Trends Pharmacol. Sci. 2001, 22: 388-391).

The P2Y₁₂ receptor on blood platelets is the target of the antithrombotic drugs ticlopidine and clopidogrel, and the newly developed analog prasugrel (Hollopeter, G. et al., Nature 2001, 409, 202-207; Zangh et al., J. Biol.Chem. 2001, 276: 8608-8615; Takasaki, J. et al., Mol. Pharmacol. 2001, 60: 432-439), representing platelet aggregation inhibitors that are effective in the prevention and treatment of arterial thrombosis. Ticlopidine, clopidogrel and prasugrel are no direct P2Y₁₂ antagonists. They have to be bioactivated by cytochrome P450 enzymes (oxidation) followed by ring-opening to the corresponding highly unstable thiol derivatives, which are believed to covalently react with the receptor protein forming a disulfide bond, and thus presumably act as covalent, possibly allosteric antagonists at P2Y₁₂ receptors (Savi, P. et al., Thromb. Haemost. 2000, 84: 891-6; Sugidachi, A. et al., Br. J. Pharmacol. 2000, 129: 1439-1446).

Major drawbacks of clopidogrel and related thienotetrahydropyridine derivatives are:
(i) slow onset of action (up to several days) due to the required metabolism;
(ii) long duration of action due to irreversible inhibition;
(iii) "drug resistance" in a high percentage of patients (up to 30%);
(iv) moderate potency, therefore high doses required;
(v) difficulties in steering and controlling the effects.

Therefore, it is highly desirable to develop P2Y₁₂ antagonists that are lacking the drawbacks associated with the standard P2Y₁₂ antagonists such as clopidogrel and other thienopyridine derivatives.

Several companies have recently been developing competitive, reversible P2Y₁₂ antagonists that may be superior to clopidogrel and related drugs. Most approaches started from adenine nucleotides (ATP, ADP) as lead compounds. Astra-Zeneca developed a series of ATP analogs (since ATP is an antagonist at P2Y₁₂ receptors), including cangrelor (AR-C66096MX) and AR-C67085XX (Ingall, A.H. et al., J. Med. Chem. 1999, 42: 213-220; Boeynaems, J.-M. et al., Curr. Opin. Invest. Drugs 2005, 6: 275-282). These compounds are very polar since they are negatively charged at a physiologic pH value of 7.4, and therefore cannot be perorally applied. In addition, they are metabolically unstable due to hydrolysis by nucleotide pyrophosphatases and therefore exhibit only a very short half-life in vivo. Furthermore, they are difficult to synthesize and in particular difficult to purify in large amounts that are required for use as drugs. Their selectivity for P2Y₁₂ receptors is unclear, since they may also bind to other nucleotide binding sites.

K.A. Jacobson and colleagues described a series of adenosine bisphosphate derivatives, e.g. MRS-2395, as P2Y₁₂ antagonists (Jacobson, K.A. et al., Curr. Top. Med. Chem. 2004, 4, 805-819), however with very moderate potency. In addition, the ester groups in the molecules may be metabolically unstable.

Inspire Pharmaceuticals Inc. developed nucleoside 5'-monophosphates and related compounds as P2Y₁₂ antagonists for intravenous application, e.g. for the treatment of postoperative bleeding and for blood product transfusion medicinal applications (see, for example, international patent publication WO 2006/119507). INS50589 has been evaluated in initial clinical trials (Johnson, F.L. et al., J. Thromb. Haemost. 2005, 3 (Suppl. 1), P2206). The compounds are again not perorally applicable and have very short half-lives due to enzymatic dephosphorylation leading to inactive nucleosides or nucleoside analogs.

An important progress was achieved by the development of the nucleoside analog AZD6140 and related compounds, which are perorally active P2Y₁₂ antagonists, and AZD6140 is currently developed for clinical application as an antithrombotic agent (Tantry, U.S.; Bliden, K.P.; Gurberl, P.A. Expert Opin. Investig. Drugs 2007, 16, 225-9; van Giezen, J.J.J., Humphries, R.G. Seminars in thrombosis and hemostasis 2005, 31, 195-204). However, the drug molecule is stereochemically sophisticated and requires a demanding, multi-step synthesis. Furthermore, recent studies have indicated dyspnoea as a possible side-effect of AZD6140 as well as other adenosine-related nucleoside and nucleotide derivatives and analogs due to the formation of adenosine receptor-activating metabolites (Serebruany, V.L., Stebbing, J., Atar, D. Int. J. Clin. Pract. 2007, 61, 529-33).

Screening strategies have yielded P2Y₁₂ antagonists that are not structurally related to nucleotides or nucleosides. An example is CT-50547, a benzothiazolo[2,3-c]thiadiazine derivative, which exhibits affinity for the human platelet P2Y₁₂ receptor of greater than 100 nM (Scarborough RM et al. Bioorg. Med. Chem. Lett. 11, 1805-08, 2001; Scarborough RM et al., EP 1734041A2, 2006, and related patents). Two recent patent applications described other groups of non-nucleotide platelet ADP receptor antagonists (US patent application 2005/0065163 and international patent publication WO 2005/000281), however no pharmacological data are given in the US patent application.

The anthraquinone derivative Reactive Blue 2 (RB-2) has been found to bind to a variety of nucleotide-binding proteins in the human body, including a number of different P2 receptor subtypes (Burnstock G. Curr Top. Med. Chem. 2004, 4, 793-803). Several publications indicated that RB-2 was able to antagonize P2Y₁₂ receptor activation and may thus be acting as a direct P2Y₁₂ receptor antagonist (Unterberger, U. et al., Br. J. Pharmacol. 2002, 135, 673-84; Kulick, M.B.; von Kügelgen, I, J. Pharmacol. Exp. Ther. 2002, 303, 520-6).

Hence, a number of RB-2 derivatives were synthesized and investigated in functional studies at P2Y₁ and P2X receptors (Tuluc, F. et al., Naunyn Schmiedebergs Arch. Pharmacol., 1998, 357, 111. Glänzel, M. et al., Eur. J. Med. Chem., 2003, 38, 303. Glänzel, M. et al. Eur. J. Med. Chem. 2005, 40, 1262). However, no data were published on their potency at the P2Y₁₂ receptor subtype. In a recent review article RB-2 was described as a P2Y₁₂ antagonist with a Kᵢ value of 1.3 µM (Abbracchio, M.P. et al., Pharmacol. Rev. 2006, 58, 281-341 ).

However, although one anthraquinone derivative, RB-2, has shown promise as P2Y₁₂ antagonist, the synthesis of analogs and the development of this class of compounds has been hampered to date by the available synthesis procedures that require harsh conditions e.g., high temperatures and long reaction times, and suffer from mostly poor yields.

The efforts aiming in the direction of developing P2Y₁₂ antagonists show that there is still need in the art for compounds that act as specific P2Y₁₂ inhibiting agents and thus may be useful as antithrombotic agents for the inhibition of platelet aggregation. Since the P2Y₁₂ receptor is also expressed in brain, e.g. in microglial cells (Hayes S.E. et al. Nature Neuroscience 2006, 9, 1512-1519), specific P2Y₁₂ antagonists may, in addition to their use as anti-thrombotics, be used to prevent or treat CNS disorders, including neuroinflammatory conditions and neurodegenerative disorders (e.g. Alzheimer's and Parkinson's disease).

Hence, it was an objective of the present invention to provide compounds that act as potent and selective P2Y₁₂ antagonists and thus may be useful as antithrombotics and avoid essentially all of the above-mentioned drawbacks of the known drugs.

Moreover, it was an objective of the present invention to provide methods for preparing said compounds. It was furthermore an objective of the present invention to provide compounds and pharmaceutical formulations for the treatment, alleviation and/or prevention of a host of diseases and disorders connected to P2Y₁₂ function. It was a further objective of the present invention to provide the use of these compounds for alleviating, preventing and/or treating diseases and disorders connected to P2Y₁₂ function, particularly for, but not limited to the use as antithrombotic agents by inhibiting platelet aggregation.

### Summary of the Invention

The present invention is directed to certain naphthalene or anthracene derivatives or heterocyclic analogs thereof which act as P2Y₁₂ antagonists and are therefore useful for inhibiting platelet aggregation.

In a first aspect, the invention is directed to a compound according to Formula I: 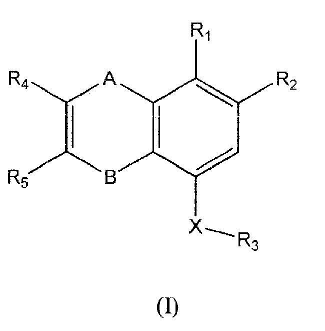 wherein:
A and B are independently CH₂, O, S, NH, C=O, C=NH, C=S, or C=N-OH;
X is selected from the group consisting ofNH, O, S, C=O, and CH₂;
   R¹ and R² are independently selected from the group consisting of hydrogen, unsubstituted or substituted C₁-C₁₀ alkyl, unsubstituted or substituted C₁-C₁₀ alkenyl, unsubstituted or substituted C₁-C₁₀ alkynyl, unsubstituted or substituted C₃-C₈ cycloalkyl, unsubstituted or substituted C₁-C₁₀ alkoxy, unsubstituted or substituted C₃-C₈ cycloalkoxy, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -C(O)R , -C(O)OR , -C(O)NRR', -NRR', - S(O)₂R, -S(O)₂OR, and -S(O)₂NRR';
R³ is selected from the group consisting of hydrogen, unsubstituted or substituted C₁-C₁₀ alkyl, unsubstituted or substituted C₁-C₁₀ alkenyl, unsubstituted or substituted C₁-C₁₀ alkynyl, unsubstituted or substituted C₃-C₈ cycloalkyl, unsubstituted or substituted C₁-C₁₀ alkoxy, unsubstituted or substituted C₃-C₈ cycloalkoxy, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, alkylaryl, alkylheteroaryl, an unsubstituted or substituted 7 to 12-membered bicyclic alkyl or heterocyclic ring wherein 1 to 3 ring members are independently nitrogen, oxygen or sulfur, an unsubstituted or substituted 10 to 16-membered tricyclic alkyl or heterocyclic ring wherein 1 to 3 ring members are independently nitrogen, oxygen or sulfur, 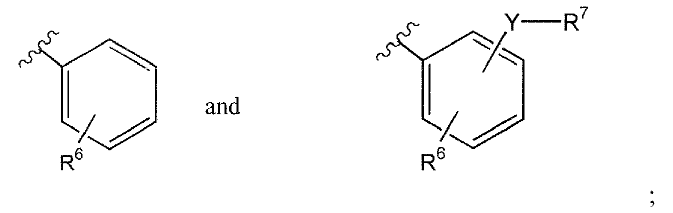
R⁴ and R⁵ are hydrogen, or, combined, R⁴ and R⁵ may, together with the carbon atoms to which they are attached, form a group selected of the groups consisting of unsubstituted or substituted C₃-C₈ cycloalkyl, unsubstituted or substituted C₆-C₁₄ aryl, unsubstituted or substituted 5- to 10-membered heteroaryl wherein 1 to 4 ring atoms are independently selected from nitrogen, oxygen or sulfur, and unsubstituted or substituted 5-to 10-membered heteroalicyclic ring wherein 1 to 3 ring atoms are independently nitrogen, oxygen or sulfur;
R⁶ represents one to four substituents which are independently selected from the group consisting of hydrogen, halogen, unsubstituted or substituted C₁-C₄ alkyl, alkenyl or alkynyl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -NRR', -NO₂, unsubstituted or substituted C₁-C₄ alkoxy, -C(O)R, -C(O)OR , -C(O)NRR', - S(O)₂R, -S(O)₂OR, and -S(O)₂NRR';
Y is selected from the group consisting of hydrogen, halogen, NH, O, S, CH₂, CH₂CH₂, C(O), C(O)O, S(O)₂O, or unsubstituted C₁-C₄ alkoxy, with the proviso that when Y is hydrogen, halogen or alkoxy R⁷ is missing;
R⁷ is selected from the group consisting of hydrogen, halogen, -OR, unsubstituted or substituted C₁-C₁₀ alkyl, alkenyl or alkynyl, unsubstituted or substituted C₁-C₁₀ alkoxy, unsubstituted or substituted C₃-C₈ cycloalkoxy, unsubstituted or substituted C₃-C₈ cycloalkyl, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic wherein 1 to 3 ring atoms are independently nitrogen, oxygen or sulfur, - C(O)R, -C(O)OR , -C(O)NRR-, -NRR', - S(O)₂R, -S(O)₂ -S(O)₂NRR', and -P(O)RR'; and
R and R' are independently selected from the group consisting of hydrogen and unsubstituted C₁-C₄ alkyl.

Also encompassed by the present invention are pharmaceutically acceptable salts, and prodrugs of these compounds.

In another aspect, the present invention relates to compounds of Formula II 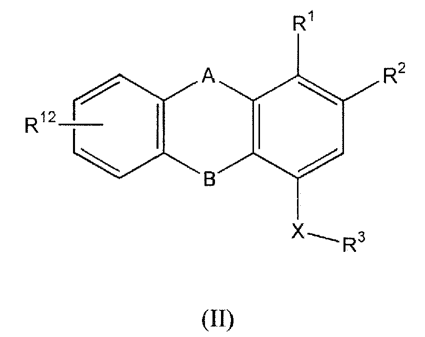 wherein A, B, R¹, R ² X and R³ are defined as above and R¹² represents one to four substituents independently selected from the group consisting of hydrogen, halogen, unsubstituted or substituted C₁-C₄ alkyl, alkenyl or alkynyl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -NRR', -NO₂, unsubstituted or substituted C₁-C₄ alkoxy, -C(O)R , -C(O)OR , - C(O)NRR', - S(O)₂R, -S(O)₂OR, and -S(O)₂NRR' with R and R' as defined above.

In certain embodiments of the compounds of Formula I and II, at least one of A and B is carbonyl. In another embodiment A and B are both carbonyl.

In another embodiment of the invention, X is NH.

In a further embodiment of the present invention, R¹ is amino.

In still another embodiment, R² is -S(O)₂OR or -C(O)OR, with R as defined above. In one embodiment R is hydrogen so that, under physiological conditions, R² is a negatively charged group. Alternatively, R² may be the respective sodium or potassium salt of the carboxylic acid or sulfonic acid group. In another embodiment, R² may be tetrazolyl.

In a specific embodiment of the present invention, R³ is an aryl group of the following formula 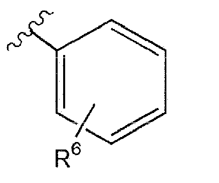 wherein R⁶ is as defined as above. In a specific embodiment, R⁶ represents one to four substituents independently selected from the group consisting of hydrogen, -S(O)₂OR, -C(O)OR, sulfonyl, carboxy, amino, halogen, C₁-C₄ alkoxy, C₁-C₄ alkyl, hydroxy, and nitro. In another specific embodiment, R⁶ is a tetrazolyl, sulfonic acid or carboxylic acid group or the respective sodium or potassium salt.

In another specific embodiment, R³ is an aryl group of the following formula 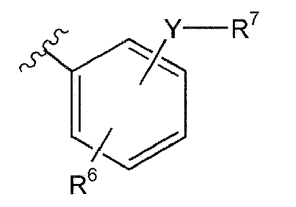 wherein Y, R⁶ and R⁷ are as defined above. In a specific embodiment, R⁶ represents one to four substituents independently selected from the group consisting of hydrogen, -S(O)₂OR, -C(O)OR, sulfonyl, carboxy, amino, halogen, C₁-C₄ alkoxy, C₁-C₄ alkyl, hydroxy, and nitro. In another specific embodiment, R⁶ is a tetrazolyl, sulfonic acid or carboxylic acid group or the respective sodium or potassium salt. In one embodiment R⁶ is in the meta and/or ortho position of the aryl ring. In a further embodiment, Y is NH and R⁷ is unsubstituted or substituted C₆-C₁₄ aryl or an unsubstituted or substituted 5- to 10-membered heteroaryl wherein 1 to 4 ring atoms are independently selected from nitrogen, oxygen or sulfur. In a specific aspect of this embodiment, R⁷ is unsubstituted or substituted C₆ aryl or an unsubstituted or substituted 6-membered heteroaryl wherein 1 to 3 ring atoms are nitrogen. In one specific embodiment, -Y-R⁷ is in the para position of the aryl ring.

In another embodiment, R⁴ and R⁵ combine to form an aryl ring.

In still another embodiment, R¹² is hydrogen.

A further aspect, the present invention thus relates to a compound of Formula III 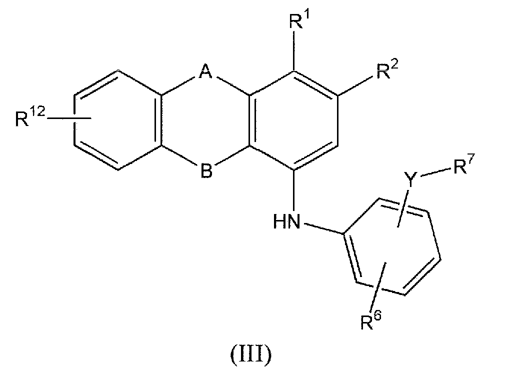 wherein A, B, R¹, R², Y, R₆ R⁷ and R¹² are defined as above.

In certain embodiments of the compounds of Formula III, at least one of A and B is carbonyl. In another embodiment A and B are both carbonyl.

In a further embodiment, in the compound of Formula III R¹ is amino and R² is -S(O)₂OR or -C(O)OR, with R as defined above. In a specific embodiment R is hydrogen so that, under physiological conditions, R² is a negatively charged group. Alternatively, R² may be the respective sodium or potassium salt of the carboxylic acid or sulfonic acid group. In still another embodiment, R² is a tetrazolyl group.

In one embodiment of the compounds of Formula III, R⁶ represents one to four substituents independently selected from the group consisting of hydrogen, -S(O)₂ -C(O)OR, sulfonyl, carboxy, amino, halogen, C₁-C₄ alkoxy, C₁-C₄ alkyl, hydroxy, and nitro. In another specific embodiment, R⁶ is a tetrazolyl, sulfonic acid or carboxylic acid group or the respective sodium or potassium salt. In one embodiment R⁶ is in the meta or ortho position of the aryl ring. In a further embodiment, Y is NH and R⁷ is unsubstituted or substituted C₆-C₁₄ aryl or an unsubstituted or substituted 5- to 10-membered heteroaryl wherein 1 to 4 ring atoms are independently selected from nitrogen, oxygen or sulfur. In a specific aspect of this embodiment, R⁷ is unsubstituted or substituted C₆ aryl or an unsubstituted or substituted 6-membered heteroaryl wherein 1 to 3 ring atoms are nitrogen. In one specific embodiment, -Y-R⁷ is in the para position of the aryl ring.

In one further embodiment of the compounds of Formula III, R¹² is hydrogen.

In still another aspect, the present invention is directed to compounds of formula IV 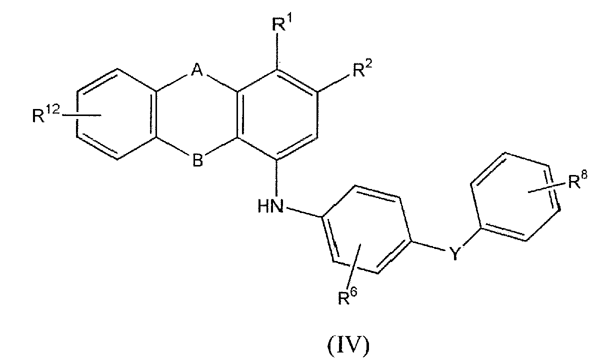 wherein A, B, R¹, R², R⁶, Y and R¹² are defined as above and R⁸ represents one to five substituents independently selected from the group consisting of C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NRR', with R and R' as defined above. In one specific embodiment R⁸ represents one to three substituents independently selected from the group consisting of halo, unsubstituted C₁-C₄ alkyl or alkoxy, carboxy, sulfonyl, amino and hydroxy.

In certain embodiments of the compounds of Formula IV, at least one of A and B is carbonyl. In another embodiment A and B are both carbonyl.

In a specific embodiment of the invention, Y in Formula IV is NH.

In a further embodiment, in the compound of Formula IV R¹ is amino and R² is tetrazolyl, -S(O)₂OR or -C(O)OR, with R as defined above. In a specific embodiment, R is hydrogen so that, under physiological conditions, R² is a negatively charged group. Alternatively, R² may be the respective sodium or potassium salt of the carboxylic acid or sulfonic acid group.

In one embodiment of the compounds of Formula IV, R⁶ represents one to four substituents independently selected from the group consisting of hydrogen, -S(O)₂OR, -C(O)OR, sulfonyl, carboxy, amino, halogen, C₁-C₄ alkoxy, C₁-C₄ alkyl, hydroxy, and nitro. In another specific embodiment, R⁶ is a tetrazolyl, sulfonic acid or carboxylic acid group or the respective sodium or potassium salt.

In a further embodiment, R¹² is hydrogen.

Representative compounds of the invention are shown in Table 1 below:

**Table 1**

| | |
|---|---|
| 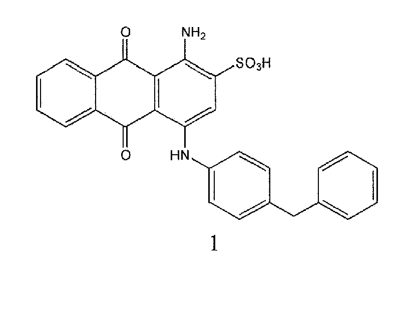 | 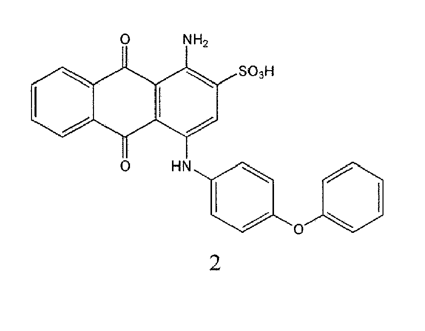 |
| 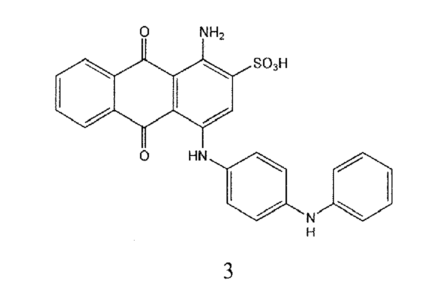 | 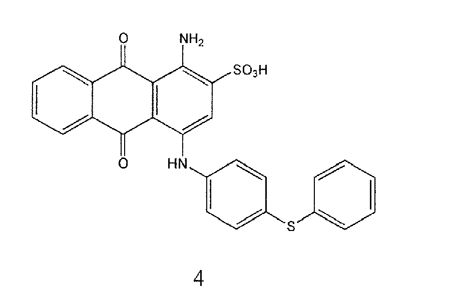 |
| 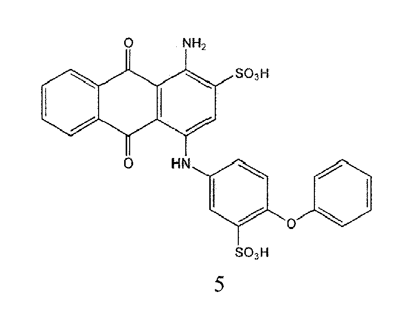 | 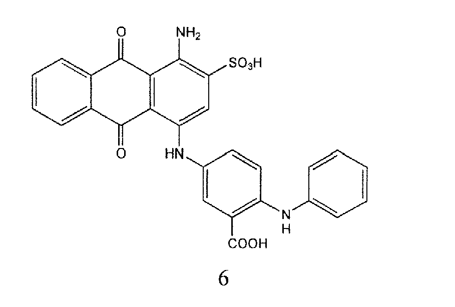 |
| 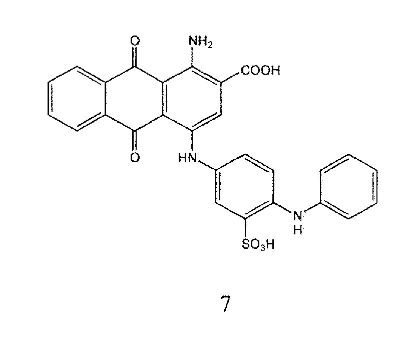 | 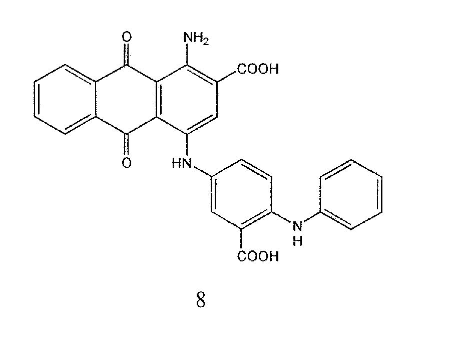 |
| 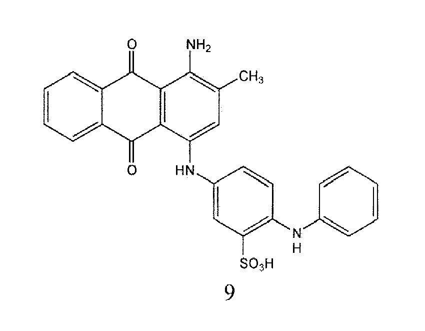 | 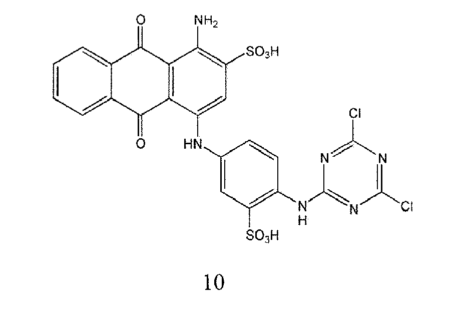 |
| 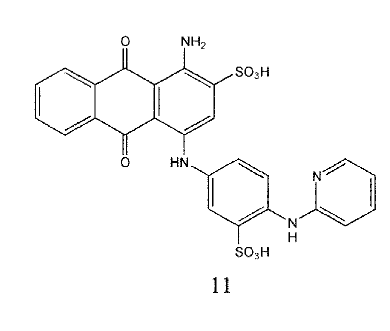 | 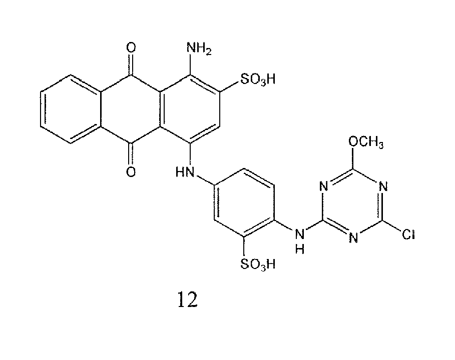 |
| 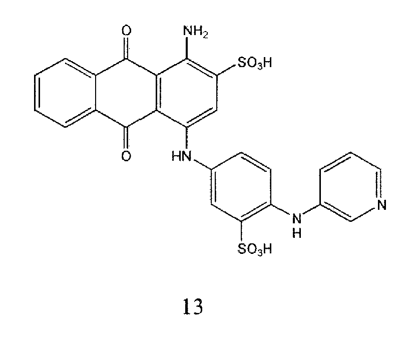 | 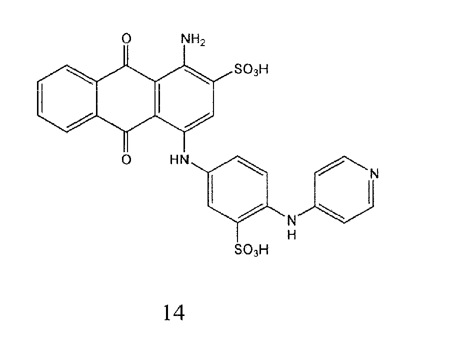 |
| 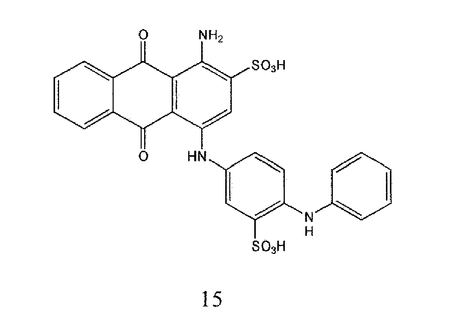 | 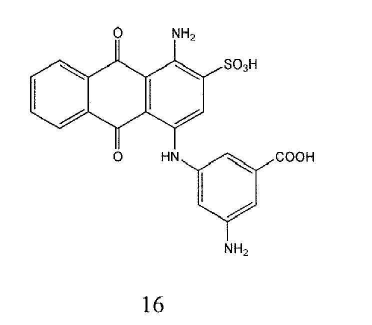 |
| 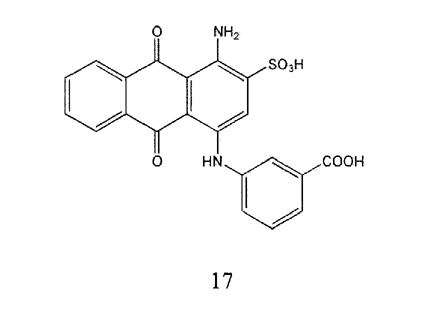 | 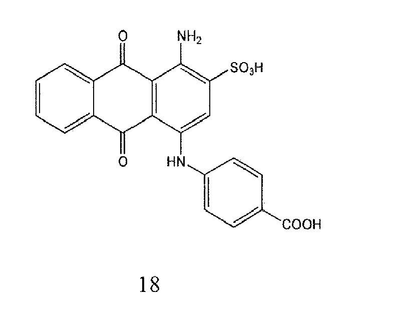 |
| 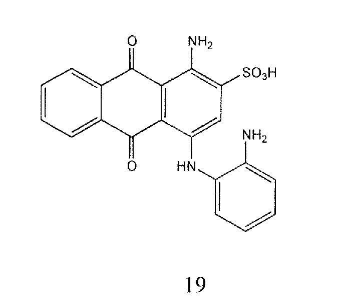 | 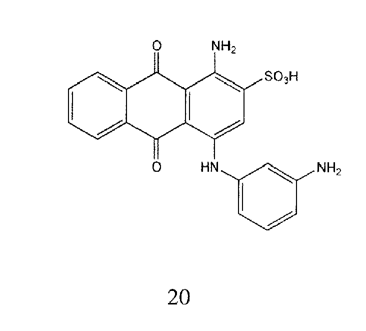 |
| 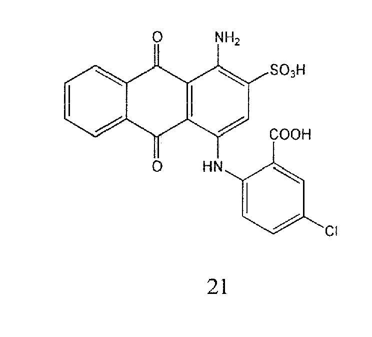 | 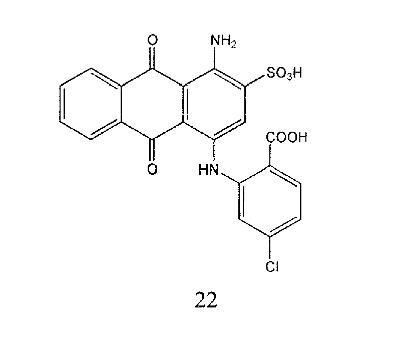 |
| 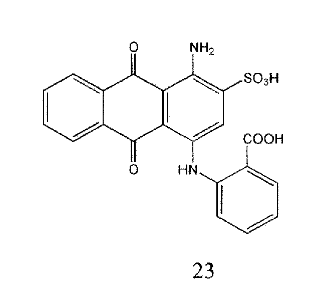 | 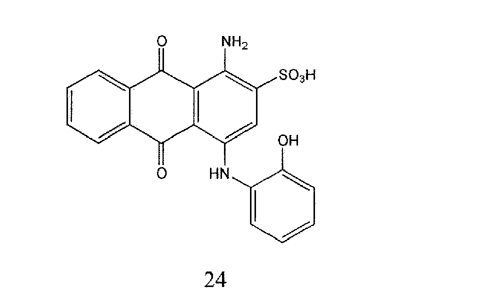 |
| 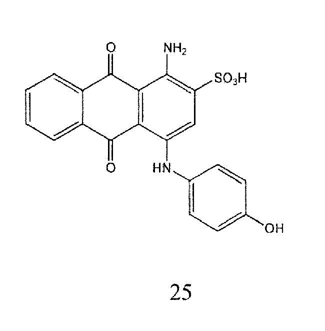 | 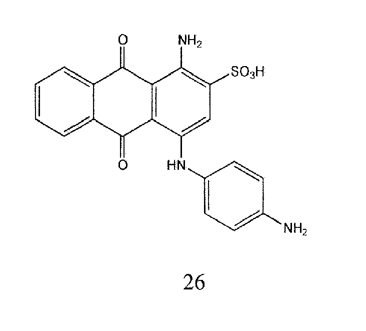 |
| 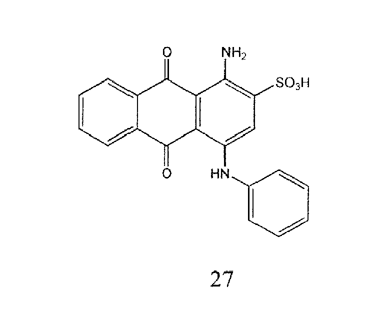 | 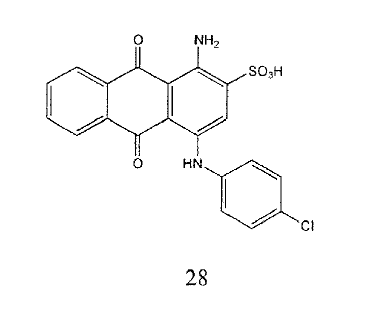 |
| 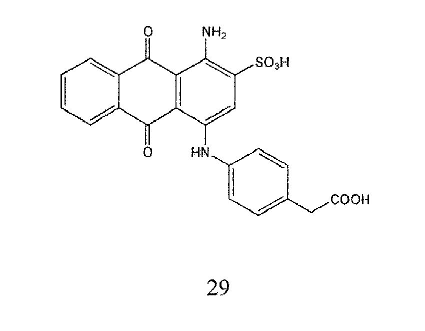 | 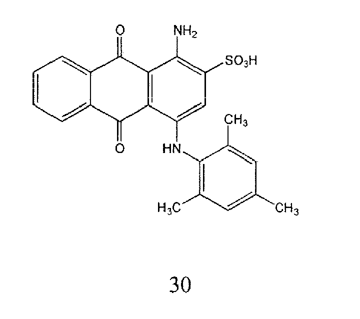 |
| 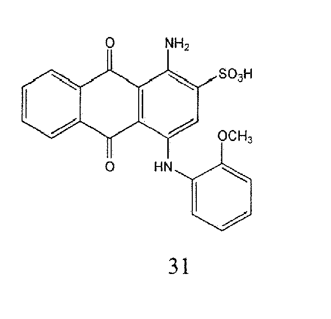 | 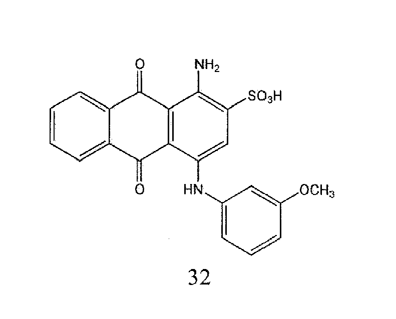 |
| 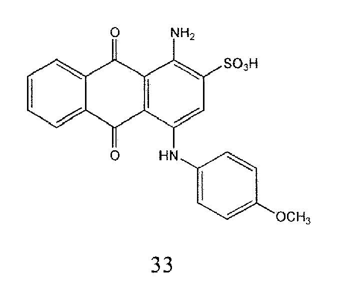 | 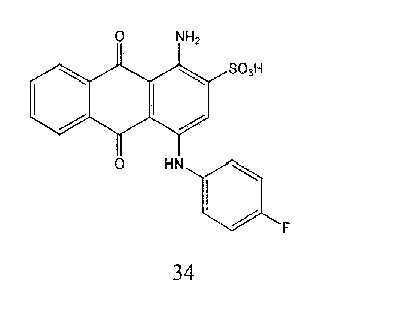 |
| 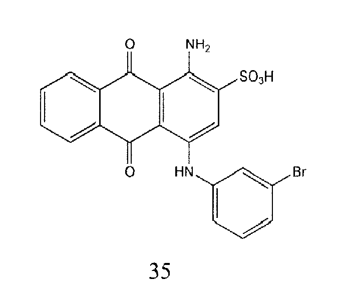 | 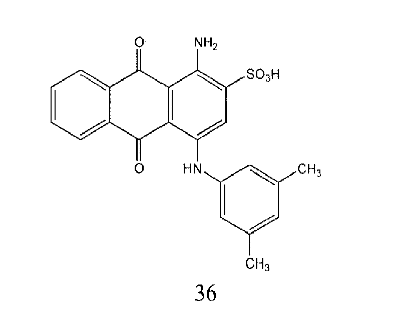 |
| 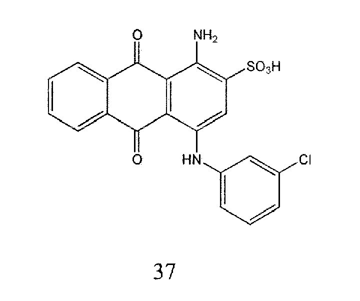 | 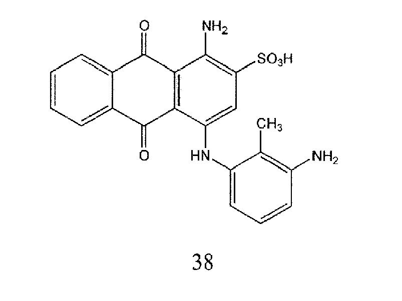 |
| 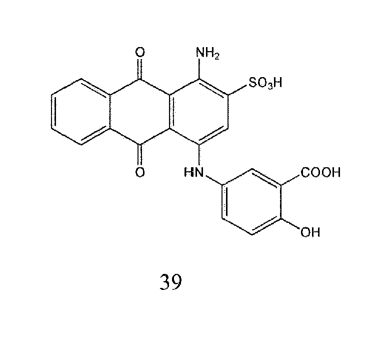 | 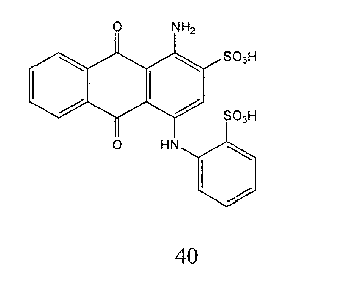 |
| 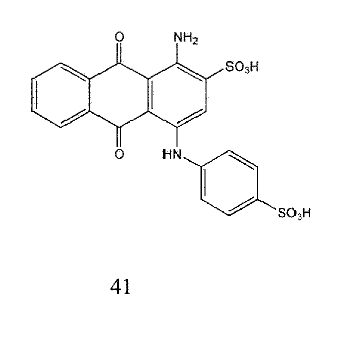 | 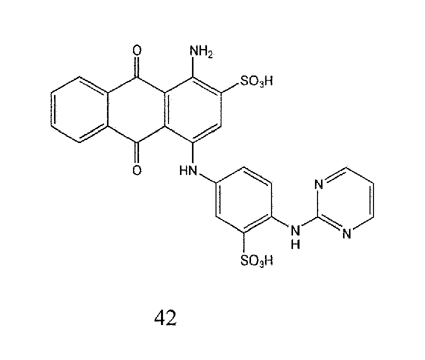 |
| 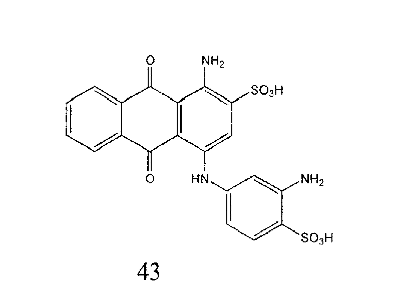 | 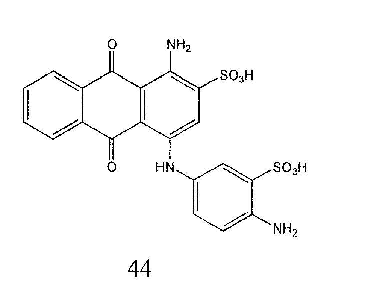 |
| 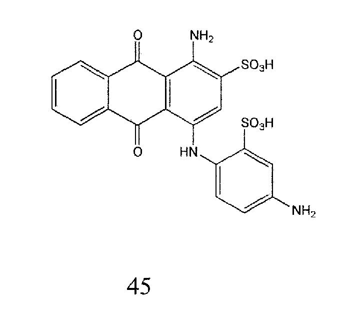 | 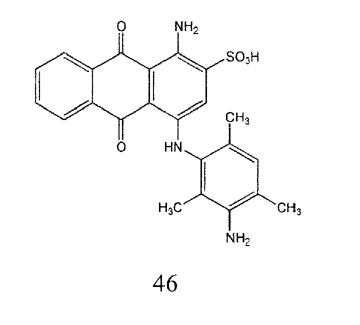 |
| 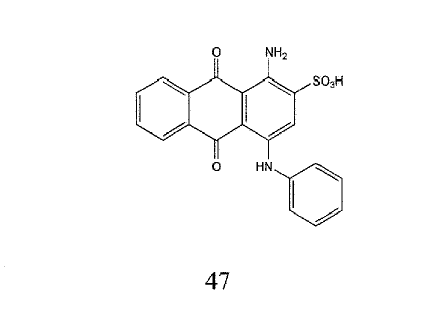 | 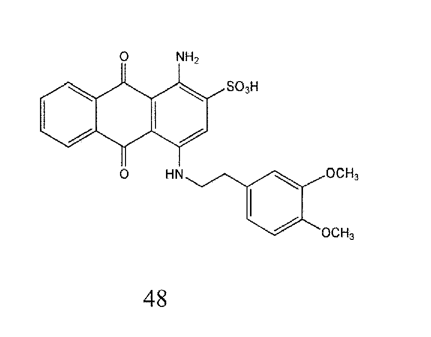 |
| 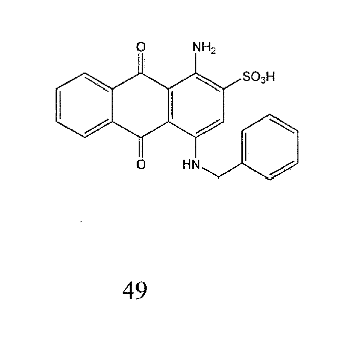 | 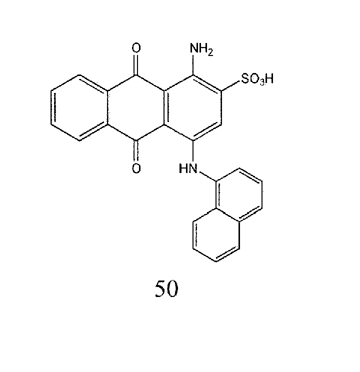 |
| 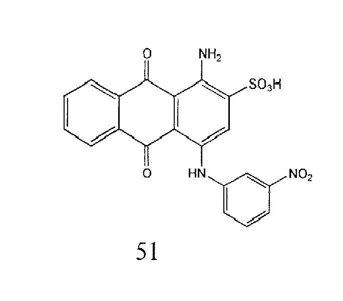 | 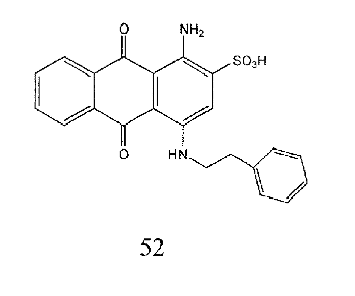 |
| 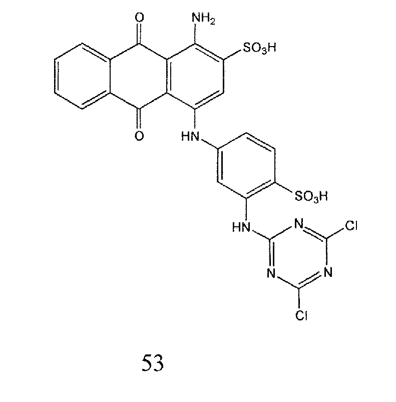 | 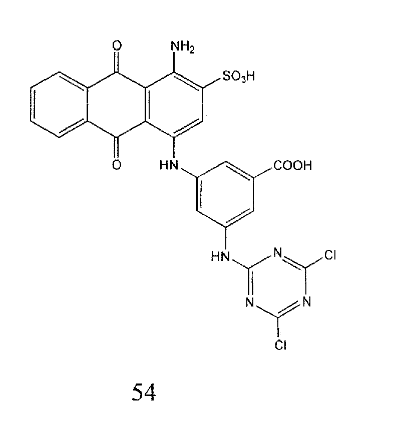 |
| 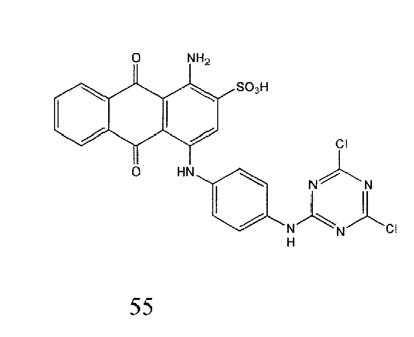 | 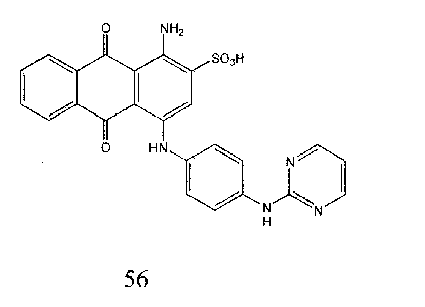 |
| 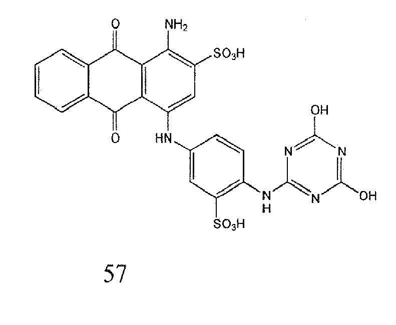 | 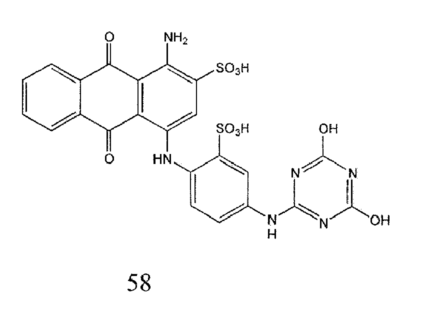 |

Also encompassed by the present invention are pharmaceutically acceptable salts or prodrugs of the above compounds of Formulae I-IV and those set forth in Table 1.

In another aspect, the present invention relates to a pharmaceutical composition comprising any of the compounds or salts of the present invention and, optionally, a pharmaceutically acceptable carrier or excipient. This composition may additionally comprise further compounds or medicaments, such as, for example, thrombolytic agents or antithrombotics besides the invented compounds.

In still another aspect, the present invention is directed to the use of the invented compounds for antagonizing P2Y₁₂ receptor function. Thus, in one embodiment, the compounds of the present invention may be used as inhibitors of platelet aggregation, i.e. anti-thrombotics.

In a further aspect, the invented compounds may thus also be used for the prevention, alleviation and/or treatment of a disease or disorder related to P2Y₁₂ receptor activity. Diseases or disorders related to P2Y₁₂ receptor function may be, without limitation, selected from the group of stroke, myocardial infarction, vascular disorders, atherosclerosis, acute coronary syndrome, peripheral artery disease, embolism and the like. Since the P2Y₁₂ receptor is also expressed in brain, these diseases and disorders may also include CNS disorders, including neuroinflammatory conditions and neurodegenerative disorders (e.g. Alzheimer's and Parkinson's disease).

Preferably, the subject afflicted by a disease treated, alleviated or prevented according to the invented use is a human.

In still another aspect, the present invention discloses a method for antagonizing the P2Y₁₂ receptor function comprising the step of contacting cells expressing a P2Y₁₂ receptor with at least one of the above compounds. This method can include a method for the treatment of a disease or disorder mediated by or involving a P2Y₁₂ receptor, the method comprising the administration of a pharmaceutically active amount of at least one of the compounds according to the invention to a patient in need thereof.

In a further aspect, the present invention also encompasses .a method of preparing compounds of Formulae I-IV. In one embodiment, the present invention is directed to a method for preparing any one of the invented compounds, wherein the method comprises the preparation of an N-substituted 5-nitroanthranilic acid having the Formula V 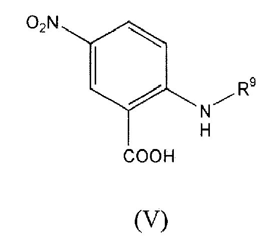 as an intermediate by reacting a 5-nitro-2-halo-benzoic acid having the Formula VI 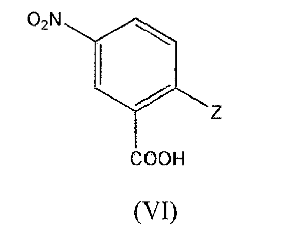 with an amine selected from the group of unsubstituted or substituted C₁-C₁₀ alkylamines, unsubstituted or substituted C₃-C₈ cycloalkylamines and unsubstituted or substituted C₆-C₁₄ arylamines without a solvent or catalyst under mild microwave reaction conditions to form the N-substituted 5-nitroanthranilic acid of formula VIII, wherein R⁹ is an unsubstituted or substituted C₁-C₁₀ alkyl, unsubstituted or substituted C₃-C₈ cycloalkyl or unsubstituted or substituted C₆-C₁₄ aryl group and Z is halogen, for example chlorine, bromine or iodine.

In case the aniline derivative is solid, the reaction is performed in a suitable solvent, e.g., without limitation, DMF, pyridine and water. In one embodiment of the reaction, the addition of a base, for example K₂CO₃ may be required.

In still another aspect, the present invention is directed to a synthetic method for the preparation of compounds of Formulae I-IV, wherein the method comprises the synthesis of N-substituted anthraquinone derivatives by use of a microwave-assisted, copper-catalyzed Ullmann C-N coupling reaction according to reaction Scheme 1: 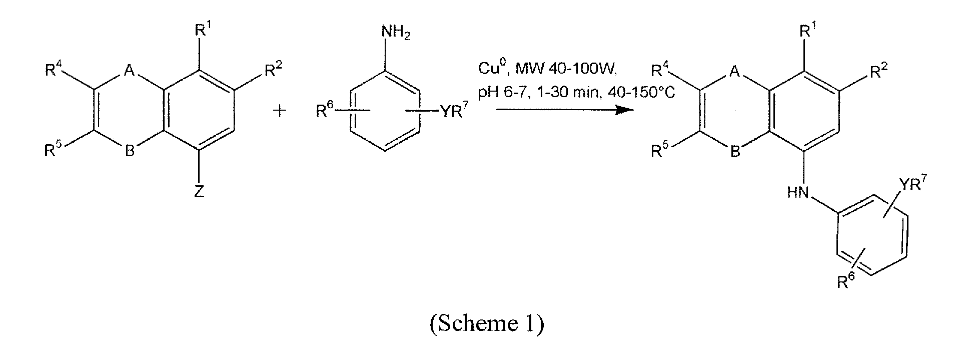 wherein A, B, R¹, R², R4, R⁵, R⁶, R⁷, and Y are defined as above and Z is a leaving group, for example bromine, chlorine or iodine.

### Brief description of the drawings

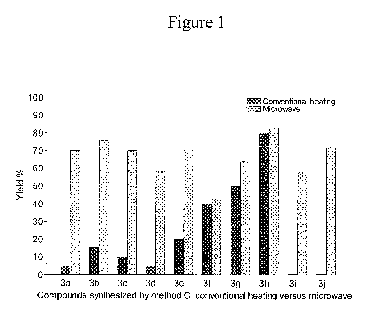

Figure 1 shows the results of the copper-catalyzed synthesis of an anilinoanthraquinone according to the present invention with microwave irradiation compared to conventional heating.

### Detailed Description of the Invention

### Definitions

Unless otherwise stated the following terms used in the specification and claims have the meanings discussed below:

"Alkyl" refers to a saturated aliphatic hydrocarbon including straight chain, or branched chain groups. Preferably, the alkyl group has 1 to 10 carbon atoms (whenever a numerical range; e.g.,"1-10", is stated herein, it means that the group, in this case the alkyl group, may contain 1 carbon atom, 2 carbon atoms, 3 carbon atoms, etc. up to and including 10 carbon atoms). More specifically, it may be a medium size alkyl having 1 to 6 carbon atoms or a lower alkyl having 1 to 4 carbon atoms e. g., methyl, ethyl, n-propyl, isopropyl, butyl, iso-butyl, tert-butyl and the like. The alkyl group may be substituted or unsubstituted. When substituted, the substituent group(s) is one or more, for example one or two groups, individually selected from the group consisting of C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰R¹¹ where R¹⁰ and R¹¹ are independently selected from the group consisting of hydrogen, C₁ -C₄ alkyl, C₃-C₈ cycloalkyl, C₆-C₁₄ aryl, carbonyl, acetyl, sulfonyl, amino, and trifluoromethanesulfonyl, or R¹⁰ and R¹¹, together with the nitrogen atom to which they are attached, combine to form a five-or six-membered heteroalicyclic ring.

A "cycloalkyl"' group refers to an all-carbon monocyclic ring (i.e., rings which share an adjacent pair of carbon atoms) of 3 to 8 ring atoms wherein one of more of the rings does not have a completely conjugated pi-electron system e.g., cyclopropyl, cyclobutyl, cyclopentyl, cyclohexyl, cyclobutenyl, cyclopentenyl, cyclohexenyl, and the like. Examples, without limitation, of cycloalkyl groups are cyclopropane, cyclobutane, cyclopentane, cyclopentene, cyclohexane, adamantane, cyclohexadiene, cycloheptane and, cycloheptatriene. A cycloalkyl group may be substituted or unsubstituted. When substituted, the substituent group(s) is one or more, for example one or two groups, individually selected from C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, C-carboxy, 0-carboxy, nitro, silyl, sulfinyl, sulfonyl, amino, and NR¹⁰R¹¹, with R¹⁰ and R¹¹ as defined above.

An "alkenyl" group refers to an alkyl group, as defined herein, consisting of at least two carbon atoms and at least one carbon-carbon double bond e. g., ethenyl, propenyl, butenyl or pentenyl and their structural isomeric forms such as 1-or 2-propenyl, 1-, 2-, or 3-butenyl and the like.

An "alkynyl" group refers to an alkyl group, as defined herein, consisting of at least two carbon atoms and at least one carbon-carbon triple bond e. g., acetylene, ethynyl, propynyl, butynyl, or pentynyl and their structural isomeric forms as described above.

An "aryl" group refers to an all-carbon monocyclic or fused-ring polycyclic (i.e., rings which share adjacent pairs of carbon atoms) groups of 6 to 14 ring atoms and having a completely conjugated pi-electron system. Examples, without limitation, of aryl groups are phenyl, naphthalenyl and anthracenyl. The aryl group may be substituted or unsubstituted. When substituted, the substituted group(s) is one or more, for example one, two, or three substituents, independently selected from the group consisting of C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰R¹¹, with R¹⁰ and R¹¹ as defined above. Preferably the substituent(s) is/are independently selected from chloro, fluoro, bromo, methyl, ethyl, hydroxy, methoxy, nitro, carboxy, methoxycarbonyl, sulfonyl, or amino.

A "heteroaryl" group refers to a monocyclic or fused aromatic ring (i.e., rings which share an adjacent pair of atoms) of 5 to 10 ring atoms in which one, two, three or four ring atoms are selected from the group consisting of nitrogen, oxygen and sulfur and the rest being carbon. Examples, without limitation, of heteroaryl groups are pyridyl, pyrrolyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, 1,3,4-triazinyl, 1,2,3-triazinyl, benzofuryl, isobenzofuryl, benzothienyl, benzotriazolyl, isobenzothienyl, indolyl, isoindolyl, 3H-indolyl, benzimidazolyl, benzothiazolyl, benzoxazolyl, quinolizinyl, quinazolinyl, pthalazinyl, quinoxalinyl, cinnnolinyl, napthyridinyl, quinolyl, isoquinolyl, tetrazolyl, 5,6,7,8-tetrahydroquinolyl, 5, 6, 7, 8-tetrahydroisoquinolyl, purinyl, pteridinyl, pyridinyl, pyrimidinyl, carbazolyl, xanthenyl or benzoquinolyl. The heteroaryl group may be substituted or unsubstituted. When substituted, the substituted group(s) is one or more, for example one or two substituents, independently selected from the group consisting of C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰R¹¹, with R¹⁰ and R¹¹ as defined above. Preferably the substituent(s) is/are independently selected from chloro, fluoro, bromo, methyl, ethyl, hydroxy, methoxy, nitro, carboxy, methoxycarbonyl, sulfonyl, or amino.

A "heteroalicyclic" group refers to a monocyclic or fused ring of 5 to 10 ring atoms containing one, two, or three heteroatoms in the ring which are selected from the group consisting of nitrogen, oxygen and -S(O)ₙ where n is 0-2, the remaining ring atoms being carbon. The rings may also have one or more double bonds. However, the rings do not have a completely conjugated pi-electron system. Examples, without limitation, of heteroalicyclic groups are pyrrolidine, piperidine, piperazine, morpholine, imidazolidine,tetrahydropyridazine, tetrahydrofuran, thiomorpholine, tetrahydropyridine, and the like. The heteroalicyclic ring may be substituted or unsubstituted. When substituted, the substituted group (s) is one or more, for example one, two, or three substituents, independently selected from the group consisting of C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰R¹¹, with R¹⁰ and R¹¹ as defined above. The substituent(s) is/are for example independently selected from chloro, fluoro, bromo, methyl, ethyl, hydroxy, methoxy, nitro, carboxy, methoxycarbonyl, sulfonyl, or amino.

A "hydroxy" group refers to an -OH group.

An "alkoxy" group refers to an -O-unsubstituted alkyl and -O-substituted alkyl group, as defined herein. Examples include and are not limited to methoxy, ethoxy, propoxy, butoxy, and the like.

A "cycloalkoxy" group refers to an -0-cycloalkyl group, as defined herein. One example is cyclopropyloxy.

An "aryloxy" group refers to both an -O-aryl and an -O-heteroaryl group, as defined herein. Examples include and are not limited to phenoxy, napthyloxy, pyridyloxy, furanyloxy, and the like.

A "mercapto" group refers to an -SH group.

An "alkylthio" group refers to both an S-alkyl and an -S-cycloalkyl group, as defined herein. Examples include and are not limited to methylthio, ethylthio, and the like.

An "arylthio" group refers to both an -S-aryl and an -S-heteroaryl group, as defined herein. Examples include and are not limited to phenylthio, napthylthio, pyridylthio, furanylthio, and the like.

A "sulfinyl" group refers to a -S(O)-R" group, wherein, R" is selected from the group consisting of hydrogen, hydroxy, alkyl, cycloalkyl, aryl, heteroaryl (bonded through a ring carbon) and heteroalicyclic (bonded through a ring carbon), as defined herein.

A "sulfonyl" group refers to a -S(O)₂R" group wherein, R" is selected from the group consisting of hydrogen, hydroxy, alkyl, cycloalkyl, aryl, heteroaryl (bonded through a ring carbon) and heteroalicyclic (bonded through a ring carbon), as defined herein.

A "trihalomethyl" group refers to a -CX₃ group wherein X is a halo group as defined herein e. g., trifluoromethyl, trichloromethyl, tribromomethyl, dichlorofluoromethyl, and the like.

"Carbonyl" refers to a -C(=O)-R" group, where R" is selected from the group consisting of hydrogen, alkyl, cycloalkyl, aryl, heteroaryl (bonded through a ring carbon) and heteroalicyclic (bonded through a ring carbon), as defined herein. Representative examples include and the not limited to acetyl, propionyl, benzoyl, formyl, cyclopropylcarbonyl, pyridinylcarbonyl, pyrrolidin-1ylcarbonyl, and the like.

A "thiocarbonyl" group refers to a -C(=S)-R" group, with R" as defined herein.

"C-carboxy" and "carboxy" which are used interchangeably herein refer to a -C(=O)O-R" group, with R" as defined herein, e. g. -COOH, methoxycarbonyl, ethoxycarbonyl, benzyloxycarbonyl, and the like.

An "O-carboxy" group refers to a -OC(=O)R" group, with R" as defined herein, e.g. methylcarbonyloxy, phenylcarbonyloxy, benzylcarbonyloxy, and the like.

An "acetyl" group refers to a -C(=O)CH₃ group.

A "carboxylic acid" group refers to a C-carboxy group in which R" is hydrogen.

A "halo" or "halogen" group refers to fluorine, chlorine, bromine or iodine.

A "cyano" group refers to a -CN group.

A "nitro" group refers to a -NO₂ group.

An "O-carbamyl" group refers to a -OC(=O)NR¹⁰R¹¹ group, with R¹⁰ and R¹¹ as defined herein.

An "N-carbamyl" group refers to a R¹¹OC (=O) NR¹⁰- group, with R¹⁰ and R¹¹ as defined herein.

An "O-thiocarbamyl" group refers to a -OC(=S)NR¹⁰R¹¹ group, with R¹⁰ and R ¹¹ as defined herein.

An "N-thiocarbamyl" group refers to a R¹¹0C(=S)NR¹⁰- group, with R¹⁰ and R¹¹ as defined herein.

An "amino" group refers to an -NR¹⁰R¹¹ group, wherein R¹⁰ and R¹¹ are independently hydrogen or unsubstituted lower alkyl, e.g, -NH₂, dimethylamino, diethylamino, ethylamino, methylamino, and the like.

A "C-amido" group refers to a -C(=O)NR¹⁰R¹¹ group, with R¹⁰ and R¹¹ as defined herein. For example, R¹⁰ is hydrogen or unsubstituted C₁-C₄ alkyl and R¹¹ is hydrogen, C₁-C₄ alkyl optionally substituted with heteroalicyclic, hydroxy, or amino. For example, C(=O)NR¹⁰R¹¹ may be aminocarbonyl, dimethylaminocarbonyl, diethylaminocarbonyl, diethylaminoethylaminocarbonyl, ethylaminoethylaminocarbonyl, and the like.

An "N-amido" group refers to a R¹¹ C(=O)NR¹⁰- group, with R¹⁰ and R¹¹ as defined herein, e.g. acetylamino, and the like.

A "pharmaceutical composition" refers to a mixture of one or more of the compounds described herein, or physiologically/pharmaceutically acceptable salts or prodrugs thereof, with other chemical components, such as physiologically/pharmaceutically acceptable carriers and excipients. The purpose of a pharmaceutical composition is to facilitate administration of a compound to an organism.

The compound of Formulae 1-IV may also act as a prodrug. A "prodrug" refers to an agent which is converted into the parent drug in vivo. Prodrugs are often useful because, in some situations, they may be easier to administer than the parent drug. They may, for instance, be bioavailable by oral administration whereas the parent drug is not. The prodrug may also have improved solubility in pharmaceutical compositions over the parent drug. An example, without limitation, of a prodrug would be a compound of the present invention which is administered as an ester (the "prodrug") to facilitate transmittal across a cell membrane where water solubility is detrimental to mobility but then is metabolically hydrolyzed to the carboxylic acid, the active entity, once inside the cell where water solubility is beneficial. A prodrug may be converted into the parent drug by various mechanisms, including enzymatic processes and metabolic hydrolysis.

A further example of a prodrug might be a short polypeptide, for example, without limitation, a 2-10 amino acid polypeptide, bonded through a terminal amino group to a carboxy group of a compound of this invention wherein the polypeptide is hydrolyzed or metabolized in vivo to release the active molecule. The prodrugs of compounds of Formulae I-IV are within the scope of this invention.

Additionally, it is contemplated that compounds of Formulae I-IV would be metabolized by enzymes in the body of the organism such as a human being to generate a metabolite that can modulate the activity of the P2Y₁₂ receptor. Such metabolites are within the scope of the present invention.

As used herein, a "physiologically/pharmaceutically acceptable carrier" refers to a carrier or diluent that does not cause significant irritation to an organism and does not abrogate the biological activity and properties of the administered compound.

A "pharmaceutically acceptable excipient" refers to an inert substance added to a pharmaceutical composition to further facilitate administration of a compound. Examples, without limitation, of excipients include calcium carbonate, calcium phosphate, various sugars and types of starch, cellulose derivatives, gelatin, vegetable oils and polyethylene glycols.

As used herein, the term "pharmaceutically acceptable salt" refers to those salts which retain the biological effectiveness and properties of the parent compound. Such salts include, but are not restricted to: (1) an acid addition salt which is obtained by reaction of the free base of the parent compound with inorganic acids such as hydrochloric acid, hydrobromic acid, nitric acid, phosphoric acid, sulfuric acid, and perchloric acid and the like, or with organic acids such as acetic acid, oxalic acid, (D) or (L) malic acid, maleic acid, methanesulfonic acid, ethanesulfonic acid, p-toluenesulfonic acid, salicylic acid, tartaric acid, citric acid, succinic acid or malonic acid and the like, preferably hydrochloric acid or (L)-malic acid; or (2) salts formed when an acidic proton present in the parent compound either is replaced by a metal ion, e. g., an alkali metal ion, such as sodium or potassium, an alkaline earth ion, such as magnesium or calcium, or an aluminum ion; or coordinates with an organic base such as ethanolamine, diethanolamine, triethanolamine, tromethamine, N-methylglucamine, and the like.

The terms "P2Y₁₂ related disease or disorder", "P2Y₁₂ associated disease or disorder" and "P2Y₁₂ connected disease or disorder" are used interchangeably herein to refer to a condition involving P2Y₁₂ activity. Examples for such diseases and disorders are stroke, myocardial infarction, such as non-ST elevation myocardial infarction (NSTEMI), myocardial ischemia, for example manifested in stable or unstable angina, various vascular diseases, such as coronary artery disease, peripheral artery disease, atherosclerosis, and cerebrovascular disease, and embolisms, such as thromboembolisms. Since the P2Y₁₂ receptor is also expressed in brain, e.g. in microglial cells, also included are CNS disorders, such as neuroinflammatory conditions and neurodegenerative disorders (e.g. Alzheimer's and Parkinson's disease).

"Treat", "treating" and "treatment" refer to a method of alleviating or abrogating a P2Y₁₂ related disease or disorder and/or its attendant symptoms.

"Prevent", "preventing" and "prevention" refer to a method of hindering a P2Y₁₂ related disease or disorder from occuring, i.e. a prophylactic method.

"Organism" refers to any living entity comprised of at least one cell. A living organism can be as simple as, for example, a single eukariotic cell or as complex as a mammal, including a human being.

"Therapeutically effective amount" refers to that amount of the compound being administered which will relieve to some extent one or more of the symptoms of the disorder being treated.

### Preferred embodiments

### Compounds

With respect to the compounds defined in the Summary of the invention, certain compounds of Formulae I-IV are exemplified below.

One specific group of compounds of Formula I is that wherein:
Both of A and B are carbonyl, or one is carbonyl and the other is imine (NH), X is NH, R¹ is amino, R² is tetrazolyl, -S(O)₂OH, -C(O)OH, -S(O)₂O-Na⁺, or -C(O)O⁻Na⁺, R³ is an aryl group of the following formula 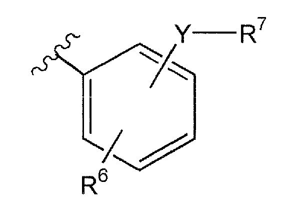 R⁴ and R⁵ are combined to form, together with the carbon atoms to which they are attached, a unsubstituted or substituted 6-membered aryl ring, R⁶ represents one to four substituents independently selected from the group consisting of hydrogen, -S(O)₂OR, -C(O)OR, tetrazolyl, sulfonyl, carboxy, amino, halogen, C₁-C₄ alkoxy, C₁-C₄ alkyl, hydroxy, and nitro, with R as defined above, Y is NH, CH₂, O or S, and R⁷ is (i) unsubstituted or substituted C₆-C₁₄ aryl, wherein, when substituted, the substituents are selected from the group of halo, unsubstituted C₁-C₄ alkoxy, unsubstituted C₁-C₄ alkyl, hydroxy, amino, nitro, -C(O)OH, and -SO₃H, or (iii) unsubstituted or substituted 5- to 10-membered heteroaryl wherein 1 to 4 ring atoms are independently selected from nitrogen, oxygen or sulfur, wherein, when substituted, the substituents are selected from the group consisting of halo, unsubstituted C₁-C₄ alkoxy, unsubstituted C₁-C₄ alkyl, hydroxy, amino, nitro, -C(O)OH, and -SO₃H.

Another specific group of compounds of Formula I is that wherein:
Both of A and B are carbonyl, X is NH, R¹ is amino, R² is tetrazolyl, -S(O)₂OH or - S(O)₂O-Na⁺, R³ is an aryl group of the following formula 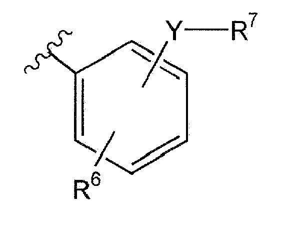 R⁴ and R⁵ are combined to form, together with the carbon atoms to which they are attached, an unsubstituted or substituted 6-membered aryl ring, R⁶ represents one to four substituents independently selected from the group consisting of hydrogen, -S(O)₂OR, -C(O)OR, tetrazolyl, sulfonyl, carboxy, amino, halogen, C₁-C₄ alkoxy, C₁-C₄ alkyl, hydroxy, and nitro, with R as defined above, Y is NH, and R⁷ is (i) unsubstituted or substituted C₆ aryl, wherein, if substituted the substituents are selected from the group consisting of halo, unsubstituted C₁-C₄ alkoxy, and hydroxy, or (ii) an unsubstituted or substituted 6-membered heteroaryl wherein 1 to 3 ring atoms are independently selected from nitrogen, oxygen or sulfur, and wherein, if substituted, the substituents are selected from the group consisting of halo, unsubstituted C₁-C₄ alkoxy.

A specific group of compounds of Formula III is that wherein:
Both of A and B are carbonyl, or one is carbonyl and the other is imine (NH), R¹ is amino, R² is tetrazolyl, -S(O)₂OH, -C(O)OH, -S(O)₂O⁻Na⁺, or -C(O)O⁻Na⁺, R⁶ represents one to four substituents independently selected from the group consisting of hydrogen, -S(O)₂OR, -C(O)OR, tetrazolyl, sulfonyl, carboxy, amino, halogen, C₁-C₄ alkoxy, C₁-C₄ alkyl, hydroxy, and nitro, with R as defined above, Y is NH, CH₂, O or S, R⁷ is (i) unsubstituted or substituted C₆-C₁₄ aryl, wherein, when substituted, the substituents are selected from the group of halo, unsubstituted C₁-C₄ alkoxy, unsubstituted C₁-C₄ alkyl, hydroxy, amino, nitro, -C(O)OH, and -SO₃H or (iii) unsubstituted or substituted 5- to 10-membered heteroaryl wherein 1 to 4 ring atoms are independently selected from nitrogen, oxygen or sulfur, wherein, when substituted, the substituents are selected from the group consisting of halo, unsubstituted C₁-C₄ alkoxy, unsubstituted C₁-C₄ alkyl, hydroxy, amino, nitro, -C(O)OH, and -SO₃H, and R¹² represents one to four substituents independently selected from the group consisting of hydrogen, halo, amino, unsubstituted C₁-C₄ alkyl or alkoxy, or hydroxy.

In still another specific group of compounds of Formula III, both of A and B are carbonyl, R¹ is amino, R² is tetrazolyl, -S(O)₂OH or -S(O)₂O⁻Na⁺, R⁶ represents one to four substituents independently selected from the group consisting of hydrogen, -S(O)₂OR, -C(O)OR, tetrazolyl, sulfonyl, carboxy, amino, halogen, C₁-C₄ alkoxy, C₁-C₄ alkyl, hydroxy, and nitro, with R as defined above, Y is NH, R⁷ is (i) unsubstituted or substituted C₆ aryl, wherein, if substituted the substituents are selected from the group consisting of halo, unsubstituted C₁-C₄ alkoxy, and hydroxy, or (ii) an unsubstituted or substituted 6-membered heteroaryl wherein 1 to 3 ring atoms are independently selected from nitrogen, oxygen or sulfur, and wherein, if substituted, the substituents are selected from the group consisting of halo, unsubstituted C₁-C₄ alkoxy, and hydroxy, and R¹² is hydrogen.

In another group of compounds of Formula IV, both of A and B are carbonyl, or one is carbonyl and the other is imine (NH), Y is NH, CH₂, O or S, R¹ is amino, R² is tetrazolyl, -S(O)₂OH, -C(O)OH, -S(O)₂O⁻Na⁺, or -C(O)O-Na⁺, R⁶ represents one to four substituents independently selected from the group consisting of hydrogen, -S(O)₂OR, -C(O)OR, tetrazolyl, sulfonyl, carboxy, amino, halogen, C₁-C₄ alkoxy, C₁-C₄ alkyl, hydroxy, and nitro, with R as defined above, R⁸ represents one to five substituents independently selected from the group of unsubstituted C₁-C₁₀ alkyl, alkenyl, alkynyl or alkoxy, hydroxy, mercapto, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, tetrazolyl, amino, and -NRR', with R and R' as defined above, and R¹² represents one to four substituents independently selected from the group consisting of hydrogen, halo, amino, unsubstituted C₁-C₄ alkyl or alkoxy, or hydroxy.

In a specific group of compounds of Formula IV, both of A and B are carbonyl, or one is carbonyl and the other is imine (NH), Y is NH, R¹ is amino, R² is tetrazolyl, -S(O)₂OH, -C(O)OH, -S(O)₂O⁻Na⁺, or -C(O)O⁻Na⁺, R⁶ represents one to four substituents selected from the group consisting of hydrogen, -S(O)₂OR, -C(O)OR, tetrazolyl, sulfonyl, carboxy, amino, halogen, C₁-C₄ alkoxy, C₁-C₄ alkyl, hydroxy, and nitro, with R as defined above, R⁸ represents one to five substituents selected from the group consisting of of halo, unsubstituted C₁-C₄ alkyl or alkoxy, carboxy, sulfonyl, tetrazolyl, amino and hydroxy, and R¹² is hydrogen.

In one embodiment of the invention, a compound of any one of Formulae I-IV is not RB-2 (reactive blue 2).

The compounds of the invention can be administered in pharmaceutical formulations, either alone, or in combination with other pharmacologically active compounds. Suitable other pharmacologically active compounds may, for example, be selected from other P2Y₁₂ inhibitors, such as clopidogrel, or acetylsalicylic acid (Aspirin).

### Utility

The compounds of the present invention bind with high specificity and selectivity to human platelet P2Y₁₂ receptors. Preferably, they bind to their target receptor P2Y₁₂ with at least 10-fold, more preferably at least 100-fold, most preferably 10³-10⁸-fold higher affinity compared to related P2Y receptors, such as, for example, human P2Y₂, human P2Y₄, mouse P2Y₂ and rat P2Y₆.

Upon binding to P2Y₁₂, the compounds of the present invention reduce or abrogate P2Y₁₂ signaling. A precise understanding of the mechanism by which the compounds of the invention inhibit P2Y₁₂ signalling is not required in order to practice the present invention. However, while not hereby bound to any particular mechanism or theory, it is believed that the compounds interact with amino acids of P2Y₁₂ in the ADP binding region or in close proximity thereto, blocking the binding of ADP and thus the activation of the receptor.

Accordingly, the compounds of the present invention are useful as inhibitors of P2Y₁₂ signalling. Because P2Y₁₂ plays a critical role in platelet aggregation, the cross-linking of platelets by fibrin, via the glycoprotein IIb/IIIa pathway, the compounds of the invention have anti-thrombotic activity and thus can be utilized in the treatment of diseases and disorders that are connected to an inappropriate or abnormal P2Y₁₂ function, in particular increased P2Y₁₂ activity, or that involve P2Y₁₂ function. Moreover, the compounds can also be used for the prevention of diseases and disorders that involve platelet aggregation, such as stroke, myocardial infarction, such as non-ST elevation myocardial infarction (NSTEMI), myocardial ischemia, for example manifested in stable or unstable angina, various vascular diseases, such as coronary artery disease, peripheral artery disease, atherosclerosis, and cerebrovascular disease, and embolisms, such as thromboembolisms.

Consequently, the present invention discloses a method of antagonizing P2Y₁₂ function by contacting cells expressing the P2Y₁₂ receptor with a compound of the present invention.

Moreover, the invention also encompasses a method for treating and/or preventing diseases and disorders related to P2Y₁₂ function by administering to a patient in need thereof a therapeutically sufficient amount of at least one of the invented compounds or a pharmaceutical composition containing one or more of these compounds.

In this context, it should be noted that for the efficacy of a compound of the invention as a pharmaceutical a number of variables besides binding affinity may play a crucial role. Accordingly, compounds of the invention can be specifically selected for use as a pharmaceutical not only based on the determined binding specificity and affinity, but also based on other factors, such as bioavailability, severity of side effects caused, metabolic conversion of the compound, half-life of the compound in the organism and the like.

### Administration and Pharmaceutical Composition

A compound of the present invention or a pharmaceutically acceptable salt thereof, can be administered as such to a human patient or can be administered in pharmaceutical compositions in which the foregoing materials are mixed with suitable carriers or excipient(s). Techniques for formulation and administration of drugs may be found in "Remington's Pharmacological Sciences," Mack Publishing Co., Easton, PA., latest edition.

As used herein, "administer" or "administration" refers to the delivery of a compound of Formula (I) or a pharmaceutically acceptable salt thereof or of a pharmaceutical composition containing a compound of Formula (I) or a pharmaceutically acceptable salt thereof of this invention to an organism for the purpose of prevention or treatment of a P2Y₁₂-related disease or disorder.

Suitable routes of administration may include, without limitation, oral, rectal, transmucosal or intestinal administration or intramuscular, subcutaneous, intramedullary, intrathecal, direct intraventricular, intravenous, intravitreal, intraperitoneal, intranasal, or intraocular injections. The preferred routes of administration are oral and parenteral.

Alternatively, one may administer the compound in a local rather than systemic manner, for example, via injection of the compound directly into a vessel, optionally in a depot or sustained release formulation.

Pharmaceutical compositions of the present invention may be manufactured by processes well known in the art, e. g., by means of conventional mixing, dissolving, granulating, drageemaking, levigating, emulsifying, encapsulating, entrapping or lyophilizing processes.

Pharmaceutical compositions for use in accordance with the present invention may be formulated in conventional manner using one or more physiologically acceptable carriers comprising excipients and auxiliaries which facilitate processing of the active compounds into preparations which can be used pharmaceutically. Proper formulation is dependent upon the route of administration chosen.

For injection, the compounds of the invention may be formulated in aqueous solutions, preferably in physiologically compatible buffers such as Hanks' solution, Ringer's solution, or physiological saline buffer. For transmucosal administration, penetrants appropriate to the barrier to be permeated are used in the formulation. Such penetrants are generally known in the art.

For oral administration, the compounds can be formulated by combining the active compounds with pharmaceutically acceptable carriers well known in the art. Such carriers enable the compounds of the invention to be formulated as tablets, pills, lozenges, dragees, capsules, liquids, gels, syrups, slurries, suspensions and the like, for oral ingestion by a patient. Pharmaceutical preparations for oral use can be made using a solid excipient, optionally grinding the resulting mixture, and processing the mixture of granules, after adding other suitable auxiliaries if desired, to obtain tablets or dragee cores. Useful excipients are, in particular, fillers such as sugars, including lactose, sucrose, mannitol, or sorbitol, cellulose preparations such as, for example, maize starch, wheat starch, rice starch and potato starch and other materials such as gelatin, gum tragacanth, methyl cellulose, hydroxypropylmethyl-cellulose, sodium carboxymethylcellulose, and/or polyvinylpyrrolidone (PVP). If desired, disintegrating agents may be added, such as cross-linked polyvinyl pyrrolidone, agar, or alginic acid. A salt such as sodium alginate may also be used.

Dragee cores are provided with suitable coatings. For this purpose, concentrated sugar solutions may be used which may optionally contain gum arable, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical compositions which can be used orally include push-fit capsules made of gelatin, as well as soft, sealed capsules made of gelatin and a plasticizer, such as glycerol or sorbitol. The push-fit capsules can contain the active ingredients in admixture with a filler such as lactose, a binder such as starch, and/or a lubricant such as talc or magnesium stearate and, optionally, stabilizers. In soft capsules, the active compounds may be dissolved or suspended in suitable liquids, such as fatty oils, liquid paraffin, or liquid polyethylene glycols. Stabilizers may be added in these formulations, also.

The compounds may also be formulated for parenteral administration, e. g., by bolus injection or continuous infusion. Formulations for injection may be presented in unit dosage form, e. g., in ampoules or in multi-dose containers, with an added preservative. The compositions may take such forms as suspensions, solutions or emulsions in oily or aqueous vehicles, and may contain formulating materials such as suspending, stabilizing and/or dispersing agents.

Pharmaceutical compositions for parenteral administration include aqueous solutions of a water soluble form, such as, without limitation, a salt, of the active compound.

Additionally, suspensions of the active compounds may be prepared in a lipophilic vehicle. Suitable lipophilic vehicles include fatty oils such as sesame oil, synthetic fatty acid esters such as ethyl oleate and triglycerides, or materials such as liposomes. Aqueous injection suspensions may contain substances which increase the viscosity of the suspension, such as sodium carboxymethyl cellulose, sorbitol, or dextran. Optionally, the suspension may also contain suitable stabilizers and/or agents that increase the solubility of the compounds to allow for the preparation of highly concentrated solutions.

Alternatively, the active ingredient may be in powder form for constitution with a suitable vehicle, e. g., sterile, pyrogen-free water, before use.

The compounds may also be formulated in rectal compositions such as suppositories or retention enemas, using, e. g., conventional suppository bases such as cocoa butter or other glycerides.

In addition to the formulations described previously, the compounds may also be formulated as depot preparations. Such long acting formulations may be administered by implantation (for example, subcutaneously or intramuscularly) or by intramuscular injection. A compound of this invention may be formulated for this route of administration with suitable polymeric or hydrophobic materials (for instance, in an emulsion with a pharmacologically acceptable oil), with ion exchange resins, or as a sparingly soluble derivative such as, without limitation, a sparingly soluble salt.

A non-limiting example of a pharmaceutical carrier for the hydrophobic compounds of the invention is a cosolvent system comprising benzyl alcohol, a nonpolar surfactant, a water-miscible organic polymer and an aqueous phase such as the VPD co-solvent system. VPD is a solution of 3% w/v benzyl alcohol,8% w/v of the nonpolar surfactant Polysorbate 80, and 65% w/v polyethylene glycol 300, made up to volume in absolute ethanol. The VPD co-solvent system (VPD: D5W) consists of VPD diluted 1: with a 5% dextrose in water solution. This cosolvent system dissolves hydrophobic compounds well, and itself produces low toxicity upon systemic administration.

Naturally, the proportions of such a co-solvent system may be varied considerably without destroying its solubility and toxicity characteristics. Furthermore, the identity of the co-solvent components may be varied: for example, other lowtoxicity nonpolar surfactants may be used instead of Polysorbate 80, the fraction size of polyethylene glycol may be varied, other biocompatible polymers may replace polyethylene glycol, e.g., polyvinyl pyrrolidone, and other sugars or polysaccharides may substitute for dextrose.

Alternatively, other delivery systems for hydrophobic pharmaceutical compounds may be employed. Liposomes and emulsions are well known examples of delivery vehicles or carriers for hydrophobic drugs. In addition, certain organic solvents such as dimethylsulfoxide also may be employed, although often at the cost of greater toxicity.

Additionally, the compounds may be delivered using a sustained-release system, such as semipermeable matrices of solid hydrophobic polymers containing the therapeutic agent.

Various sustained-release materials have been established and are well known by those skilled in the art. Sustained-release capsules may, depending on their chemical nature, release the compounds for a few weeks up to over 100 days. Depending on the chemical nature and the biological stability of the therapeutic reagent, additional strategies for stabilization may be employed.

The pharmaceutical compositions herein also may comprise suitable solid or gel phase carriers or excipients. Examples of such carriers or excipients include, but are not limited to, calcium carbonate, calcium phosphate, various sugars, starch, cellulose derivatives, gelatin, and polymers such as polyethylene glycols.

Many of the compounds of the invention may be provided as physiologically acceptable salts wherein the claimed compound may form the negatively or the positively charged species. Examples of salts in which the compound forms the positively charged moiety include, without limitation, the sodium, potassium, calcium and magnesium salts formed by the reaction of a carboxylic acid or sulfonic acid group in the compound with an appropriate base (e.g. sodium hydroxide (NaOH), potassium hydroxide (KOH), Calcium hydroxide (Ca(OH)₂), etc.).

Pharmaceutical compositions suitable for use in the present invention include compositions wherein the active ingredients are contained in an amount sufficient to achieve the intended purpose, e. g., the inhibition of P2Y₁₂ function or the treatment or prevention of a P2Y₁₂-related disease or disorder.

More specifically, a therapeutically effective amount means an amount of compound effective to prevent, alleviate or ameliorate symptoms of disease or prolong the survival of the subject being treated.

Determination of a therapeutically effective amount is well within the capability of those skilled in the art, especially in light of the detailed disclosure provided herein.

For any compound used in the methods of the invention, the therapeutically effective amount or dose can be estimated initially from cell culture assays. Then, the dosage can be formulated for use in animal models so as to achieve a circulating concentration range that includes the IC₅₀ as determined in cell culture (i. e., the concentration of the test compound which achieves a half-maximal inhibition of the P2Y₁₂ activity). Such information can then be used to more accurately determine useful doses in humans.

Toxicity and therapeutic efficacy of the compounds described herein can be determined by standard pharmaceutical procedures in cell cultures or experimental animals, e.g., by determining the IC₅₀ and the LD₅₀ for a subject compound. The data obtained from these cell culture assays and animal studies can be used in formulating a range of dosage for use in humans. The dosage may vary depending upon the dosage form employed and the route of administration utilized. The exact formulation, route of administration and dosage can be chosen by the individual physician in view of the patient's condition.

Dosage amount and interval may be adjusted individually to provide plasma levels of the active species which are sufficient to maintain the P2Y₁₂ inhibiting effects. These plasma levels are referred to as minimal effective concentrations (MECs). The MEC will vary for each compound but can be estimated from in vitro data, e. g., the concentration necessary to achieve 50-90% inhibition of P2Y₁₂.

Dosages necessary to achieve the MEC will depend on individual characteristics and route of administration. HPLC assays or bioassays can be used to determine plasma concentrations.

Dosage intervals can also be determined using MEC value.

Compounds should be administered using a regimen that maintains plasma levels above the MEC for 10-90% of the time, preferably between 30-90% and most preferably between50-90%.

In cases of local administration or selective uptake, the effective local concentration of the drug may not be related to plasma concentration and other procedures known in the art may be employed to determine the correct dosage amount and interval.

The amount of a composition administered will, of course, be dependent on the subject being treated, the severity of the affliction, the manner of administration, the judgment of the prescribing physician, etc.

The compositions may, if desired, be presented in a pack or dispenser device, such as a kit approved by a regulatory authority, such as EMEA or FDA, which may contain one or more unit dosage forms containing the active ingredient. The pack may for example comprise metal or plastic foil, such as a blister pack. The pack or dispenser device may be accompanied by instructions for administration.

The pack or dispenser may also be accompanied by a notice associated with the container in a form prescribed by a governmental agency regulating the manufacture, use or sale of pharmaceuticals, which notice is reflective of approval by the agency of the form of the compositions or of human or veterinary administration. Compositions comprising a compound of the invention formulated in a compatible pharmaceutical carrier may also be prepared, placed in an appropriate container, and labeled for treatment of an indicated condition.

It is also an aspect of this invention that a compound described herein, or its salt or prodrug, might be combined with other agents for the treatment of the diseases and disorders discussed above.

### Synthesis

The most widely employed strategy for the synthesis of anilinonaphthalene or anilinoanthracene derivatives utilizes the Ullmann coupling reaction, which involves the treatment of a naphthalene or anthracene derivative substituted with a leaving group, such as halogen, with an arylamine in the presence of a copper catalyst (cf. Scheme 2, wherein R¹, R², R⁴, R⁵, A, B are defined as above, Z is a leaving group, and R^{x} can be any one to five substituents). As noted above, the reaction typically requires harsh conditions e.g., high temperatures and long reaction times, and suffers from poor yields.

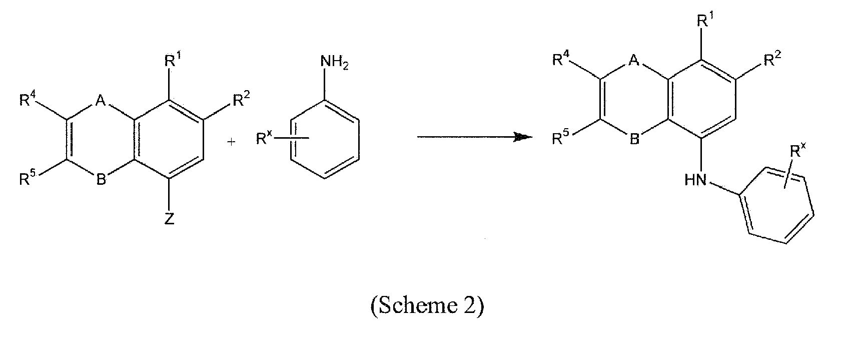

Typical reaction conditions include:
(a) reaction in the presence of CuCl, Na₂CO₃, and Na₂SO₃ in H₂O at room temperature for 8-24 h, or under reflux at 120 °C for 8-10 h (method A);
(b) reaction in the presence of CuSO₄ and Na₂CO₃ in H₂O at 120 °C for 12-48 h (method B); and
(c) reaction in the presence of Cu⁰ in sodium phosphate buffer pH 6-7 at 120 °C for 2-15 h (method C).

However, all three methods suffer from unsatisfactory product yield.

It has been found by the inventors of the present invention that microwave irradiation could improve the Ullmann coupling reactions by increasing product yield and accelerating the reaction. The present invention thus discloses an improved method for the synthesis of compounds of Formulae I-IV, wherein the method includes a microwave-assisted copper-catalyzed Ullmann coupling reaction according to Scheme 1 wherein R¹, R², R³, R⁴, R⁵, Z, R⁶, Y and R⁷ are defined as above.

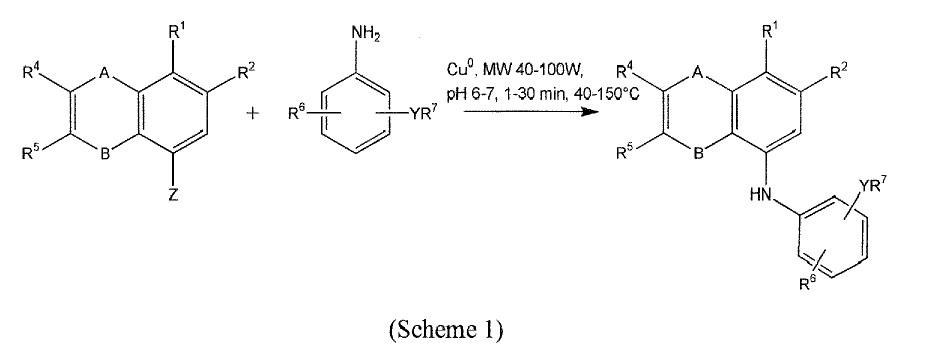

According to the invented method, the reaction mixtures were subjected to microwave irradiation, typically for only 1-30 min at 40-150 °C applying 40-100 W. All other conditions (catalyst, salts, solvent) were the same as in the classical method C. Instead of Cu(0), copper salts (Cu(I) or Cu(II)) can also be used.

The invented method is characterized by dramatically increased yields (30-90 % isolated) especially of compounds poorly accessible without microwaves and a reaction duration of only about 1-30 minutes.

In another aspect, the present invention discloses an improved method for preparing N-substituted 5-nitroanthranilic acids, which are intermediates in the synthesis of the compounds of Formulae I-IV, wherein the method comprises the coupling of a 5-nitro-2-halo-benzoic acid with an amine without a solvent or catalyst under mild microwave reaction conditions.

Hence, this method comprises the preparation of an N-substituted 5-nitroanthranilic acid having the Formula V 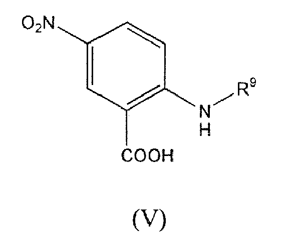 wherein R⁹ is an unsubstituted or substituted C₁-C₁₀ alkyl, unsubstituted or substituted C₃-C₈ cycloalkyl or unsubstituted or substituted C₆-C₁₄ aryl group;
as an intermediate by reacting a 5-nitro-2-halo-benzoic acid having the Formula VI 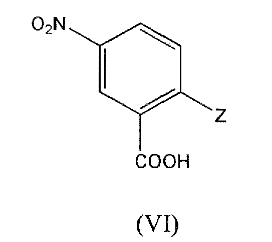 wherein Z is halogen, for example chlorine, bromine or iodine;
with an amine selected from the group of unsubstituted or substituted C₁-C₁₀ alkylamines, unsubstituted or substituted C₃-C₈ cycloalkylamines and unsubstituted or substituted C₆-C₁₄ arylamines without a solvent or catalyst under mild microwave reaction conditions to form the N-substituted 5-nitroanthranilic acid of formula V.

In one embodiment the amine can be an arylamine of Formula VII 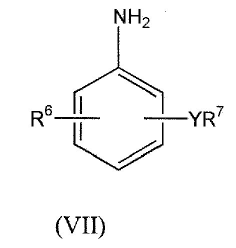 wherein R⁶, Y and R⁷ are defined as above.

The inventions illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims and non-limiting examples. In addition, where features or aspects of the invention are described in terms of Markush groups, those skilled in the art will recognize that the invention is also thereby described in terms of any individual member or subgroup of members of the Markush group.

### Examples

The present inventions will be explained in more detail in the following examples. However, the examples are only used for illustration and do not limit the scope of the present invention.

### Synthetic procedures

**General:** All materials were used as purchased (Sigma-Aldrich or Acros, Germany). Thin-layer chromatography was performed using TLC aluminum sheets silica gel 60 F₂₅₄, or TLC aluminum sheets RP silica gel 18 F₂₅₄ (Merck, Darmstadt, Germany). Colored compounds were visible at daylight; other compounds were visualized under UV light (254 nm). Flash chromatography was performed on a Büchi system using silica gel RP-18 (Merck, Darmstadt, Germany). ¹H- and ¹³C-NMR data were collected on a Bruker Avance 500 MHz NMR spectrometer at 500 MHz (¹H), or 126 MHz (¹³C), respectively. DMSO-d₆ was used as a solvent. Chemical shifts are reported in parts per million (ppm) relative to the deuterated solvent, i. e. DMSO, δ ¹H: 2.49 ppm, ¹³C: 39.7 ppm, coupling constants *J* are given in Hertz and spin multiplicities are given as s (singlet), d (doublet), t (triplet), q (quartet), m (multiplet), br (broad). The purities of isolated products were determined by ESI-mass spectra obtained on an LCMS instrument (Applied Biosystems API 2000 LCMS/MS, HPLC Agilent 1100) using the following procedure: the compounds were dissolved at a concentration of 0.5 mg/ml in H₂O : MeOH = 1 :1, containing 2 mM NH₄CH₃COO. Then, 10 µl of the sample was injected into an HPLC column (Phenomenix Luna 3µ C18, 50 x 2.00 mm). Elution was performed with a gradient of water : methanol (containing 2 mM NH₄CH₃COO) from 90 : 10 to 0 : 100 for 30 min at a flow rate of 250 µl/min, starting the gradient after 10 min. UV absorption was detected from 200 to 950 nm using a diode array detector. Purity of the compounds was determined at 254 nm. For microwave reactions a CEM Focused^{™} Microwave Synthesis type Discover apparatus was used. A freeze dryer (CHRIST ALPHA 1-4 LSC) was used for lyophilization, reduction was achieved by using a Hogen^{®} GC hydrogen generator from the Proton Energy System (USA) company.

### Example 1: General synthesis procedure

### Procedure A: Ullmann coupling reactions of bromaminic acid or related derivatives with an aryl amine or an aliphatic amine or another amino-substituted compound

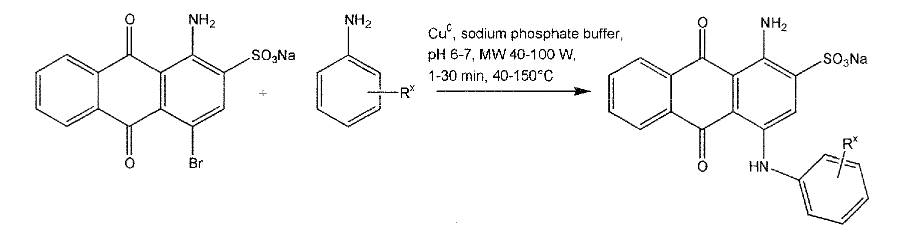

To a 5 mL microwave reaction vial equipped with a magnetic stirring bar were added a bromo-substituted phenyl derivative or another mono-, bi- or tricyclic bromo-substituted compound (e.g. bromaminic acid sodium salt) (0.20 mmol) and the appropriate aniline derivative or other amino-substituted compound (0.40 mmol), followed by a buffer solution of Na₂HPO₄ (pH 9.6) (4 mL) and NaH₂PO₄ (pH 4.2) (1 mL). A catalytic amount (e.g. 0.002-0.003 g) of finely powdered elemental copper (or copper(I)-salt, or copper(II)-salt, respectively) was added. The mixture was capped and irradiated in the microwave oven (e.g. 40-100 W) for 1-30 min at 40-150 °C. Then the reaction mixture was cooled to rt, and the product was purified using the following procedure. The contents of the vial were filtered to remove the elemental copper. Then ca. 200 mL of water was added to the filtrate and the aqueous solution was extracted with chloroform (200 mL). The extraction procedure was repeated until the chloroform layer became colorless (2-3 times). Then the aqueous layer was reduced by rotary evaporation to a volume of 10-20 mL, which was subsequently submitted to flash column chromatography using RP-18 silica gel and water as an eluent. The polarity of the eluent was then gradually decreased by the addition of methanol in the following steps: 20, 40, 60, 80, and 100 %. Fractions containing blue product were collected. For some compounds the last step of purification (RP-18 flash chromatography) had to be repeated two to three times to obtain pure product (≥ 95% purity as determined by LC-MS). The pooled product-containing fractions were evaporated under vacuum to remove the methanol, and the remaining water was subsequently removed by lyophilization to yield the products (30-90%) as blue powders.

According to the following reaction scheme the results shown in Table 2 were obtained.

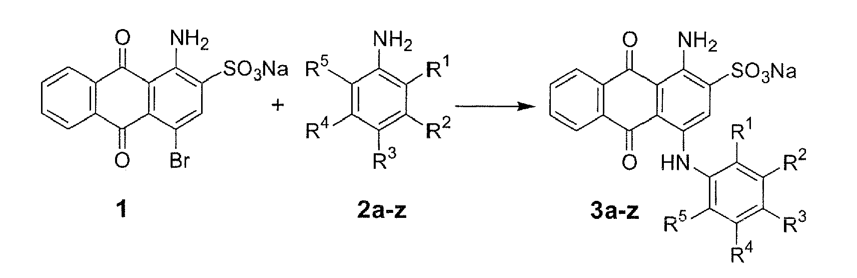

**Table 2**

| entry | R¹ | R² | R³ | R⁴ | R⁵ | products 3 (yield [%])b |
|---|---|---|---|---|---|---|
| 1 | H | COOH | H | NH₂ | H | 3a (70) |
| 2 | H | COOH | H | H | H | 3b (76) |
| 3 | H | H | COOH | H | H | 3c (70) |
| 4 | NH₂ | H | H | H | H | 3d (58) |
| 5 | H | NH₂ | H | H | H | 3e (70) |
| 6 | COOH | H | Cl | H | H | 3f (43) |
| 7 | COOH | H | H | Cl | H | 3g (64) |
| 8 | COOH | H | H | H | H | 3h (83) |
| 9 | OH | H | H | H | H | 3i (58) |
| 10 | H | H | OH | H | H | 3j (72) |
| 11 | H | H | NH₂ | H | H | 3k (90) |
| 12 | H | H | H | H | H | 31 (55) |
| 13 | H | H | Cl | H | H | 3m (87) |
| 14 4 | H | H | 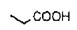 | H | H | 3n (44) |
| 15 | CH₃ | H | CH₃ | H | CH₃ | 3o(77) |
| 16 | OMe | H | H | H | H | 3p (79) |
| 17 | H | OMe | H | H | H | 3q (67) |
| 18 | H | H | OMe | H | H | 3r (85) |
| 19 | H | H | F | H | H | 3s (76) |
| 20 | H | Br | H | H | H | 3t (41) |
| 21 | H | CH₃ | H | CH₃ | H | 3u (32) |
| 22 | H | Cl | H | H | H | 3v (56) |
| 23 | CH₃ | NH₂ | H | H | H | 3w (34) |
| 24 | H | COOH | OH | H | H | 3x (30) |
| 25 | SO₃H | H | H | H | H | 3y (30) |
| 26 | H | H | SO₃H | H | H | 3z (40) |

| | | | | | | |
|---|---|---|---|---|---|---|
| *^{a}*Reaction mixtures were irradiated for 2 to 20 min in sodium phosphate buffer, pH 6-7, in the presence of Cu at 80-120 °C. Isolated yields (purity of products ≥ 95% as determined by HPLC-LTV (254 mn ESI-MS) calculated based on educt 1; true yields were higher since commercial 1 was only 90% pure (main contaminant: desbromo derivative, 8%). | | | | | | |

For some selected compounds the obtained product yields were compared with those obtained by the conventional method C, which was proven superior to methods A and B (See above). The results of this comparison, shown in Figure 1, clearly demonstrate that the method of the present invention leads to improved product yields compared to the conventional method C.

### Specific Example for procedure A

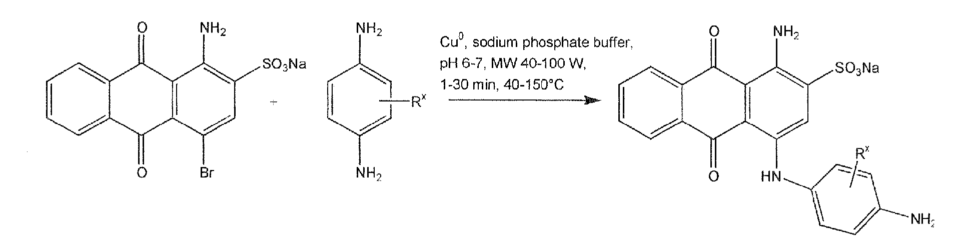

### Procedure B: Preparation of compound 4

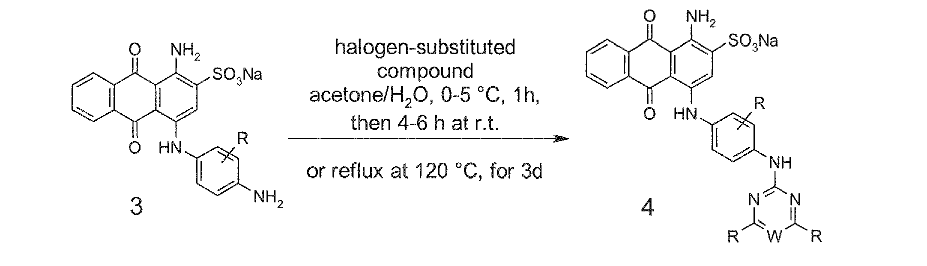

An ice-cooled solution of an halogen-substituted derivative (0.5-1 mmol) in water (25 ml) and acetone (25 ml) was added to a stirred solution of compound 3 (0.5 mmol) in water (25 ml) at 0-5 °C. The resulting mixture was stirred for 1 h at 0-5 °C, then at room temperature for 4-6 h. The formation of product was monitored by RP-TLC using a mobile phase of acetone/water (2:3). After completion of the reaction the solvents were evaporated and the residue was purified by FCC RP-18 silica gel using an acetone/water eluent to obtain compound 4 (or analogs).

### Procedure C: Alternative preparation of compound 4

An ice-cooled solution of 2-bromopyrimidine or another halogen compound (0.5-1 mmol) in water (25 ml) and acetone (25 ml) was added to a stirred solution of the respective compound 3 (0.5 mmol) in water (25 ml) at 0-5 °C. Then the temperature was graduelly increased for refluxing at 120 °C for 1 -3d. The formation of product was monitored by RP-TLC using a mobile phase of methanol/water (2:3). After completion of the reaction the solvents were evaporated and the residue was purified by FCC RP-18 silica gel using an methanol/water eluent to obtain compound **4.**

### General procedure D: Preparation of compound 5

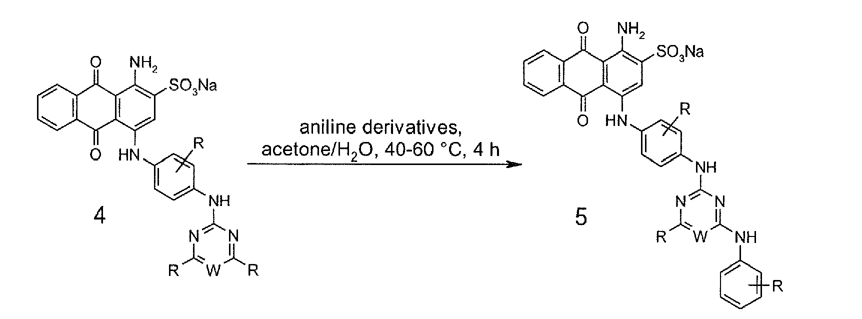

### Example for the preparation of products 5

An ice-cooled solution of an aniline derivative or another amino-substituted compound (1-2 mmol) in water (25 ml) and acetone (25 ml) was added to a stirred solution of the compound **4** (1 mmol) in water (25 ml) at 0-5 °C. Then the temperature was graduelly increased to reach 40-60 °C for 4 h. The formation of product was monitored by RP-TLC using a mobile phase of acetone/water (3:2). After completion of the reaction the solvents were evaporated and the residue was purified by FCC RP-18 silica gel using an acetone/water eluent to obtain compound **5.**

### General procedure E: Synthesis of intermediate compounds having Formula VIII

To an 80 mL microwave reaction vial equipped with a magnetic stirring bar were added 5-nitro-2-chlorobenzoic acid (2.520 g, 12.5 mmol) and an excess (e.g. 6 eq.) of the appropriate aliphatic or aromatic amine derivative (ca. 75 mmol) to obtain a homogenous mixture. The mixture was irradiated in a microwave oven (80-100 W) for 5-30 min at 80-120 °C. Then the reaction mixture was cooled to rt, and the product was purified using the following procedure. The contents of the vial were dissolved in ca. 500 mL of dichloromethane and the organic solution was extracted with diluted aq. sodium hydroxide solution (ca. 500 mL). The extraction procedure was repeated until the aqueous layer became light yellow (3-4 times). Then the aqueous layer was collected and acidified using concentrated aq. hydrochloric acid (37 %) until pH ≤ 3 and the desired product precipitated in the acidic medium. The precipitated solid was filtered off and washed several times (2-3 times) with 100 mL water each to remove the remaining sodium chloride as well as hydrochloric acid. The product was dried in an oven at 100 °C. The purity of the product was ≥95% as determined by LC-MS.

### General procedure F: Reduction of the intermediate nitro derivative of Formula VIII

To a 10 mL vial equipped with a magnetic stirring bar were added the nitro-substituted compound **(VIII,** 1 mmol) followed by a buffer solution of Na₂HPO₄ (pH 9.6) (4 mL) and NaH₂PO₄ (pH 4.2) (1 mL). A catalytic amount (ca. 0.005-0.010 g) of 10% palladium on activated charcoal, finely powdered, was added. The mixture was hydrogenated at 40-60 psi for 3-6 h. The resulting mixture was used directly for the next step without prior purification.

### General procedure G: Coupling reaction of the reduction products of general procedure F with halogen-substituted compounds

To a 5 mL microwave reaction vial equipped with a magnetic stirring bar were added bromaminic acid sodium salt, or another halogen-substituted compound, (0.20 mmol) and the mixture obtained by general procedure F without further purification, followed by a buffer solution of Na₂HPO₄ (pH 9.6) (4 mL) and NaH₂PO₄ (pH 4.2) (1 mL). A catalytic amount (ca. 0.004-0.006 g) of finely powdered elemental copper was added. The mixture was irradiated in a microwave oven (100 W) for 5 min at 120 °C, or according to the general procedure **A.** The purification of the compounds was the same as described in general procedure **A.**

### Example 2: Sodium 1-amino-4-(3-amino-5-carboxyphenylamino)-9,10-dioxo-9,10-dihydroanthracene 2-sulfonate.

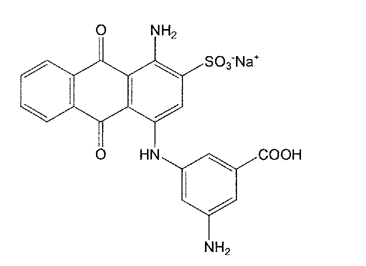

**Reaction conditions:** according to the general procedure A: 20 min, 120 °C, 100 W; pressure up to 10 bar.
**Analytical data: mp** > 300 °C, blue powder. **¹H-NMR:** δ 5.29 (bs, 2H, 3'-NH₂), 6.53 (dd, 1H, 6'-H), 7.03 (d, 1H, 2'-H), 7.08 (d, 1H, 4'-H), 7.83 (m, 2H, 6-H, 7-H), 7.96 (s, 1H, 3-H), 8.26 (m, 2H, 5-H, 8-H), 10.13 (br, 2H, 1-NH2), 12.04 (s, 1H, 4-NH). **^{13C}-NMR**: δ 109.05 (C-9a), 110.75 (C-4a), 110.93, 112.33, 112.51 (C-2', C-4', C-6'), 123.40 (C-3), 126.07 (C-5), 126.15 (C-8), 132.86 (C-6), 133.17 (C-7), 133.83 (C-10a), 134.29 (C-8a), 139.11 (C-1'), 142.12 (C-4), 142.94 (C-2, C-5'), 144.35 (C-1), 149.77 (C-3'), 172.50 (COOH), 181.78 (C-9), 182.14 (C-10). **LC-MS (m/z):** 471 [M-Na+NH₄⁺]⁺, 454 [M-Na]⁺, 452 [M-Na]⁻. Purity by **HPLC-UV (254 nm)-ESI-MS**: 95%.

### Example 3: Sodium 1-amino-4-(3-carboxyphenylamino)-9,10-dioxo-9,10-dihydroanthracene 2-sulfonate.

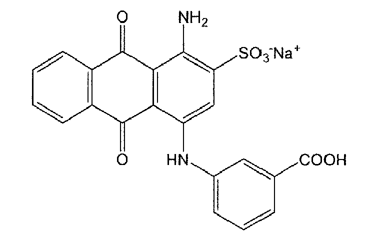

**Reaction conditions:** according to the general procedure A: 20 min, 120 °C, 80 W; pressure up to 10 bar.
**Analytical data:** mp > 300 °C, blue powder. **¹H-NMR:** δ 7.38 (dd, 1H, 6'-H), 7.47 (dd, 1H, 5'-H), 7.76 (dd, 1H, 4'-H), 7.84 (m, 3H, 2'-H, 6-H, 7-H), 7.97 (s, 1H, 3-H), 8.26 (m, 2H, 5-H, 8-H), 10.00 (br, 2H, 1-NH₂), 12.69 (s, 1H, 4-NH). **¹³C-NMR:** δ 109.35 (C-9a), 111.72 (C-4a), 122.81 (C-3), 124.05 (C-2'), 125.45 (C-5), 125.62 (C-8), 126.11, 126.19 (C-4', C-6'), 129.38 (C-5'), 132.94 (C-6), 133.35 (C-7), 133.72 (C-10a), 134.30 (C-8a), 139.18 (C-1'), 140.95 (C-4), 142.91 (C-2), 144.51 (C-1), 145.21 (C-3'), 169.63 (COOH), 182.00 (C-9), 182.71 (C-10). **LC-MS (m/z):** 439 [M-Na]⁺, 437 [M-Na]⁻_{.} Purity by **HPLC-UV (254 nm)-ESI-MS:** 96%.

### Example 4: Sodium 1-amino-4-(4-carboxyphenylamino)-9,10-dioxo-9,10-dihydroanthracene 2-sulfonate.

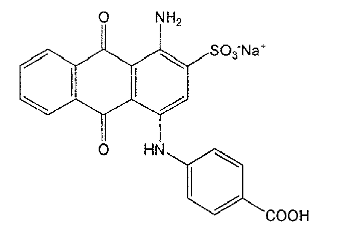

**Reaction conditions:** according to the general procedure A: 20 min, 120 °C, 80 W; pressure up to 10 bar.
**Analytical data:** mp > 300 °C, blue powder. ¹H-NMR: δ 7.17 (d, 2H, 2'-H, 6'-H), 7.84 (m, 2H, 6-H, 7-H), 7.93 (d, 2H, 3'-H, 5'-H), 8.07 (s, 1H, 3-H), 8.26 (m, 2H, 5-H, 8-H), 10.22 (br, 2H, 1-NH₂), 12.07 (s, 1H, 4-NH). ¹³**C-NMR:** 109.28 (C-9a), 111.62 (C-4a), 121.43 (C-2', C-6'), 123.11 (C-3), 126.11 (C-5), 126.17 (C-8), 130.64 (C-3', C-5'), 132.91 (C-6), 133.29 (C-7), 133.74 (C-10a), 134.29 (C-8a), 136.59 (C-4'), 139.77 (C-1'), 140.78 (C-4), 142.85 (C-2), 144.53 (C-1),169.78 (COOH), 181.93 (C-9), 182.56 (C-10). **LC-MS** (m/z): 439 [M-Na]⁺, 437 [M-Na]⁻. Purity by **HPLC-UV (254 nm)-ESI-MS**: 98%.

### Example 5: Sodium 1-amino-4-(2-aminophenylamino)-9,10-dioxo-9,10-dihydroanthracene 2-sulfonate.

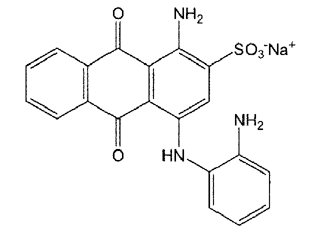

**Reaction conditions:** according to the general procedure A: 20 min, 120 °C, 80 W; pressure up to 10 bar.
**Analytical data:** mp > 300 °C, blue powder. **¹H-NMR:** δ 5.02 (bs, 2H, 2'-NH₂), 6.62 (ddd, 1H, 4'-H), 6.84 (dd, 1H, 3'-H), 7.01 (m, 2H, 5'-H, 6'-H), 7.61 (s, 1H, 3-H), 7.83 (m, 2H, 6-H, 7-H), 8.27 (m, 2H, 5-H, 8-H), 10.15 (br, 2H, 1-NH₂), 11.67 (s, 1H, 4-NH). ¹³C-**NMR:** 109.10 (C-9a), 110.58 (C-4a), 115.77, 116.72 (C-4', C-5'), 123.22 (C-3), 123.70, 126.01, 126.12, 126.64 (C-5, C-8, C-6', C-3'), 127.06 (C-1'), 132.71 (C-6), 132.93 (C-7), 133.96 (C-10a), 134.33 (C-8a), 143.08 (C-4), 143.66 (C-2), 144.26(C-1), 144.28 C-2'), 181.67 (C-9), 181.92 (C-10). **LC-MS** (m/z): 410 [M-Na]⁺, 408 [M-Na]⁻. Purity by **HPLC-UV (254 nm)-ESI-MS:** 95%.

### Example 6: Sodium 1-amino-4-(3-aminophenylamino)-9,10-dioxo-9,10-dihydroanthracene 2-sulfonate.

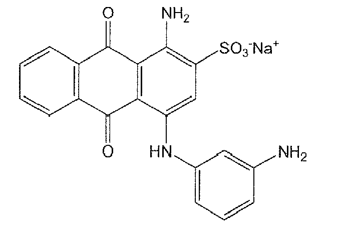

**Reaction conditions:** according to the general procedure A: 2 min, 120 °C, 80 W; pressure up to 10 bar.
**Analytical data:** mp > 300 °C, blue powder. **¹H-NMR:** δ 5.28 (bs, 2H, 3'- NH₂), 6.42 (m, 3H, 2'-H, 4'-H, 6'-H), 7.06 (dd, 1H, 5'-H), 7.83 (m, 2H, 6-H, 7-H), 8.02 (s, 1H, 3-H), 8.26 (m, 2H, 5-H, 8-H), 10.12 (br, 2H, 1-NH₂), 12.03 (s, 1H, 4-NH). ¹³C-NMR: 108.69, 109.02, 110.76, 110.83, 110.95 (C-9a, C-4a, C-2', C-4', C-6'), 123.39 (C-3), 126.05 (C-5), 126.13 (C-8), 130.06 (C-5'), 132.79 (C-6), 133.10 (C-7), 133.82 (C-10a), 134.29 (C-8a), 139.76 (C-1'), 141.86 (C-4), 143.03 (C-2), 144.39 (C-1), 150.27 (C-3'), 181.74 (C-9), 182.11 (C-10). **LC-MS** (m/z): 410 [M-Na]⁺, 408 [M-Na]⁻. Purity by **HPLC-UV (254 nm)-ESI-MS:** 95%.

### Example 7: Sodium 1-amino-4-(2-carboxy-4-chlorophenylamino)-9,10-dioxo-9,10-dihydro-anthracene 2-sulfonate.

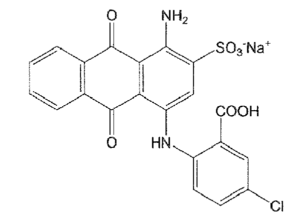

**Reaction conditions:** according to the general procedure A: 5 min, 120 °C, 80 W; pressure up to 10 bar.
**Analytical data:** mp > 300 °C, blue powder. **¹H-NMR:** δ 7.13 (d, 1H, 6'-H), 7.29 (dd, 1H, 5'-H), 7.81 (m, 2H, 6-H, 7-H), 7.90 (d, 1H, 3'-H), 8.11 (s, 1H, 3-H), 8.22 (m, 2H, 5-H, 8-H), 9.97 (br, 2H, 1-NH₂), 12.72 (s, 1H, 4-NH). ¹³**C-NMR:** δ 110.28 (C-9a), 115.81 (C-4a), 121.00 (C-3), 124.35, 126.08, 126.14 (C-2', C-4', C-6'), 125.43 (C-5, C-8), 129.50, 131.15 (C-5', C-3'), 32.92 (C-6), 133.23 (C-7), 133.93 (C-10a), 134.20 (C-8a), 136.71 (C-1'), 140.04 (C-4), 141.22 (C-2) 144.91 (C-1), signal for COOH not detectable (too broad and flat), 181.80 (C-9), 182.54 (C-10). **LC-MS** (m/z): 439 [M-Na]⁺, 437 [M-Na]⁻. Purity by **HPLC-UV (254 nm)-ESI-MS:** 95%.

### Example 8: Sodium 1-amino-4-(2-carboxy-5-chiorophenylammo)-9,10-dioxo-9,10-dihydro-anthracene 2-sulfonate.

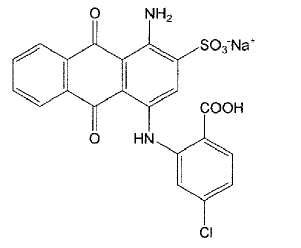

**Reaction conditions:** according to the general procedure **A:** 5 min, 120 °C, 80 W; pressure up to 10 bar.
**Analytical data:** mp > 300 °C, blue powder. ¹**H-NMR:** δ 6.92 (d, 1H, 6'-H), 7.05 (d, 1H, 4'-H), 7.70 (d, 1H, 3'-H), 7.80 (m , 2H, 6-H, 7-H), 8.12 (s, 1H, 3-H), 8.21 (m, 2H, 5-H, 8-H), 9.93 (br, 2H, 1-NH₂), 12.65 (s, 1H, 4-NH). ¹³**C-NMR:** δ 110.58 (C-9a), 117.22, 117.50 (C-4a, C-2', C-4'), 120.02 (C-3), 125.90 (C-5'), 126.13, 126.19 (C-6', C-5, C-8) 132.91 (C-6), 133.48 (C-7), 133.78 (C-10a), 134.18 (C-8a), 135.05, 135.39 (C-3', C-1'), 141.01 (C-4), 143.26 (C-2), 145.21 (C-1), signal for COOH not detectable (too broad and flat), 182.36 (C-9), 182.69 (C-10). **LC-MS** (m/z): 473 [M-Na]⁺, 471 [M-Na]⁻. Purity by **HPLC-UV (254 nm)-ESI-MS:** 95%.

### Example 9: Sodium 1-amino-4-(2-carboxyphenylamino)-9,10-dioxo-9,10-dihydroanthracene 2-sulfonate.

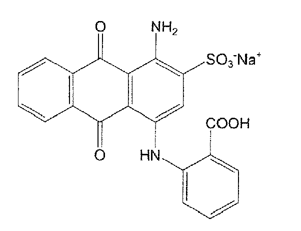

**Reaction conditions:** according to the general procedure A: 5 min, 120 °C, 80 W; pressure up to 10 bar.
**Analytical data:** mp > 300 °C, blue powder. ¹**H-NMR:** 6.67 (dd, 1H, 4'-H), 7.13 (d, 1H, 6'-H), 7.30 (d, 1H, 5'-H), 7.80 (m, 2H, 6-H, 7-H), 8.15 (s, 1H, 3-H), 8.23 (m, 2H, 5-H, 8-H), 10,00 (br, 2H, 1-NH₂), 12.69 (s, 1H, 4-NH). ¹**³C-NMR**: 110.12 (C-9a), 115.18 (C-4a), 119.75 (C-2'), 121.24 (C-3), 125.31, 126.06, 126.21 (C-6', C-4', C-5, C-8), 130.73, 131.88 (C-5', C-3'), 132.76 (C-6), 133.20 (C-7), 133.91 (C-10a), 134.23 (C-8a), 137.34 (C-1'), 140.98 (C-4), 141.40 (C-2), 144.89 (C-1), signal for COOH not detectable (too broad and flat), 181.88 (C-9), 182.37 (C-10). **LC-MS (m/z)**: 439 [M-Na]⁺, 437 [M-Na]⁻. Purity by HPLC-UV **(254 nm)-ESI-MS:** 95%.

### Example 10: Sodium 1-amino-4-(2-hydroxyphenylamino)-9,10-dioxo-9,10-dihydroanthracene 2-sulfonate.

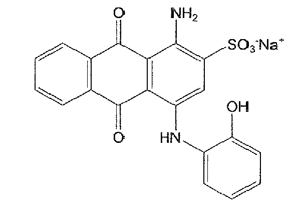

**Reaction conditions:** according to the general procedure **A**: 10 min, 120 °C, 80 W; pressure up to 10 bar.
**Analytical data:** mp > 300 °C, blue powder. ¹**H-NMR:** δ 6.86 (ddd, 1H, 4'-H), 6.99 (dd, 1H, 6'-H), 7.04 (ddd, 1H, 5'-H), 7.21 (dd, H, 3'-H), 7.83 (m, 2H, 6-H, 7-H), 7.93 (s, 1H, 3-H), 8.27 (m, 2H, 5-H, 8-H), 9.87 (br, 2H, 1-NH₂), 10.15 (br, 1H, 2'-OH), 11.92 (s, 1H, 4-NH). **¹³C-NMR:** 109.19 (C-9a), 110.97 (C-4a), 116.30, 119.55, 123.24, 123.91, 125.60, 126.03, 126.12, 126.67 (C-1', C-3', C-4', C-5', C-6', C-3, C-5, C-8), 132.79 (C-6) 133.23 (C-7), 133.88 (C-10a), 134.28 (C-8a), 141.66 (C-4), 142.78 (C-2), 144.29 (C-1), 150.65 (C-2'), 181.81 (C-9), 181.99 (C-10). **LC-MS (m/z)**: 411 [M-Na]⁺, 409 [M-Na]⁻. Purity by **HPLC-UV (254 nm)-ESI-MS:** 96%.

### Example 11: Sodium 1-amino-4-(4-hydroxyphenylamino)-9,10-dioxo-9,10-dihydroanthracene 2-sulfonate.

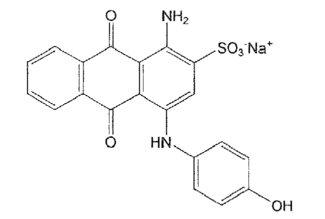

**Reaction conditions:** according to the general procedure A: 10 min, 120 °C, 80 W; pressure up to 10 bar.
**Analytical data:** mp > 300 °C, blue powder. **¹H-NMR:** δ 6.82 (d, 2H, 2'-H, 6'-H), 7.08 (d, 2H, 3'-H, 5'-H), 7.81 (s, 1H, 3-H), 7.83 (m, 2H, 6-H, 7-H), 8.27 (m, 2H, 5-H, 8-H), 10.15 (br, 2H, 1-NH₂), 12.05 (s, 1H, 4-NH). **¹³C-NMR:** δ 108.97 (C-9a), 110.03 (C-4a), 116.39 (C-2', C6'), 122.93 (C-3), 125.98 (C-5), 126.13 (C-8), 126.36 (C-3', C5'), 129.98 (C-1'), 132.75 (C-6), 132.96 (C-7), 133.90 (C-10a), 134.29 (C-8a), 143.22 (C-4), 143.25 (C-2), 144.19 (C-1), 155.52 (C-4'), 181.69 (C-9), 181.70 (C-10). LC-MS (m/z): 411 [M-Na]⁺, 409 [M-Na]⁻. Purity by **HPLC-UV (254 nm)-ESI-MS:** 95%.

### Example 12: Sodium 1-amino-4-(4-aminophenylaniino)-9,10-dioxo-9,10-dihydroanthracene 2-sulfonate.

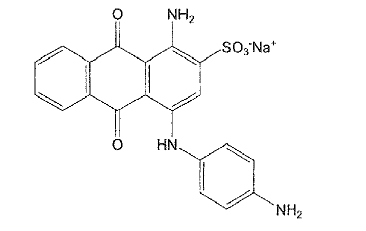

**Reaction conditions:** according to the general procedure A: 5 min, 100 °C, 60 W; pressure up to 10 bar.
**Analytical data:** mp > 300 °C, blue powder. **¹H-NMR:** :δ 5.17 (bs, 2H, 4'-NH₂), 6.64 (d, 2H, 2'-H, 6'-H), 6.94 (d, 2H, 3'-H, 5'-H), 7.81 (s, 1H, 3-H), 7.82 (m, 2H, 6-H, 7-H), 8.27 (m, 2H, 5-H, 8-H), 10.17 (br, 2H, 1-NH₂), 12.09 (s, 1H, 4-NH). **¹³C-NMR:** δ 108.84 (C-9a), 109.52 (C-4a), 114.78 (C-2', C6'), 122.85 (C-3), 125.93 (C-5), 126.09 (C-8), 126.14, 126.82 (C-3', C5', C-1'), 132.65 (C-6), 132.75 (C-7), 133.99 (C-10a), 134.26 (C-8a), 143.24 (C-4), 143.88 (C-2), 144.15 (C-1), 147.08 (C-4'), 181.17 (C-9), 181.52 (C-10). LC-**MS (m/z):** 432 [M]⁺, 427 [M-Na+NH₄⁺]⁺, 410 [M-Na]⁺, 408 [M-Na]⁻. Purity by HPLC-**UV (254 nm)-ESI-MS:** 95%.

### Example 13: Sodium 1-amino-9,10-dioxo-4-phenylamino-9,10-dihydroanthracene 2-sulfonate.

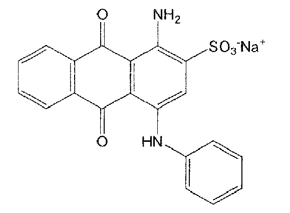

**Reaction conditions:** according to the general procedure A: 5 min, 80 °C, 40 W; pressure up to 10 bar.
**Analytical data:** mp > 300 °C, blue powder**. ¹H-NMR:** δ 7.20 (ddd, 1H, 4'-H), 7.28 (dd, 2H, 2'-H, 6'-H), 7.44 (ddd, 2H, 3'-H, 5'-H), 7.84 (m, 2H, 6-H, 7-H), 8.02 (s, 1H, 3-H), 8.27 (m, 2H, 5-H, 8-H), 10.10 (br, 2H, 1-NH₂), 12.04 (s, 1H, 4-NH). **¹³C-NMR:** δ 109.30 (C-9a), 111.56 (C-4a), 122.82 (C-3), 123.20 (C-2', C6'), 124.58 (C-4'), 126.09 (C-5), 126.17 (C-8), 129.84 (C-3', C5'), 132.90 (C-6), 133.30 (C-7), 133.72 (C-10a), 134.29 (C-8a), 139.41 (C-1'), 140.93 (C-4), 142.95 (C-2), 144.50 (C-1), 181.94 (C-9), 182.62 (C-10). **LC-MS** (m/z): 395 [M-Na]⁺, 393 [M-Na]⁻. Purity by **HPLC-UV (254 nm)-ESI-MS:** 98%.

### Example 14: Sodium 1-amino-4-(4-chlorophenylaniino)-9,10-dioxo-9,10-dihydroanthracene 2-sulfonate.

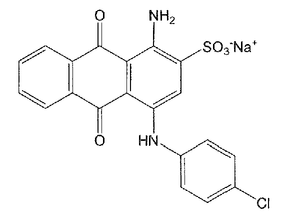

**Reaction conditions:** according to the general procedure A: 5 min, 100 °C, 80 W; pressure up to 10 bar.
**Analytical data:** mp > 300 °C, blue powder. ¹**H-NMR:** δ 7.30 (d, 2H, 2'-H, 6'-H), 7.47 (d, 2H, 3'-H, 5'-H), 7.85 (m, 2H, 6-H, 7-H), 7.96 (s, 1H, 3-H), 8.26 (m, 2H, 5-H, 8-H), 10.05 (br, 2H, 1-NH₂), 11.87 (s, 1H, 4-NH). ¹**³C-NMR:** 0109.46 (C-9a), 112.24 (C-4a), 122.79 (C-3), 124.61 (C-2', C6'), 126.08 (C-2'), 126.20 (C-5), 126.20 (C-8), 128.18 (C-4'), 129.70 (C-3' and C-5'), 133.00 (C-6), 133.47 (C-7), 133.63 (C-10a), 134.28 (C-8a), 138.66 (C-1'), 140.15 (C-4), 142.80 (C-2), 144.59 (C-1), 182.08 (C-9), 182.94 (C-10). **LC-MS (m/z):** 446 [M-Na+NH₄⁺]⁺, 429 [M-Na]⁺, 427 [M-Na]⁻. Purity by **HPLC-UV (254** nm)-**ESI-MS:** 96%.

### Example 15: Sodium 1-ammo-4-(4-carboxymethylphenylamino)-9,10-dioxo-9,10-dihydro-anthracene 2-sulfonate.

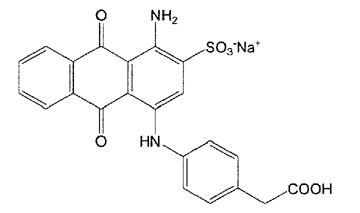

**Reaction conditions:** according to the general procedure A: 10 min, 100 °C, 80 W; pressure up to 10 bar.
**Analytical data:** mp > 300 °C, blue powder. **¹H-NMR:** δ 3.24 (s, 2H, CH₂), 7.12 (d, 2H, 2'-H, 6'-H), 7.30 (d, 2H, 3'-H, 5'-H), 7.84 (m, 2H, 6-H, 7-H), 7.99 (s, 1H, 3-H), 8.27 (m, 2H, 5-H, 8-H), 10.12 (br, 2H, 1-NH₂), 12.12 (s, 1H, 4-NH). ¹³C-NMR: δ 45.83 (CH₂), 109.11 (C-9a), 110.75 (C-4a), 122.78 (C-3), 122.99 (C-2', C6'), 126.05 (C-5), 126.15 (C-8), 130.49 (C-3', C-5'), 132.82 (C-6), 133.11 (C-7), 133.83 (C-10a) 134.30 (C-8a), 136.00, 137.11 (C-4', C-1'), 141.93 (C-4), 143.10 (C-2), 144.38 (C-1), 174.50 (COOH), 181.78 (C-9), 182.10 (C-10). LC-MS (m/z): 470 [M-Na+NH₄⁺]⁺, 453 [M-Na]⁺, 451 [M-Na]⁻. Purity by **HPLC-UV (254 nm)-ESI-MS** : 95%.

### Example 16: Sodium 1-amino-9,10-dioxo-4-(2,4,6-trimethylphenylamino)-9,10-dihydroanthracene 2-sulfonate.

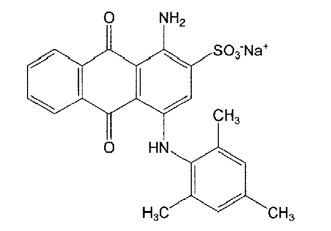

**Reaction conditions:** according to the general procedure A: 5 min, 120 °C, 100 W; pressure up to 10 bar.
**Analytical data:** mp > 300 °C, blue powder. ¹**H-NMR:** δ 2.10 (s, 6H, 2'-CH₃, 6'CH₃), 2.30 (s, 3H, 4'-CH₃), 7.03 (s, 2H, 3'-H, 5'-H), 7.13 (s, 1H, 3-H), 7.84 (m, 2H, 6-H, 7-H), 8.29 (m, 2H, 5-H, 8-H), 10.10 (br, 2H, 1-NH₂), 11.73 (s, 1H, 4-NH). ¹³C-NMR: δ 20.71 (C4'-CH₃), 18.10 (C2'-CH₃, C3'-CH₃), 109.20 (C-9a), 109.49 (C-4a), 121.92 (C-3), 126.04 (C-5), 126.16 (C-8), 129.38 (C-3' and C-5'), 132.77 (C-6), 133.02 (C-7), 133.43 (C-4'), 133.82 (C-10a), 134.32 (C-8a), 135.30 (C-2', C6'), 136.20 (C-1'), 143.59 (C-4), 143.86 (C-2), 144.08 (C-1), 181.77 (C-9), 182.18 (C-10). LC-MS (m/z): 454 [M-Na+NH₄⁺]⁺, 437 [M-Na]⁺, 435 [M-Na]⁻. Purity by HPLC-UV (254 nm)-ESI-1VIS: 95%.

### Example 17: Sodium 1-amino-4-(2-methoxyphenylamino)-9;10-dioxo-9,10-dihydroanthracene 2-sulfonate.

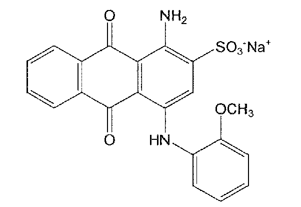

**Reaction conditions:** according to the general procedure A: 5 min, 100 °C, 80 W; pressure up to 10 bar.
**Analytical data:** mp > 300 °C, blue powder. ¹**H-NMR:** δ 3.86 (s, 3H, 3'-OCH₃), 7.01 (ddd, 1H, 4'-H), 7.17 (m, 2H, 5'-H, 6'-H), 7.29 (dd, 1H, 3 '-H), 7.84 (m, 2H, 6-H, 7-H), 7.96 (s, 1H, 3-H), 8.27 (m, 2H, 5-H, 8-H), 10.10 (br, 2H, 1-NH₂), 11.92 (s, 1H, 4-NH). ¹³**C-NMR:δ** 55.93 (C3'-OCH₃), 109.31 (C-9a), 111.56 (C-4a), 112.38, 120.93, 122.88, 123.06 (C-3', C6', C-3 C-4'), 125.32 (C-5), 126.11 (C-8), 126.15 (C-5'), 128.07 (C-6), 132.86 (C-7), 133.20 (C-10a), 133.80 (C-8a), 134.29 (C-1'), 140.81 (C-4), 142.72 (C-2), 144.38 (C-1), 151.99 (C-2'), 181.92 (C-9), 182.37 (C-10). **LC-MS** (m/z): 442 [M-Na+NH₄⁺]⁺425 [M-Na]⁺, 423 [M-Na]⁻. Purity by **HPLC-UV (254 nm)-ESI-MS:** 98%.

### Example 18: Sodium 1-amino-4-(3-methoxyphenylamino)-9,10-dioxo-9,10-dihydroanthracene 2-sulfonate.

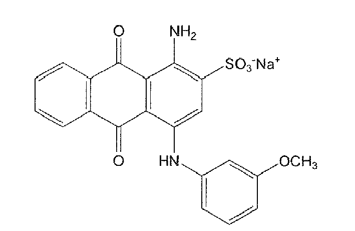

**Reaction conditions:** according to the general procedure **A:** 5 min, 100 °C, 80 W; pressure up to 10 bar.
**Analytical data:** mp > 300 °C, blue powder. ¹ H-NMR: δ 3.78 (s, 3H, 3'-OCH_;), 6.75 (dd, 1H, 4'-H), 6.85 (m, 2H, 2'-H, 6'-H), 7.33 (dd, 1H, 5'-H), 7.85 (m, 2H, 6-H, 7-H), 8.09 (s, 1H, 3-H), 8.27 (m, 2H, 5-H, 8-H), 10.10 (br, 2H, 1-NH₂), 11.99 (s, 1H, 4-NH). ¹³**C-NMR:** δ 55.33 (C3'-OCH₃), 108.41, 110.34, (C-2', C4'), 109.32 (C-9a), 111.77 (C-4a), 115.08 (C-6'), 123.23 (C-3), 126.10 (C-5), 126.18 (C-8), 130.54 (C-5'), 132.93 (C-6), 133.35 (C-7), 133.69 (C-10a), 134.29 (C-8a), 140.61 (C-1'), 140.71 (C-4), 142.81 (C-2), 144.52 (C-1), 160.51 (C-3'), 181.97 (C-9), 182.69 (C-10). **LC-MS** (m/x): **442** [M-Na+NH₄⁺]⁺, 425 [M-Na] 423 [M-Na]⁻. Purity by **HPLC-UV (254** nm)-ESI-MS: 100%.

### Example 19: Sodium 1-amino-4-(4-methoxyphenylamino)-9,10-dioxo-9,10-dihydroanthracene 2-sulfonate.

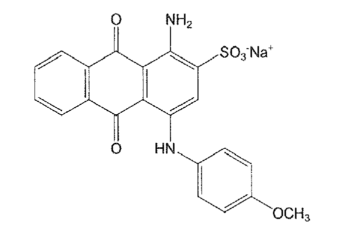

**Reaction conditions:** according to the general procedure A: 5 min, 100 °C, 80 W; pressure up to 10 bar.
**Analytical data:** mp > 300 °C, blue powder. ¹**H-NMR:** δ 3.80 (s, 3H, 4'-OCH₃), 7.03 (d, 2H, 2'-H, 6'-H), 7.22 (d, 2H, 3'-H, 5'-H), 7.83 (m, 2H, 6-H, 7-H), 7.84 (s, 1H, 3-H), 8.27 (m, 2H, 5-H, 8-H), 10.10 (br, 2H, 1-NH₂), 12.04 (s, 1H, 4-NH). ¹³**C-NMR**: δ 55.49 (C4'-OCH₃), 109.07 (C-9a), 110.43 (C-4a), 115.13 (C-2', C6), 122.57(C-3), 126.03, 126.15, (C-5, C-8, C-3', C-5'), 131.77 (C-6), 132.81 (C-7), 133.08 (C-10a), 133.84 (C-8a), 134.30 (C-1'),142.71 (C-4), 143.19 (C-2), 144.26 (C-1), 157.05 (C-4') 181.77 (C-9), 181.99 (C-10). **LC-MS (m/z):** 442 [M-Na+NH₄⁺]⁺, 425 [M-Na]⁺ 423 [M-Na]⁻. Purity by **HPLC-UV (254 nm)-ESI-MS:** 100%.

### Example 20: Sodium 1-amino-4-(4-fluorophenylamino)-9,10-dioxo-9,10-dihydroanthracene 2-sulfonate.

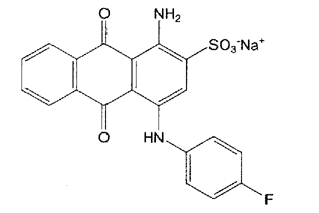

**Reaction conditions:** according to the general procedure A: 5 min, 120 °C, 80 W; pressure up to 10 bar.
**Analytical data:** mp > 300 °C, blue powder. ¹**H-NMR:** δ 7.31 (m, 4H, 2'-H, 3'-H, 5'-H, 6'-H), 7.84 (m, 2H, 6-H, 7-H), 7.88 (s, 1H, 3-H), 8.26 (m, 2H, 5-H, 8-H), 10.07 (br, 2H, 1-NH₂), 11.94 (s, 1H, 4-NH). ¹³**C-NMR:** δ 109.26 (C-9a), 111.32 (C-4a), 116.48, 116.65 (d, *J* =22.5 Hz, C-2', C6'), 122.47 (C-3), 125.85 (C-5), 125.92 (C-8), 126.07, 126.18 (d, *J* =14.0 Hz, C-3', C-5'), 132.92 (C-6), 133.31 (C-7), 133.71 (C-10a), 134.30 (C-8a), 135.73 (C-1'), 141.50 (C-4), 143.06 (C-2), 144.40 (C-1), 158.46, 160.39 (d, *J*= 242.3 Hz, C-4') 181.96 (C-9), 182.58 (C-10). **LC-MS (m/z):** 430 [M-Na+NH₄⁺]⁺, 413 [M-Na]⁺, 411 [M-Na]⁻. Purity by **HPLC-UV (254 nm)-ESI-MS:** 99%.

### Example 21: Sodium 1-amino-4-(3-bromophenylamino)-9,10-dioxo-9,10-dihydroanthracene 2-sulfonate.

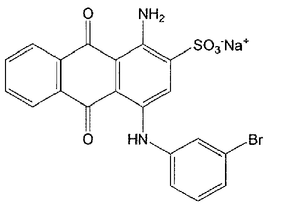

**Reaction conditions:** according to the general procedure A: 20 min, 120 °C, 100 W; pressure up to 10 bar.
**Analytical data:** mp > 300 °C, blue powder. ¹H-NMR: δ 7.27 (dd, 1H, 4'-H), 7.33 (dd, 1H, 6'-H), 7.35 (dd, 1H, 5'-H), 7.49 (dd, 1H, 2'-H), 7.85 (m, 2H, 6-H, 7-H), 8.00 (s, 1H, 3-H), 8.26 (m, 2H, 5-H, 8-H), 10.03 (br, 2H, 1-NH₂), 11.78 (s, 1H, 4-NH). ¹³**C-NMR:** δ 109.58 (C-9a), 112.89 (C-4a), 121.36, 122.52 (C-2', C6'), 123.07 (C-3), 124.97 (C-4'), 126.15, 126.22, 126.67 (C-5, C-8, C-5'), 131.53 (C-6), 133.04 (C-7), 133.57 (C3', C-10a), 134.27 (C-8a), 139.37 (C-1 '), 141.96 (C-4), 142.62 (C-2), 144.72 (C-1), 182.18 (C-9), 183.19 (C-10). **LC-MS (m/z)**: 490 [M-Na+NH₄⁺],⁺, 473 [M-Na]⁺, 471 [M-Na]⁻. Purity by **HPLC-UV** (254 mn)-ESI-1VIS: 99%.

### Example 22: Sodium 1-amino-4-(3,5-dimethylphenylamino)-9,10-dioxo-9,10-dihydroanthracene 2-sulfonate.

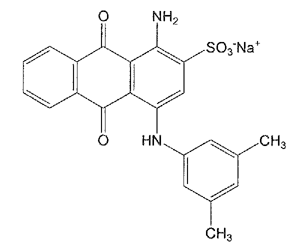

**Reaction conditions:** according to the general procedure A: 5 min, 100 °C, 80 W; pressure up to 10 bar.
**Analytical data:** mp > 300 °C, blue powder. ¹**H-NMR:** δ 2.29 (s, 6H, 3'-CH₃, 5'-CH₃), 6.85 (s, 1H, 4'-H), 6.89 (s, 2H, 2'-H, 6'-H), 7.84 (m, 2H, 6-H, 7-H), 7.99 (s, 1 H, 3-H), 8.27 (m, 2H, 5-H, 8-H), 10.10 (br, 2H, 1-NH₂), 12.01 (s, 1H, 4-NH). ¹³ **C-NMR:** δ 21.07 (C3'-CH₃, C5'-CH₃ ), 109.18 (C-9a), 111.21 (C-4a), 121.18 (C-2', C6'), 123.11 (C-3), 126.07 (C-4'), 126.16, 126.42 (C-5, C-8), 132.87 (C-6), 133.23 (C-7), 133.76 (C-10a), 134.29 (C-8a), 139.05 (C-3' and C-5'), 139.16 (C-1'), 141.40 (C-4), 142.95 (C-2), 144.42 (C-1), 181.87 (C-9), 182.40 (C-10). LC-MS (m/z): 440 [M-Na+NH₄⁺]⁺, 422 [M-Na]⁺, 420 [M-Na]⁻. Purity by **HPLC-UV (254 nm)-ESI-MS:** 99%.

### Example 23: Sodium 1-amino-4-(3-chlorophenylamino)-9,10-dioxo-9,10-dihydroanthracene 2-sulfonate.

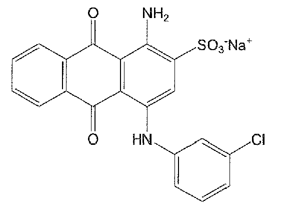

Reaction conditions: according to the general procedure A: 5 min, 100 °C, 80 W; pressure up to 10 bar.
**Analytical data:** mp > 300 °C, blue powder**. ¹H-NMR:** δ 7.20 (dd, 1H, 6'-H), 7.23 (dd, 1H, 4'-H), 7.35 (dd, 1H, 2'-H), 7.43 (dd, 1H, 5'-H), 7.85 (m, 2H, 6-H, 7-H), 8.00 (s, 1H, 3-H), 8.24 (m, 2H, 5-H, 8-H), 10.02 (br, 2H, 1-NH₂), 11.78 (s, 1H, 4-NH). ¹**³C-NMR:** δ 109.59 (C-9a), 112.90 (C-4a), 120.96 (C-6'), 122.09 (C-3), 123.10, 123.77 (C-2', C-4'), 126.16 (C-5), 126.22 (C-8), 131.28 (C-6), 133.05 (C-7), 133.58 (C-3', C-5') 134.13 (C-10a), 134.28, (C-8a), 139.37 (C-1'), 141.56 (C-4), 142.62 (C-2), 144.72 (C-1), 182.19 (C-9), 183.20 (C=10). **LC-MS** (m/z): 439 [M-Na]⁺, 437 [M-Na]⁻. LC-MS (m/z): 446 [M-Na+NH₄⁺]⁺, 429 [M-Na]⁺, 427 [M-Na]⁻. Purity by HPLC-UV (254 nm)-ESI-MS: 95%.

### Example 24: Sodium 1-amino-4-(3-amino-2-methylphenylamino)-9,10-dioxo-9,10-dihydroanthracene 2-sulfonate.

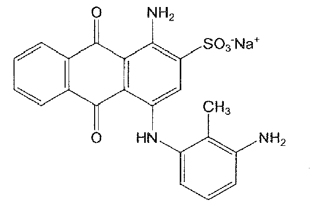

**Reaction conditions:** according to the general procedure **A:** 5 min, 120 °C, 80 W; pressure up to 10 bar.
**Analytical data:** mp > 300 °C, blue powder. **¹H-NMR:** δ 1.99 (s, 3H, C2'-CH₃), 5.05 (bs, 2H, 3'-NH₂), 6.44 (d, 1H, 6'-H), 6.59 (d, 1H, 4'-H), 6.96 (dd, 1H, 5'-H), 7.66 (s, 1H, 3-H), 7.83 (m, 2H, 6-H, 7-H), 8.28 (m, 2H, 5-H, 8-H), 10.17 (br, 2H, 1-NH₂), 12.04 (s, 1H, 4-NH). ¹³**C-NMR:** δ 11.84 (C2'-CH₃), 108.90 (C-9a), 109.96 (C-4a), 112.21 (C-6'), 113.66 (C-4'), 116.45 (C-2'), 123.14 (C-3), 126.03 (C-5), 126.13 (C-8), 126.65 (C-5'), 132.72 (C-6), 132.98 (C-7), 133.89 (C-10a), 134.33 (C-8a), 137.58 (C-1 '), 143.22 (C-4), 143.44 (C-2), 144.16 (C-1), 148.27 (C-3'), 181.63 (C-9), 181.92 (C-10). **LC-MS (m/z):** 441 [M-Na+NH₄⁺]⁺, 424 [M-Na]⁺, 422 [M-Na]⁻. Purity by HPLC-UV (254 nm)-ESI-MS: 96%.

### Example 25: Sodium 1-amino-4-(3-carboxy-4-hydroxyphenylamino)-9,10-dioxo-9,10-dihydroanthracene 2-sulfonate.

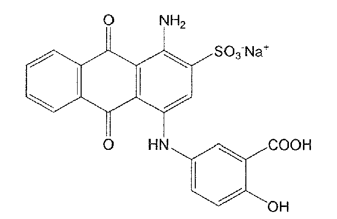

**Reaction conditions:** according to the general procedure **A:** 5 min, 120 °C, 80 W; pressure up to 10 bar.
**Analytical data:** mp > 300 °C, blue powder. ¹**H-NMR:** δ 6.70 (d, 1H, 5'-H), 7.07 (dd, 1H, 6'-H), 7.53 (d, 1H, 2'-H), 7.75 (s, 1H, 3-H), 7.82 (m, 2H, 6-H, 7-H), 8.27 (m, 2H, 5-H, 8-H), 10.18 (br, 2H, 1-NH₂), 12.07 (s, 1H, 4-NH). ¹³**C-NMR:** δ 108.86 (C-9a), 114.37 (C-4a), 116.96 (C-2'), 122.74 (C-3), 125.98 (C-5), 126.09 (C-8), 126.67 (C-6'), 128.87 (C-5'), 132.66 (C-6), 132.80 (C-7), 132.93 (C-3'), 133.59 (C-10a), 134.29 (C-8a), 134.62 (C-1'), 143.30 (C-4), 143.96 (C-2), 144.15 (C-1), 161.45 (C-4') 170.76 (COOH), 181.40 (C-9), 181.59 (C-10). **LC-MS (m/z)**: 472 [M-Na+NH₄⁺]⁺, 455 [M-Na]⁺, 453 [M-Na]⁻. Purity by **HPLC-UV (254 mn)-ESI-MS:** 95%.

### Example 26: Disodium 1-amino-4-(2-sulfophenylamino)-9,10-dioxo-9,10-dihydroanthracene 2-sulfonate.

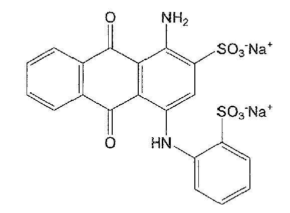

**Reaction conditions:** according to the general procedure A: 5 min, 120 °C, 80 W; pressure up to 10 bar.
**Analytical data:** mp > 300 °C, blue powder. **¹H-NMR:** 7.02 (ddd, 1H, 4'-H), 7.13 (dd, 1H, 6'-H), 7.28 (ddd, 1H, 5'-H), 7.75 (dd, 1H, 3'-H), 7.80 (m, 2H, 6-H, 7-H), 7.99 (s, 1H, 3-H), 8.23 (m, 2H, 5-H, 8-H), 10.03 (br, 2H, 1-NH₂), 11.78 (s, 1H, 4-NH). ¹³C-NMR: 109.72 (C-9a), 113.42 (C-4a), 122.32 (C-4'), 122.69 (C-6'), 124.86 (C-3), 126.05 (C-5), 126.09 (C-8), 128.01 (C-3'), 129.45 (C-5'), 132.84 (C-6), 132.98 (C-7), 133.99 (C-10a), 134.24 (C-8a), 137.43 (C-1'), 139.14 (C-4), 139.59 (C-2'), 141.67 (C-2), 144.62 (C-1), 181.51 (C-9), 182.25 (C-10). **LC-MS** (m/z): 497 [M-Na]⁺, 495 [M-Na]⁻, 475 [M-2Na]⁺, 473 [M-2Na]⁻, 236 [M-2Na]²⁻. Purity by **HPLC-UV (254 nm)-ESI-MS:** 98%.

### Example 27: Disodium 1-amino-4-(4-sulfophenylamino)-9,10-dioxo-9,10-dihydroanthracene 2-sulfonate.

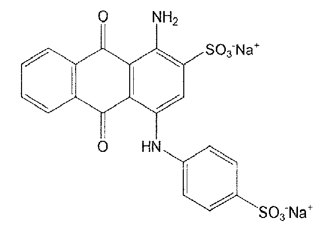

**Reaction conditions:** according to the general procedure A: 5 min, 120 °C, 80 W; pressure up to 10 b ar.
**Analytical data:** mp > 300 °C, blue powder. **¹H-NMR:** δ 7.19 (d, 2H, 2'-H, 6'-H), 7.62 (d, 2H, 3'-H, 5'-H), 7.84 (m, 2H, 6-H, 7-H), 8.04 (s, 1H, 3-H), 8.27 (m, 2H, 5-H, 8-H), 10. 10 (br, 2H, 1-NH₂), 12.01 (s, 1H, 4-NH). ¹³**C-NMR:** 109.33 (C-9a), 111.87 (C-4a), 121.76 (C-2', C-6'), 122.98 (C-3), 126.12 (C-5), 126.17 (C-8), 127.14 (C-3', C-5'), 132.92 (C-6), 133.34 (C-7), 133.71 (C-10a), 134.29 (C-8a), 139.40 (C-1'), 140.45 (C-4), 142.86 (C-2), 144.56 (C-4'), 144.58 (C-1), 181.98 (C-9), 182.72 (C-10). **LC-MS** (m/x): 492 [M-Na+NH₄⁺]⁺, 473 [M-2Na]⁻, 236 [M-2Na]²⁻. Purity by **HPLC-UV (254 nm)-ESI-MS:** 96%.

### Example 28: 1-Amino-4-[4-([1,3]diazine-2-ylamino)-3-sulfonatophenylamino]-9,10-dioxo-9,10 dihydroanthracene-2-sulfonicaciddisodiumsalt.

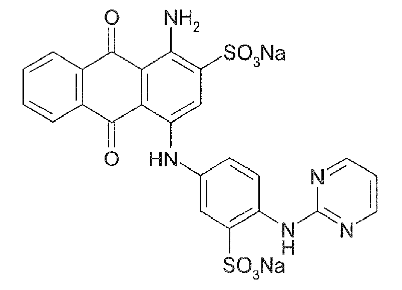

According to the general procedure **B,** the solution of 160 mg (1 mmol) of 2-bromopyrimidine and 268 mg (0.5 mmol) of 1-amino-4-[4-amino-3-sulfophenylamino]-9,10-dioxo-9,10 dihydroanthracene-2-sulfonic acid disodium salt was stirred at 0-5 °C and then the temperature was increased for refluxing at 120 °C for three days. RP-FCC was performed with a methanol/water (2:3) eluent.
**Analytical data:** yield (295 mg) 97 %, a blue powder, mp > 300 °C,
¹**H-NMR:** δ 6.88 (dd, 1H, 4"-H), 7.26 (dd, 1H, 6'-H), 7.55 (d, 1H, 2'-H), 7.84 (m, 2H, 6-H, 7-H), 7.92 (s, 1H, 3-H), 8.28 (m, 2H, 5-H, 8-H), 8.52 (d, 2H, 3"-H, 5"-H), 8.58 (d, 1H, 5'-H), 10.15 (br, 2H, 1-NH2), 10.03 (br, 1H, 4'-NH), 12.12 (br, 1H, 4-NH). ¹³**C-NMR:** δ 108.89 (C-9a), 110,56 (C-4a), 112.61 (C-5"), 119.67 (C-5'), 122.41 (C-3, C-2'), 124.33 (C-6'), 125.76 (C-5, C-8), 131.23 (C-1'), 132.37 (C-6), 132.67 (C-7), 133.54 (C-4', C-10a), 134.01 (C-8a), 136.06 (C-3'), 141.78 (C-4), 142.98 (C-2), 144.12 (C-1), 157.80 (C-4", C-6"), 159.24 (C-2"), 181.51 (C-9), 181.84 (C-10). LC-MS (m/z): 585 [M-2Na+NH₄⁺]⁺.Purity by **HPLC-UV (254 nm)-ESI-MS:** 95%.

### Example 29: 1-Amino-4-[4-phenylamino-3-sulfophenylaminol-9,10-dioxo-9,10 dihydroanthracene-2-sulfonic acid disodium salt.

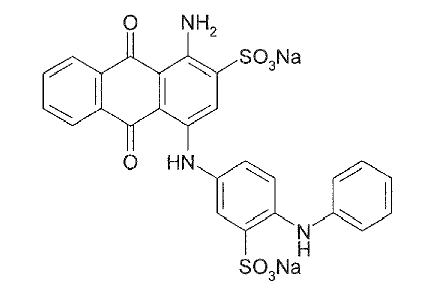

Reaction conditions: according to the general procedure A: 5 min, 120 °C, 100 W; pressure up to 10 bar.
**Analytical data:** mp > 300 °C, blue powder.
¹**H-NMR**: δ 6.91 (dd, 1H, 4"-H), 7.12 (m, 3H), 7.30 (m, 3H), 7.50 (d, 1H), 7.83 (s, 1H, 3-H), 7.83 (m, 2H, 5-H, 8-H), 8.28 (m, 2H, 6-H, 7-H), 12.12 (br, 1H, 4-NH).
¹³**C-NMR:** δ 109.11, 110.42, 115.79, 118.28, 120.98, 122.68, 124.10, 126.07, 126.13, 129.51, 132.78, 133.01, 133.89, 134.28, 135.31, 137.88, 142.38, 142.76, 143.25, 144.25, 181.75,181.88.
**LC-WS (m/z)**: 564 [M-2Na]⁻, 281 [M-2Na]²⁻ 583 [M-2Na+NH₄⁺]⁺.
Purity by **HPLC-UV (254 nm)-ESI-MS:** 100%.

### Example 30: 1-Amino-4-[4-phenoxy-3-sulfophenylamino]-9,10-dioxo-9,10 dihydroanthracene-2-sulfonic acid disodium salt.

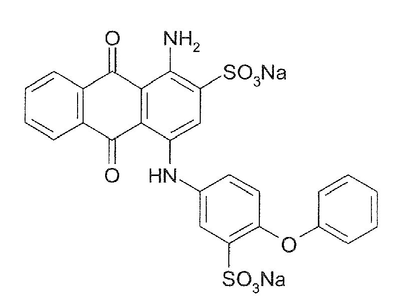

**Reaction conditions:** according to the general procedure A: 5 min, 120 °C, 100 W; pressure up to 10 bar.
Analytical data: mp > 300 °C, blue powder.
**¹H-NMR:** δ 6.84 (d, 1H), 6.98 (dd, 2H), 7.06 (dd, 1H), 7.21 (dd, 1H), 7.34 (dd, 2H), 7.63 (d, 1H), 7.84 (m, 2H, 5-H, 8-H), 7.91 (s, 1H, 3-H), 8.28 (m, 2H, 6-H, 7-H), 10.15 (br, 2H, 1-NH2), 12.07 (br, 1H, 4-NH).
**¹³C-NMR:** δ 109.22, 111.20, 119.01, 121.31, 122.61, 122.72, 124.26, 125.18, 126.14, 129.63, 132.87, 133.22, 133.80, 134.28, 140.86, 141.60, 143.14, 144.44, 150.71, 158.12, 181.91, 182.47.
**LC-MS (m/z)** : 565 [M-2Na]⁻, 282 [M-2Na]²⁻ , 584 [M-2Na+NH₄⁺]⁺.
Purity by **HPLC-UV (254 nm)-ESI-MS:** 99%.

### Example 31: 1-Amino-4-[4-phenylamino-3-carboxyphenylamino]-9,10-dioxo-9,10 dihydroanthracene-2- sulfonic acid sodium salt.

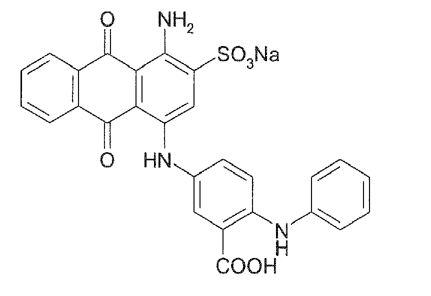

**Reaction conditions:** according to the general procedures **E, F**, and **G:**
**Analytical data:** mp > 300 °C, blue powder.
**¹H-NMR: δ** 7.09 (dd, 1H, 4"-H), 7.29 (m, 3H), 7.37 (m, 3H), 7.78 (d, 1H), 7.81 (s, 1H, 3-H), 7.83 (m, 2H, 5-H, 8-H), 8.27 (m, 2H, 6-H, 7-H), 9.60 (br, 2H, 1-NH2), 10.03 (br, 1H, 4'-NH), 12.00 (br, 1H, 4-NH).
¹³**C-NMR:** δ 109.1, 110.6, 113.5, 115.3, 121.6, 122.5, 123.3, 126.0, 126.1, 127.8, 128.8, 129.7, 131.4, 132.8, 133.1, 133.8, 134.3, 140.6, 142.6, 143.19, 144.3, 144.8, 169.5, 181.8, 182.1.
**LC-MS (m/z)**: 528 [M-Na]⁻, 529 [M-Na]⁺, 528 [M-Na]⁻ , 547 [M-Na+NH₄⁺]⁺.
Purity by **HPLC-UV (254 nm)-ESI-MS:** 98%.

### Example 32: 1-Amino-4-[4-phenylamino-3-sulfophenylamino]-9,10-dioxo-9,10 dihydroanthracene-2-carboxylic acid sodium salt.

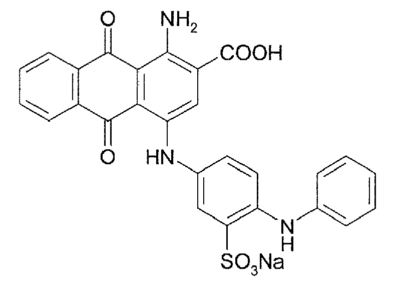

**Reaction conditions:** according to the general procedure A: 5 min, 120 °C, 100 W; pressure up to 10 bar.
**Analytical data:** mp > 300 °C, blue powder.
¹H-NMR: δ 6.93 (dd, 1H, 4"-H), 7.13 (d, 2H), 7.21 (d, 1H), 7.31 (dd, 2H), 7.35 (d, 1H), 7.56 (d, 1H), 7.88 (m, 2H, 6-H, 7-H), 8.19 (s, 1H, 3-H), 8.29 (m, 2H, 5-H, 8-H), 11.71 (br, 1H, 4-NH).
¹³**C-NMR:** δ 110.44, 113.86, 115.91, 118.25, 121.00, 122.97, 123.69, 125.92, 126.18, 126.29, 129.17, 129.7, 133.19, 133.47, 133.163, 134.13, 135.25, 137.72, 140.82, 142.37, 147.24, 167.68, 182.29, 183.13.
**LC-MS (m/z):** 528 [M-Na]⁻ , 529 [M-Na]⁺, 528 [M-Na]⁻ , 547 [M-Na+NH4⁺]⁺.
Purity by **HPLC-UV (254 nm)-ESI-MS:** 98%.

### Example 33: 1-Amino-2-methyl-4-[4-phenylamino-3-sulfophenylamino]-9,10-dioxo-9,10 dihydroanthracene sodium salt.

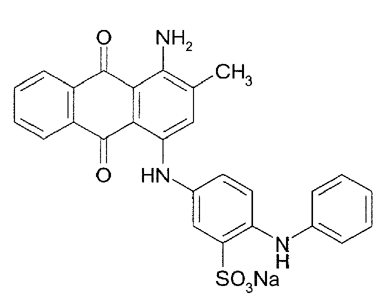

**Reaction conditions:** according to the general procedure A: 5 min, 120 °C, 100 W; pressure up to 10 bar.
**Analytical data:** mp > 300 °C, blue powder.
¹**H-NMR:** δ 2.25 (s, 3H, CH₃), 6.91 (dd, 1H, 4"-H), 7.11 (d, 2H), 7.18 (dd, 1H), 7.30 (m, 4H), 7.51 (d, 1H), 7.81 (m, 2H, 5-H, 8-H), 8.27 (m, 2H, 6-H, 7-H), 8.55 (s, 1H, 3-H), 12.27 (br, 1 H, 4-NH).
¹³**C-NMR:** δ 18.77, 107.9, 108.6, 115.9, 118.2, 120.9, 123.9, 124.3, 125.9, 126.1, 126.2, 129.4, 129.5, 132.7, 134.1, 135.2, 137.6, 137.7, 142.4, 143.7, 147.0, 181.2, 181.8.
**LC-MS (m/z):** 498 [M-Na]⁻, 500 [M-Na]⁺.
Purity by **HPLC-UV (254 nm)-ESI-MS:** 96%.

### Example 34: 5-Nitro-2-phenylaminobenzoic acid.

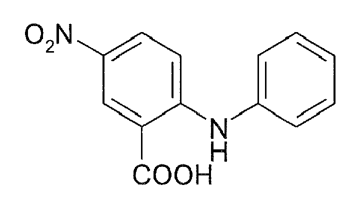

**Reaction conditions:** according to the general procedure E: 10 min, 100 °C, 80 W; pressure up to 10 bar.
**Analytical data:** yellow solid.
¹**H-NMR:** δ 7.11 (d, 1H), 7.26 (ddd, 1H), 7.35 (dd, 2H), 7.45 (m, 2H), 8.15 (dd, 1H), 8.70 (d, 1H, 10.3 5(br, 1H, NH), 13.7 (br, 1H, COOH).
**¹³C-NMR:** δ 111.13, 113.35, 124.17, 125.94, 128.58, 129.45, 129.93, 136.73, 138.32, 152.52, 168.77.
**LC-MS (m/z):** 259 [M]⁺, 276 [M+NH₄⁺]⁺, 257 [M]⁻, 213 [M-COO⁻]⁻ .
Purity by **HPLC-UV (254 nm)-ESI-MS:** 99%.

### Example 35: 5-Nitro-2-(4-ethyl-phenylamino)benzoic acid.

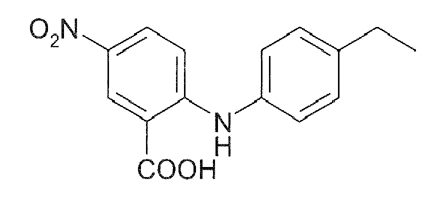

**Reaction conditions:** according to the general procedure E: 10 min, 100 °C, 80 W; pressure up to 10 bar.
**Analytical data:** dark green solid.
¹H-NMR: δ 1.19 (t, 3H, CH₃), 2.62 (q, 2H, CH₂), 7.05 (d, 1H), 7.23 (d, 2H), 7.28 (d, 2H), 8.11 (dd, 1H), 8.72 (d, 1H), 10.81 (br, 1H, NH).
¹³**C-NMR:** δ 15.66, 27.79, 112.80, 124.04, 128.61, 128.88, 129.15, 136.17, 136.37, 141.34, 152.81, 168.92.
**LC-MS (m/z):** 287 +[M]⁺, 304 [M+NH₄⁺1', 285 [M]⁻, 241 [M-COO⁻]⁻.
Purity by **HPLC-UV (254 nm)-ESI-MS:** 99%.

### Example 36: 5-Nitro-2-(3-ethyl-phenylamino)benzoic acid.

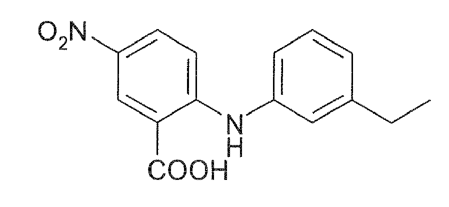

**Reaction conditions:** according to the general procedure **E**: 5 min, 80 °C, 80 W; pressure up to 10 bar.
**Analytical data:** yellow solid.
**¹H-NMR:** δ 1.19 (t, 3H, CH₃), 2.62 (q, 2H, CH₂), 7.11 (d, 2H), 7.13 (m, 2H), 7.35 (dd, 1H), 8.15 (dd, 1H), 8.70 (d, 1H), 10.33 (br, 1H, NH), 13.7 (br, 1H, COOH).
¹³**C-NMR:** δ 15.53, 28.12, 110.99, 113.42, 121.36, 123.52, 125.47, 128.59, 129.46, 129.77, 136.62, 138.25, 145.88, 152.59, 168.80.
LC-MS (m/z): 287 [M]⁺, 304 [M+NH₄⁺]⁺, 285 [M]⁻, 241 [M-COO].
Purity by **HPLC-UV (254 nm)-ESI-MS:** 99%.

### Example 37: 5-Nitro-2-(2-ethyl-phenylanino)benzoic acid.

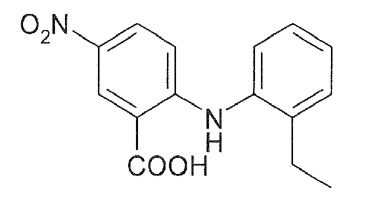

**Reaction conditions:** according to the general procedure E: 20 min, 120°C, 150 W; pressure up to 10 bar.
Analytical data: yellowish green solid.
¹**H-NMR:** δ 1.10 (t, 3H, CH₃), 2.55 (q, 2H, CH₂), 6.72 (d, 1H), 7.31 (m, 3H), 7.40 (m, 1 H), 8.13 (dd, 1 H), 8.71 (d, 1H), 10.28 (br, 1H, NH), 13.5 (br, 1H, COOH).
¹³**C-NMR:** δ 14.62, 24.23, 110.50, 112.98, 126.25, 127.23, 127.37, 128.62, 129.54, 129.94, 136.14, 136.37, 139.67, 153.56, 169.04.
**LC-MS** (m/z): 287 [M]⁺, 304 [M+NH₄⁺]⁺, 285 [M]⁻, 241 [M-COO⁻]⁻.
Purity by **HPLC-UV (254 nm)-ESI-MS:** 95%.

### Example 38: 5-Nitro-2-(3-chloro-phenylamino)benzoic acid.

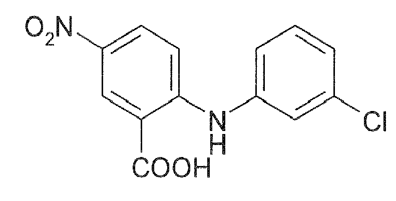

**Reaction conditions:** according to the general procedure E: 30 min, 120 °C, 150 W; pressure up to 10 bar.
Analytical data: yellowish green solid.
**¹H-NMR:** δ 7.18 (d, 1H), 7.29 (dd, 1H), 7.33 (dd, 1H), 7.45 (m, 2H), 8.19 (dd, 1H), 8.70 (d, 1H), 10.34 (br, 1H, NH), 13.8 (br, 1H, COOH).
**¹³C-NMR:** δ 112.00, 114.00, 122.42, 123.62, 125.47, 128.40, 129.45, 131.38, 134.08, 137.33, 140.19, 151.80, 168.55.
**LC-MS** (m/z): 291 [M]⁻, 247 [M-COO⁻]⁻.
Purity by **HPLC-UV (254 nm)-ESI-MS**: 97%.

### Example 39: 5-Nitro-2-(2-methoxy-phenylamino)benzoic acid.

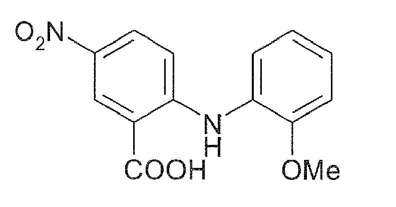

**Reaction conditions:** according to the general procedure E: 15 min, 120 °C, 100 W; pressure up to 10 bar.
**Analytical data:** green solid.
**¹H-NMR:** δ 3.81 (s, 3H, OCH₃), 7.00 (m, 2H), 7.17 (dd, 1H), 7.25 (ddd, 1H), 7.40 (dd, 1H), 8.15 (dd, 1H), 8.71 (d, 1H), 10.28 (br, 1H, NH), 13.7 (br, 1H, COOH).
**¹³C-NMR:** δ 55.89, 111.12, 112.50, 113.48, 120.91, 124.21, 126.75, 126.91, 128.50, 129.32, 136.56, 152.35,152.66,168.77.
**LC-MS (**m/z): 289 [M]⁺, 306 [M+NH₄⁺]⁺ 287 [M]⁻, 243 [M-COO⁻]⁻.
Purity by **HPLC-UV (254 nm)-ESI-MS:** 100%.

### Example 40: 5-Nitro-2-(3-methoxy-phenylamino)benzoic acid.

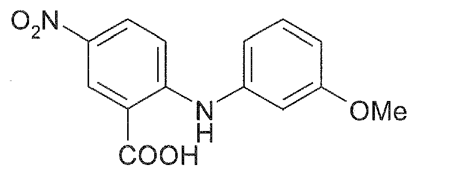

**Reaction conditions:** according to the general procedure E: 10 min, 110 °C, 100 W; pressure up to 10 bar.
**Analytical data:** yellowish green solid.
¹**H-NMR:** δ 3.77 (s, 3H, OCH₃), 6.83 (m, 1H), 6.91 (m, 2H), 7.18 (d, 1H), 7.35 (dd, 1H), 8.16 (dd, 1H), 8.70 (d, 1H), 10.32 (br, 1H, NH), 13.7 (br, 1H, COOH).
¹³**C-NMR:** δ 55.41, 109.61, 111.22, 111.73, 113.71, 116.04, 128.52, 129.43, 130.65, 136.79, 139.53, 152.38, 160.53, 168.74.
**LC-MS** (m/z): 289 [M]⁺, 306 [M+NH₄⁺]⁺, 287 [M]⁻, 243 [M-COO⁻]⁻.
Purity by **HPLC-UV (254 nm)-ESI-MS:** 95%.

### Example 41: 5-Nitro-2-(2-chloro-phenylamino)benzoic acid.

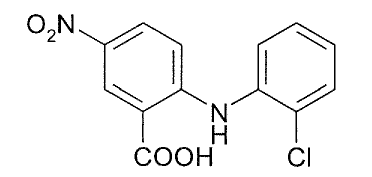

**Reaction conditions:** according to the general procedure **E**: 30 min, 150 °C, 150 W; pressure up to 10 bar.
**Analytical data:** yellowish green solid.
**¹H-NMR**: δ 6.97 (d, 1H), 7.31 (dd, 1H), 7.44 (ddd, 1 H), 7.59 (dd, 1H), 7.63 (dd, 1H), 8.19 (dd, 1H), 8.72 (d, 1H), 10.44 (br, 1H, NH).
**¹³C-NMR:** δ 111.77, 113.70, 126.06, 127.52, 128.38, 128.42, 128.53, 129.49, 130.55, 135.54, 137.43, 151.82, 168.75.
**LC-MS (m/z):** 291 [M], 247 [M-COO]
Purity by **HPLC-UV** (254 **nm)-ESI-MS:** 99%.

### Example 42: 5-Nitro-2-(4-fluoro-phenylanino)benzoic acid.

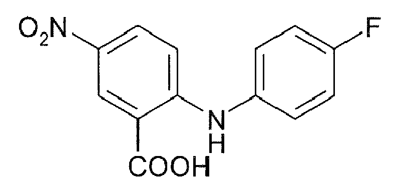

**Reaction conditions:** according to the general procedure E: 10 min, 120 °C, 100 W; pressure up to 10 bar.
**Analytical data:** greenish yellow solid.
**¹H-NMR:** δ 6.96 (d, 1H), 7.28 (m, 2H), 7.38 (m, 2H), 8.13 (dd, 1H), 8.69 (d, 1H), 10.28 (br, 1 H, NH).
¹**³C-NMR:** δ 111.13, 113.18, 116.57, 116.75, 126.94, 127.01, 128.55, 129.40, 134.68, 134.70, 136.68, 152.98, 159.12, 161.05, 168.73.
**LC-MS** (m/z): 277 [M]⁺, 294 [M+NH₄⁺]⁺, 275 [M]⁻, 230 [M-COO⁻]⁻.
Purity by **HPLC-UV (254 nm)-ESI-MS:** 99%.

### Example 43: 5-Nitro-2-(3-bromo-phenylamino)benzoic acid.

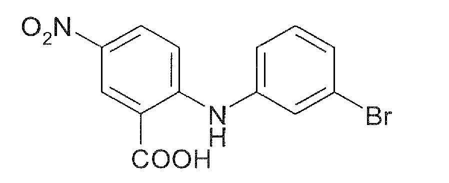

**Reaction conditions:** according to the general procedure E: 15 min, 120°C, 100 W; pressure up to 10 bar.
**Analytical data:** greenish yellow solid.
¹**H-NMR:** δ 7.17 (d, 1H), 7.40 (m, 3H), 7.58 (m, 1H), 8.19 (dd, 1H), 8.70 (d, 1H), 10.33 (br, 1H, NH), 13 .6 (br, 1H, COOH).
¹³**C-NMR:** δ 111.98, 113.87, 122.41, 122.85, 126.48, 128.39,129.47, 131.63, 137.32, 140.32, 141.81, 168.53.
**LC-MS** (m/z): 337 [M]⁺, 354 [M+NH₄⁺]⁺, 335 [M]⁻, 291 1 [M-COO⁻]⁻.
Purity by **HPLC-UV (254** nm)-ESI-MS: 95%.

### Example 44: 5-Nitro-2-(3,5-dimethyl-phenylamino)benzoic acid.

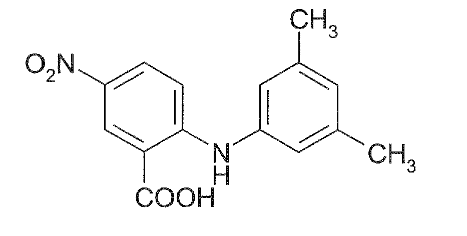

**Reaction conditions:** according to the general procedure **E**: 10 min, 120 °C, 100 W; pressure up to 10 bar.
**Analytical data:** yellow solid.
¹**H-NMR:** δ 2.48 (s, 6H, 2CH₃), 6.88 (br, 1H), 6.93 (br, 2H), 7.11 (d, 1H), 8.13 (dd, 1H), 8.70 (d, 1H), 10.47 (br, 1H, NH), 13.5 (br, 1 H, COOH).
¹³**C-NMR:** 820.94,111.56, 113.34, 121.45, 127.30, 128.54, 129.20, 136.47, 138.17, 139.18, 152.54, 168.84.
**LC-MS** (m/z): 287 [M]⁺, 304 [M+NH₄⁺]⁺, 285 [M]⁻, 241 [M-COO⁻]⁻.
Purity by **HPLC-UV (254 nm)-ESI-MS:** 99%.

### Example 45: 5-Nitro-2-(2,5-dimethyl-phenylamino)benzoic acid.

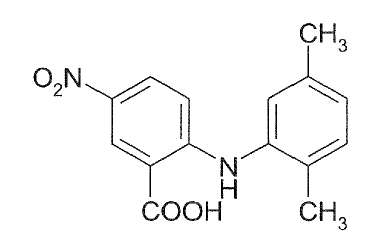

**Reaction conditions:** according to the general procedure E: 20 min, 120 °C, 100 W; pressure up to 10 bar.
Analytical data: yellowish green solid.
**¹H-NMR: δ 2.13** (s, 3H, CH₃), 2.25 (s, 3H, CH₃), 6.70 (d, 1H), 7.05 (dd, 1H), 7.13 (br, 1H), 7.24 (d, 1 H), 8.12 (dd, 1H), 8.70 (d, 1H), 10.20 (br, 1 H, NH), 13 .7 (br, 1 H, COOH).
¹³**C-NMR:** δ 17.11, 20.58, 110.51, 113.10, 126.25, 127.59, 128.60, 129.47, 130.49, 131.22, 136.27, 136.51, 136.63, 153.23, 168.92.
**LC-WS (m/z):** 287 [M]⁺, 304 [M+NH₄⁺]⁺, 285 [M]⁻, 241 [M-COO⁻]⁻.
Purity by **HPLC-UV (254 nm)-ESI-MS:** 97%.

### Example 46: 5-Nitro-2-(3-methyl-4-chloro-phenylamino)benzoic acid.

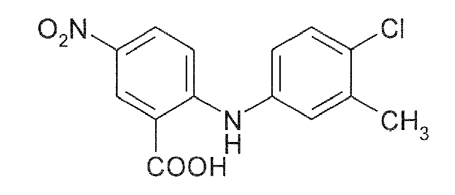

**Reaction conditions:** according to the general procedure E: 30 min, 120 °C, 100 W; pressure up to 10 bar.
Analytical data: dark green solid.
**¹H-NMR:** :δ 2.20 (s, 3H, CH₃), 6.81 (d, 1H), 7.27 (m, 1H), 7.34 (d, 1H), 7.40 (d, 1H), 8.01 (dd,1H), 8.75 (d,1H),12.30 (br, 1H, COOH).
**¹³C-NMR:** δ 17.65, 111.70, 118.57, 124.80, 126.77, 127.35, 128.35, 128.62,130.75, 134.59, 136.40, 137.26, 152.48, 168.90.
**LC-MS (m/z):** 307 [M]⁺, 324 [M+NH₄⁺]⁺, 305 [M]⁻, 261 [M-COO⁻]⁻.
Purity by **HPLC-UV (254 nm)-ESI-MS:** 97%.

### Example 47: 5-Nitro-2-(3,4-dimethyl-phenylamino)benzoic acid.

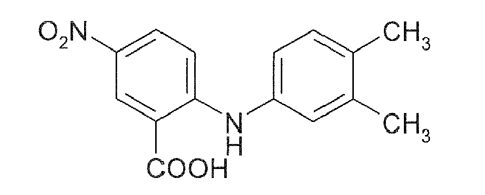

**Reaction conditions:** according to the general procedure E: 10 min, 100 °C, 100 W; pressure up to 10 bar.
**Analytical data:** yellowish green solid.
**¹H-NMR:** δ 2.12 (s, 3H, CH₃), 2.30 (s, 3H, CH₃), 6.61 (d, 1H), 7.05 (dd, 1H), 7.16 (d, 2H), 8.10 (dd, 1H), 8.69 (d, 1H), 10.11 (br, 1H, N H).
¹**³C-NMR:** δ 17.48, 20.70, 110.29, 112.99, 126.12, 127.69, 128.67, 129.51, 131.98, 133.78, 134.08, 136.18, 136.40, 153.59, 168.92.
**LC-MS (m/z):** 287 [M]⁺, 304 [M+NH₄⁺]⁺, 285 [M]⁻, 241 [M-COO⁻]⁻.
Purity by HPLC-UV (254 nm)-ESI-MS: 95%.

### Example 48: 5-Nitro-2-(2,3-dimethyl-phenylamino)benzoic acid.

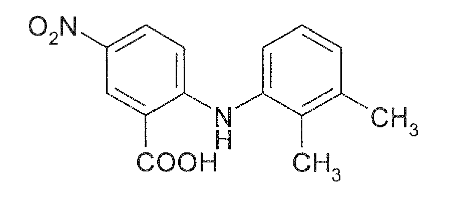

**Reaction conditions:** according to the general procedure E: 30 min, 120 °C, 100 W; pressure up to 10 bar.
Analytical data: yellowish green solid.
**¹H-NMR:** δ 2.08 (s, 3H, CH₃), 2.29 (s, 3H, CH₃), 6.58 (d, 1H), 7.16 (m, 3H), 8.10 (dd, 1H), 8.70 (d, 1H), 10.18 (br, 1H, NH),13.6 (br, 1H, COOH).
¹³**C-NMR**. δ 13.86, 20.21, 110.26, 113.10, 124.06, 126.58, 128.54, 128.63, 129.47, 132.84, 136.20, 136.53, 138.50, 153.62, 168.95.
**LC-MS (m/z):** 287 [M]⁺, 304 [M+NH₄⁺]⁺, 285 [M]⁻, 241 [M-COO⁻]⁻.
Purity by **HPLC-UV (254 nm)-ESI-MS:** 95%.

### Example 49: 5-Nitro-2-(2-ethoxy-phenylanxino)benzoic acid.

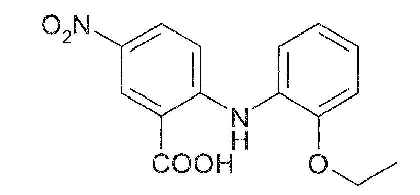

**Reaction conditions:** according to the general procedure **E**: 15 min, 120 °C, 100 W; pressure up to 10 bar.
**Analytical data:** yellowish green solid.
**¹H-NMR:** δ 1.28 (t, 3H, CH₃), 4.08 (q, 2H, CH₂), 7.00 (ddd, 1H), 7.15 (m, 3H), 7.44 (dd, 1H), 8.16 (dd, 1H), 8.71 (d, 1H), 10.45 (br, 1H, NH), 13.7 (br, 1H, COOH).
**¹³C-NMR:** δ 14.68, 64.12, 111.28, 113.42, 113.52, 120.83, 123.02, 126.32, 127.27, 128.50, 129.30, 136.60, 151.37, 151.92, 168.81.
**LC-MS** (m/z): 303 [M]⁺, 320 [M+NH₄⁺]⁺, 301 [M]⁻, 257 [M-COO⁻]⁻.
Purity by HPLC-UV **(254** nm)-ESI-MS: 99%.

### Example 50: 5-Nitro-2-(4-ethoxy-phenylamino)benzoic acid.

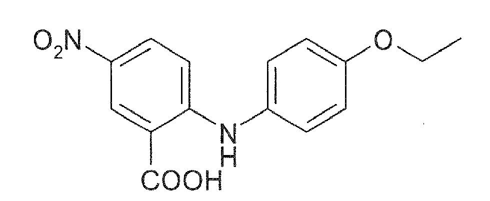

**Reaction conditions:** according to the general procedure E: 5 min, 100 °C, 100 W; pressure up to 10 bar.
**Analytical data:** green solid.
¹**H-NMR:** δ 1.33 (t, 3H, CH₃), 4.04 (q, 2H, CH₂), 6.88 (d, 1H), 7.00 (d, 2H), 7.24 (d, 1H), 8.11 (dd, 1 H), 8.68 (d, 1H), 10.17 (br, 1H, NH), 13.8 (br, 1H, COOH).
¹³**C-NMR:** δ 14.77, 63.47, 110.35, 113.03, 115.61, 126.66, 128.65, 129.41, 130.67, 136.18, 153.62, 156.97, 168.81.
**LC-MS** (m/z): 303 [M]⁺, 320 [M+NH₄⁺]⁺ 301 [M]⁻, 257 [M-COO⁻]⁻.
Purity by HPLC-UV **(254** nm)-ESI-MS: 100%.

### Example 51: 5-Nitro-2-(2,6-diethyl-phenylamino)benzoic acid.

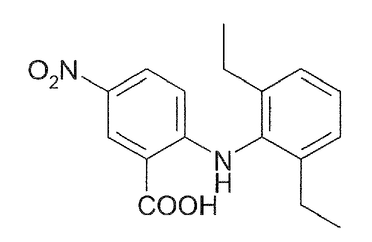

**Reaction conditions:** according to the general procedure E: 40 min, 150 °C, 150 W; pressure up to 10 bar.
**Analytical data:** green solid.

### Example 52: 5-Nitro-2-cyclohexylaminobenzoic acid.

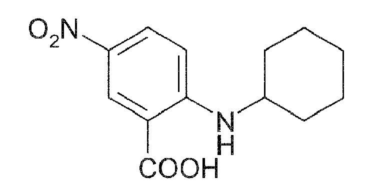

**Reaction conditions:** according to the general procedure E: 5 min, 120 °C, 100 W; pressure up to 10 bar.
Analytical data: pale yellow solid.
¹**H-NMR:** δ 1.29 (m, 3H), 1.40 (m, 2H), 1.55 (m, 1H), 1.65 (m, 2H), 1.91 (m, 2H), 3.59 (m, 1H), 6.91 (d, 1H), 8.11 (dd, 1H), 8.62 (d, 1H), 8.79 (d, 1H, NH), 13.7 (br, 1H, COOH).
¹³**C-NMR**: δ 23.94,25.16,32.00, 50.22,108.96,112.02,128.96, 129.52, 134.54, 153.83, 169.01.

### Example 53: 5-Nitro-2-cyclopentylaminobenzoic acid.

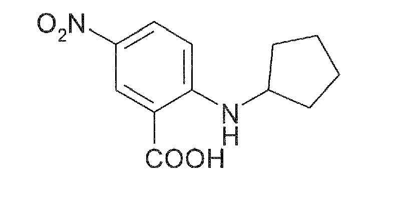

**Reaction conditions:** according to the general procedure E: 5 min, 120 °C, 100 W; pressure up to 10 bar.
**Analytical data:** pale yellow solid.
¹**H-NMR:** δ 1.47 (m, 2H), 1.64 (m, 4H), 2.04 (m, 2H), 3.99 (m, 1H), 6.87 (d, 1H), 8.13 (dd, 1H), 8.60 (d, 1H), 8.77 (d, 1H, NH), 13.4 (br, 1H, COOH).
¹³**C-NMR:** δ 23.65, 32.86, 53.63, 109.16, 112.27, 128.73, 129.45, 134.73, 154.24, 168.94.

### Example 54: 5-Nitro-2-phenethylaminobenzoic acid.

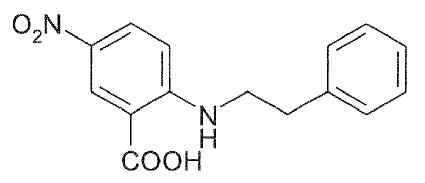

**Reaction conditions:** according to the general procedure **E:** 10 min, 120 °C, 100 W; pressure up to 10 bar.
**Analytical data:** pale yellow solid.

### Example 55: 5-Nitro-2-benzylminobenzoic acid.

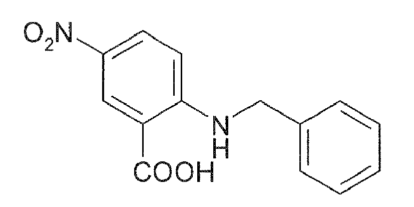

**Reaction conditions:** according to the general procedure E: 10 min, 120 °C, 100 W; pressure up to 10 bar.
**Analytical data:** yellow solid.

### Biological Assays

### Example 56: Human platelet membrane preparation

Membranes were prepared from human outdated platelets from the blood bank. Outdated human platelets were obtained from the University of Bonn blood bank 4-5 days after donation. Platelet rich plasma was washed by centrifugation (10 min at 1000 g) in a buffer consisting of 50 mM Tris-HCl, pH 7.4, 5 mM EDTA and 150 mM NaCl. The supernatant was centrifuged twice (48400 g for 60 min) and the resultant platelet pellet were resuspended in 5 mM Tris-HCl (pH 7.4) containing 5 mM EDTA. The platelets were homogenized by a homogenizer. The final suspension was stored frozen as multiple aliquots at -80°C until needed. Protein concentrations were determined by the method of Lowry using a Sigma Chemie protein assay kit.

### Example 57: Radioligand binding assay

Radioligand binding assays were performed using 5 nM [³H]PSB-0413 and 100 µg of protein in Tris-HCl buffer 50 mM, pH 7.4, as previously described. (El-Tayeb, A., Griessmeier, K.J, Muller, C.E. Bioorg. Med. Chem. Lett. 2005, 15, 5450-52; Atzler, K. Doctoral Thesis, University of Bonn, 2006). Competition by 10 µM of test compound was initially determined. The mixture was incubated for 1 h at rt followed by filtration through GF/B filters. Nonspecific binding was determined with 1 mM ADP or 250 µM RB 2. For potent compounds concentration-inhibition curves were determined using at least 6-7 different concentrations spanning 3 orders of magnitude. At least three independent experiments were performed each in triplicate.

In Table 3 Kᵢ values of selected compounds as antagonist of human platelets are given. In order to investigate selectivity of selected compounds, potencies at other P2Y receptor subtypes were determined for comparison.

Selected compounds were investigated for their functionality in GTP shift experiments. Radioligand binding studies as described above were performed in the presence and absence of GTP (100 µM). Agonists, such as ADP, showed a significant right-shift of the concentration-inhibition curve, while the investigated compounds (e.g. Reactive Blue 2, and entry 33 and entry 37 in Table 3) did not. These results show that the compounds behave as antagonists rather than agonists at P2Y₁₂ receptors.

### Example 58: Determination of potency as inhibitors of P2Y₂, P2Y₄, and P2Y₆ receptors

Inhibition of agonist-induced calcium mobilization was determined as previously described (Kaulich M. et al, Drug Dev. Res. 2003, 59, 72-81) using NG108-15 cells for the mouse P2Y₂ receptor, or human recombinant P2Y receptors stably expressed in human astrocytoma cells (El-Tayeb, A. et al., J Med. Chem. 2006, 49, 7076-87).

### Data analysis

The data were analyzed using GraphPad Prism 4.0 (GraphPad Software, Inc., San Diego, CA). The values are presented as means of the percent inhibition at 10 µM ± SEM (n=3) or Kᵢ value in µM ± SEM (n=3).

**Table 3. Affinities of selected compounds for human platelet P2Y₁₂ receptors and antagonistic potency of selected compounds at P2Y receptor subtypes The data represent means ± SEM of usually three separate experiments each run in triplicate. (Abbreviations: h = human, m = mouse; n.d. = not determined).**

| **Entry** | *Structure* | hP2Y₂ Receptor IC₅₀ (µM ± SEM) | **mP2Y2** Receptor IC₅₀ (µM ± ±SEM) | **hP2Y₄** Receptor IC₅₀ (µM | **rP2Y₆** Receptor IC₅₀ (µM | **hP2Y₁₂** Receptor Kᵢ [µM± SEM) vs. [3H]PSB -0413 |
|---|---|---|---|---|---|---|
| **1** | 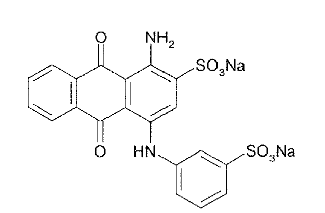 | n.d. | n.d. | **18.5**±2.5 | ca. 100 | **31.81**±1.08 |
| **2** | 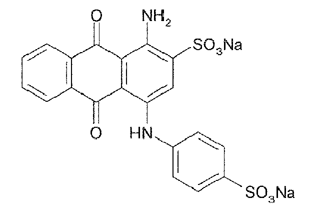 | n.d. | n.d. | n.d. | n.d. | **3.13** ± 0.39 |
| **3** | 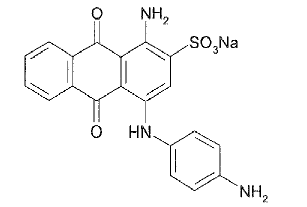 | n.d. | n.d. | n.d. | n.d. | >**10** |
| **4** | 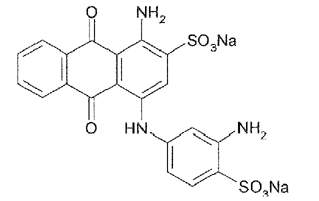 | n.d. | n.d. | n.d. | n.d. | **2.10** ± 0.47 |
| **5** | 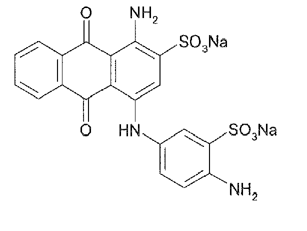 | n.d. | n.d. | 11.0± 3.0 | 24.3 ± 3.4 | **2.39** ± 0.65 |
| **6** | 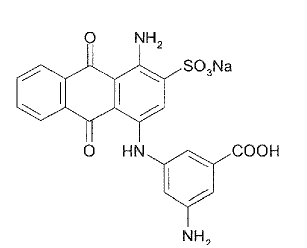 | n.d. | n.d. | n.d. | n.d. | **7.07** ± |
| **7** | 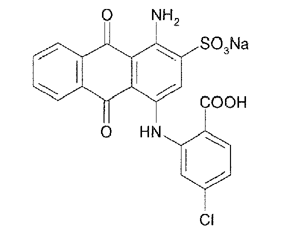 | n.d. | n.d. | n.d. | n.d. | **12.2** ± 0.3 |
| **8** | 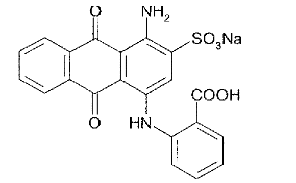 | n.d. | n.d. | n.d. | n.d. | **25.1** ± 7.0 |
| **9** | 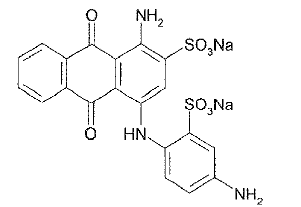 | n.d. | n.d. | n.d. | n.d. | **19.74** ± 8.27 |
| **10** | 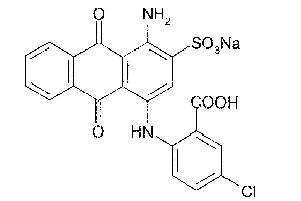 | n.d. | n.d. | n.d. | n.d. | **12.3** ± 1.7 |
| **11** | 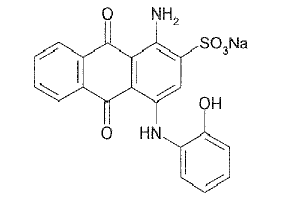 | n.d. | n.d. | n.d. | n.d. | **ca. 10** |
| **12** | 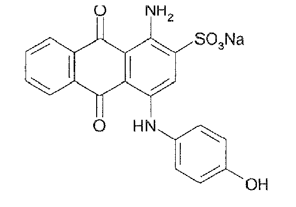 | n.d. | n.d. | n.d. | n.d. | **ca. 10** |
| **13** | 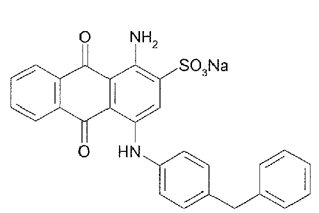 | n.d. | n.d. | n.d. | n.d. | **0.614** |
| **14** | 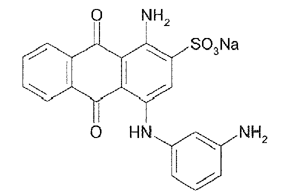 | n.d. | n.d. | n.d. | n.d. | **>10** |
| **15** | 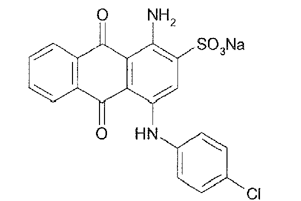 | 4.46 ± 0.75 | 20.0± 7.0 | »3 | » 10 | **7,35** ± 1,72 |
| **16** | 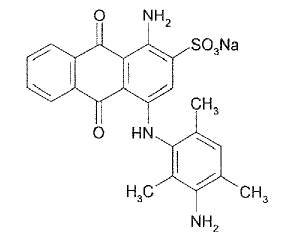 | n.d. | n.d. | n.d. | n.d. | **>10** |
| **17** | 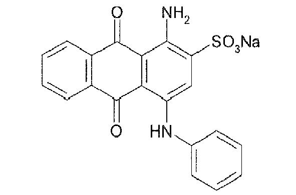 | 5.61± 0.47 | 11.1± 3.0 | n.d. | n.d. | **9.83** ± 2.43 |
| **18** | 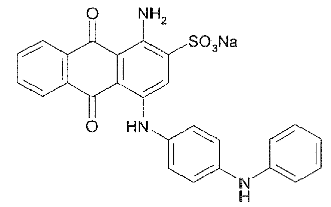 | n.d. | 30.0 ± 7.0 | >>3 | >>1 | **1.85** ± 0.57 |
| **19** | 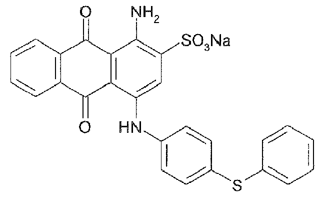 | n.d. | n.d. | n.d. | n.d. | **0.0884** ± 0.525 |
| **20** | 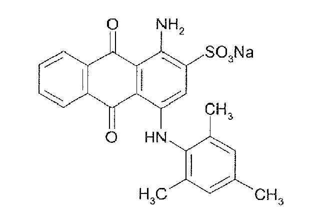 | n.d. | n.d. | n.d. | n.d. | **>10** |
| **21** | 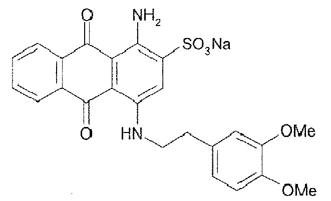 | n.d. | n.d. | >30 | >100 | **>10** |
| **22** | 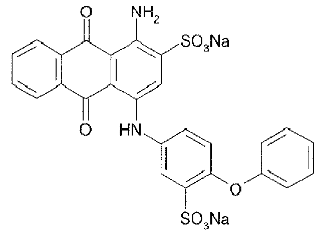 | n.d. | n.d. | n.d. | n.d. | **0.063** ± 0.009 |
| **23** | 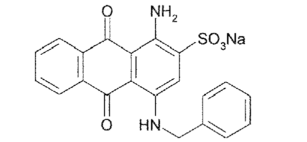 | n.d. | n.d. | n.d. | n.d. | **>>10** |
| **24** | 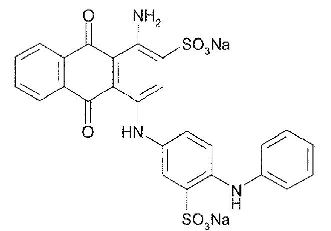 | n.d. | n.d. | n.d. | n.d. | **0.0249** ± 0.0032 |
| **25** | 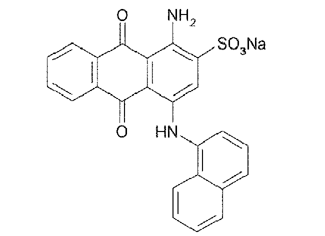 | 6.67 ± 0.32 | 10.7 ± 1.0 | 10.1 ± 1.4 | 26.7 | **> 10** |
| **26** | 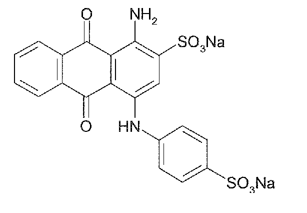 | n.d. | n.d. | n.d. | n.d. | **3.13** ± 0.39 |
| **27** | 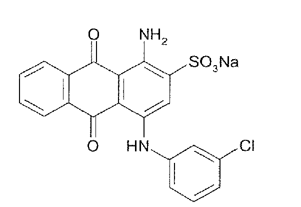 | 24.5 ± 10.4 | > 100 | >> 3 | >> 100 | **6.76** ± 2.07 |
| **28** | 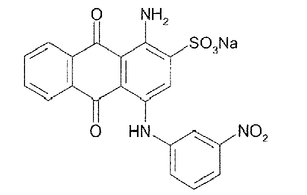 | 7.52 ± 0.82 | >100 | n.d. | n.d. | **>>10** |
| **29** | 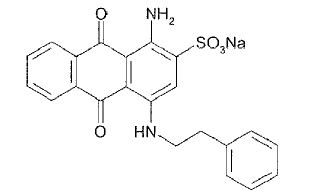 | n.d. | n.d. | n.d. | n.d. | **>>10** |
| **30** | 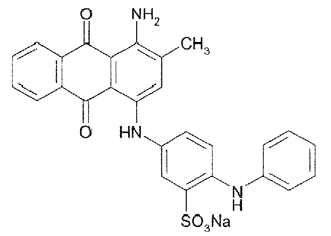 | n.d. | n.d. | n.d. | n.d. | **0.541** |
| **31** | 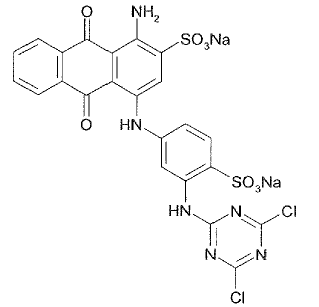 | n.d. | n.d. | n.d. | n.d. | **17.1** ± 7.8 |
| **32** | 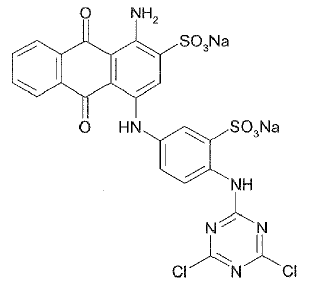 | n.d. | n.d. | n.d. | n.d. | **2.45** ± 1.00 |
| **33** | 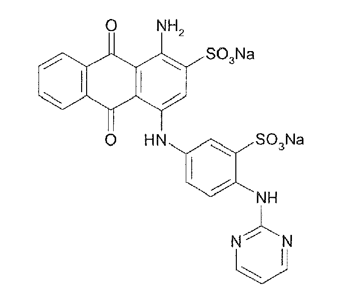 | n.d. | n.d. | 9.04 ± 1.20 | 28.6± 2.7 | **0.0507** ± 0.0160 |
| **34** | 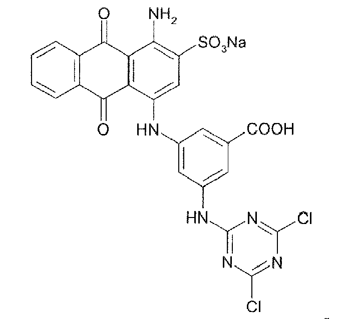 | n.d. | n.d. | n.d. | n.d. | **3.76** ± 1.03 |
| **35** | 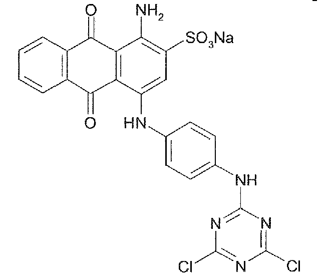 | n.d. | n.d. | n.d. | n.d. | **1.90** ± 0.24 |
| **36** | 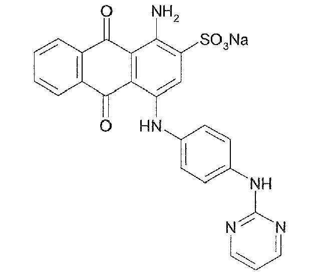 | n.d. | n.d. | n.d. | n.d. | ca.10 |
| **37** | 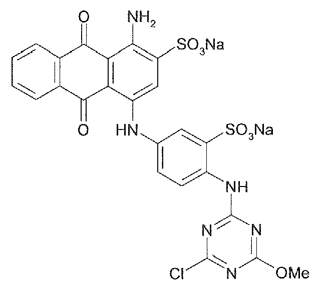 | n.d. | n.d. | n.d. | n.d. | **0.66** ± 0.12 |
| **38** | 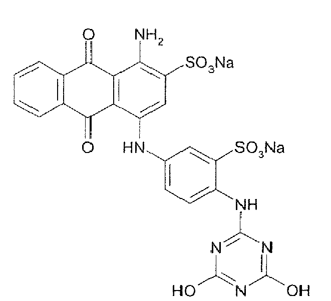 | n.d. | n.d. | n.d. | n.d. | **ca. 10** |
| **39** | 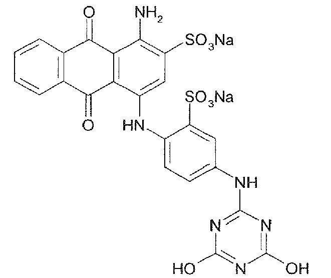 | n.d. | n.d. | n.d. | n.d. | **>>10** |
| **40** | 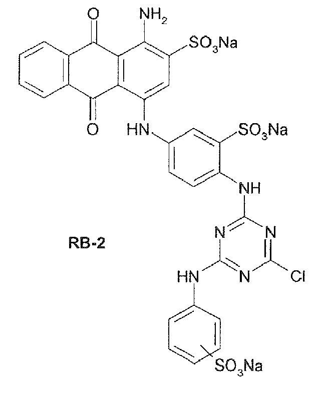 | 12.0± 5.7 | 2.90 | 9.79± 3.91 | 4.34± 0.89 | **0.68** ± 0.26 |

## Claims

1. A compound of Formula I: 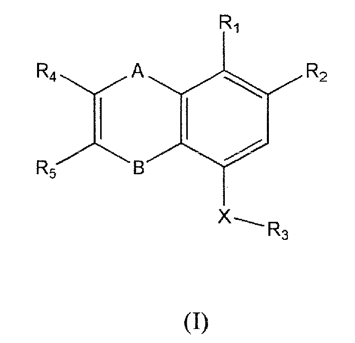 wherein:
A and B are independently selected from the group consisting of CH₂, O, S, NH, C=O, C=NH, C=S, or C=N-OH;
X is selected from the group consisting of NH, O, S, C=O, and CH₂;
R¹ and R² are independently selected from the group consisting of hydrogen, unsubstituted or substituted C₁-C₁₀ alkyl, unsubstituted or substituted C₁-C₁₀ alkenyl, unsubstituted or substituted C₁-C₁₀ alkynyl, unsubstituted or substituted C₃-C₈ cycloalkyl, unsubstituted or substituted C₁-C₁₀ alkoxy, unsubstituted or substituted C₃-C₈ cycloalkoxy, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -C(O)R, -C(O)OR , -C(O)NRR', -NRR', - S(O)₂R, -S(O)₂OR, and -S(O)₂NRR';
R³ is selected from the group consisting of hydrogen, unsubstituted or substituted C₁-C₁₀ alkyl, unsubstituted or substituted C₁-C₁₀ alkenyl, unsubstituted or substituted C₁-C₁₀ alkynyl, unsubstituted or substituted C₃-C₈ cycloalkyl, unsubstituted or substituted C₁-C₁₀ alkoxy, unsubstituted or substituted C₃-C₈ cycloalkoxy, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, alkylaryl, alkylheteroaryl, an unsubstituted or substituted 7 to 12-membered bicyclic alkyl or heterocyclic ring wherein 1 to 3 ring members are independently nitrogen, oxygen or sulfur, an unsubstituted or substituted 10 to 16-membered tricyclic alkyl or heterocyclic ring wherein 1 to 3 ring members are independently nitrogen, oxygen or sulfur, 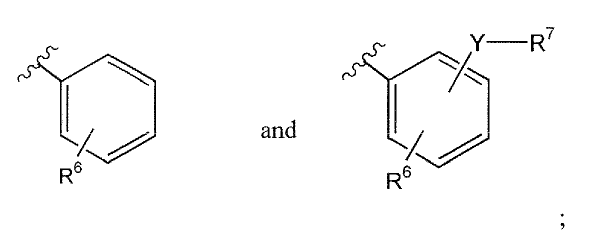
R⁴ and R⁵ are hydrogen, or, combined, R⁴ and R⁵ may form, together with the carbon atoms to which they are attached, a group selected from the group consisting of unsubstituted or substituted C₃-C₈ cycloalkyl, unsubstituted or substituted C₆-C₁₄ aryl, unsubstituted or substituted 5- to 10-membered heteroaryl wherein 1 to 4 ring atoms are independently selected from nitrogen, oxygen or sulfur, or unsubstituted or substituted 5-to 10-membered heteroalicyclic ring wherein 1 to 3 ring atoms are independently nitrogen, oxygen or sulfur;
R⁶ represents one to four substituents independently selected from the group consisting of hydrogen, halogen, unsubstituted or substituted C₁-C₄ alkyl, alkenyl or alkynyl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -NRR', -NO₂, unsubstituted or substituted C₁-C₄ alkoxy, -C(O)R , -C(O)OR , -C(O)NRR', - S(O)₂R, -S(O)₂OR, and -S(O)₂NRR';
Y is selected from the group consisting of hydrogen, halogen, NH, O, S, CH₂ , CH₂CH₂, C(O), C(O)O, S(O)₂O, or unsubstituted C₁-C₄ alkoxy, wherein when Y is hydrogen, halogen or alkoxy R⁷ is missing;
R⁷ is selected from the group consisting of hydrogen, halogen, -OR, unsubstituted or substituted C₁-C₁₀ alkyl, alkenyl or alkynyl, unsubstituted or substituted C₁-C₁₀ alkoxy, unsubstituted or substituted C₃-C₈ cycloalkoxy, unsubstituted or substituted C₃-C₈ cycloalkyl, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic wherein 1 to 3 ring atoms are independently nitrogen, oxygen or sulfur, -C(O)R , -C(O)OR , -C(O)NRR', -NRR', - S(O)₂R, -S(O)₂OR, -S(O)₂NRR', and - P(O)RR'; and
R and R' are independently selected from the group consisting of hydrogen and unsubstituted C₁-C₄alkyl;
or a pharmaceutically acceptable salt or prodrug thereof.

2. A compound of Formula II: 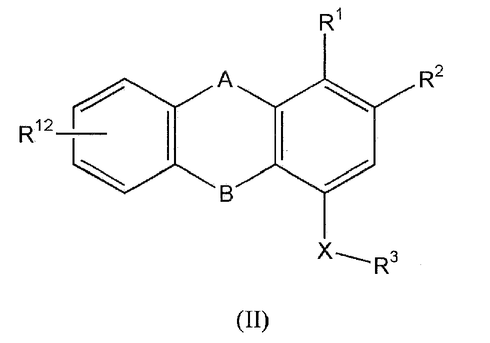 wherein:
A and B are independently selected from the group consisting of CH₂, O, S, NH, C=O, C=NH, C=S, or C=N-OH;
X is selected from the group consisting ofNH, O, S, C=O, and CH₂;
R¹ and R² are independently selected from the group consisting of hydrogen, unsubstituted or substituted C₁-C₁₀ alkyl, unsubstituted or substituted C₁-C₁₀ alkenyl, unsubstituted or substituted C₁-C₁₀ alkynyl, unsubstituted or substituted C₃-C₈ cycloalkyl, unsubstituted or substituted C₁-C₁₀ alkoxy, unsubstituted or substituted C₃-C₈ cycloalkoxy, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -C(O)R , -C(O)OR , -C(O)NRR', -NRR', - S(O)₂ -S(O)₂OR, and -S(O)₂NRR';
R³ is selected from the group consisting of hydrogen, unsubstituted or substituted C₁-C₁₀ alkyl, unsubstituted or substituted C₁-C₁₀ alkenyl, unsubstituted or substituted C₁-C₁₀ alkynyl, unsubstituted or substituted C₃-C₈ cycloalkyl, unsubstituted or substituted C₁-C₁₀ alkoxy, unsubstituted or substituted C₃-C₈ cycloalkoxy, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, alkylaryl, alkylheteroaryl, an unsubstituted or substituted 7 to 12-membered bicyclic alkyl or heterocyclic ring wherein 1 to 3 ring members are independently nitrogen, oxygen or sulfur, an unsubstituted or substituted 10 to 16-membered tricyclic alkyl or heterocyclic ring wherein 1 to 3 ring members are independently nitrogen, oxygen or sulfur, 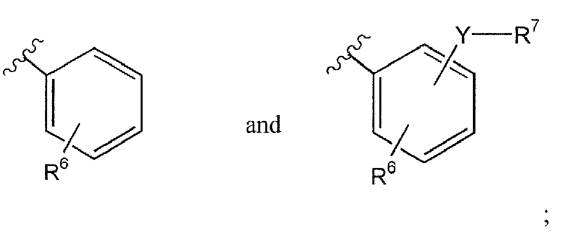
R⁶ represents one to four substituents independently selected from the group consisting of hydrogen, halogen, unsubstituted or substituted C₁-C₄ alkyl, alkenyl or alkynyl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -NRR', -NO₂ unsubstituted or substituted C₁-C₄ alkoxy, -C(O)R, -C(O)OR , -C(O)NRR', - S(O)₂R, -S(O)₂OR, and -S(O)₂NRR';
Y is selected from the group consisting of hydrogen, halogen, NH, O, S, CH₂ , CH₂CH₂, C(O), C(O)O, S(O)₂O, or unsubstituted C₁-C₄ alkoxy, wherein when Y is hydrogen, halogen or alkoxy R⁷ is missing;
R⁷ is selected from the group consisting of hydrogen, halogen, -OR, unsubstituted or substituted C₁-C₁₀ alkyl, alkenyl or alkynyl, unsubstituted or substituted C₁-C₁₀ alkoxy, unsubstituted or substituted C₃-C₈cycloalkoxy, unsubstituted or substituted C₃-C₈ cycloalkyl, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic wherein 1 to 3 ring atoms are independently nitrogen, oxygen or sulfur, - C(O)R, -C(O)OR , -C(O)NRR', -NRR', - S(O)₂R, -S(O)₂OR, -S(O)₂NRR', and -P(O)RR';
R¹² represents one to four substituents independently selected from the group consisting of hydrogen, halogen, unsubstituted or substituted C₁-C₄ alkyl, alkenyl or alkynyl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -NRR', -NO₂, unsubstituted or substituted C₁-C₄ alkoxy, -C(O)R , -C(O)OR , -C(O)NRR', - S(O)₂R, -S(O)₂OR, and -S(O)₂NRR'; and
R and R' are independently selected from the group consisting of hydrogen and unsubstituted C₁-C₄ alkyl;
or a pharmaceutically acceptable salt or prodrug thereof.

3. The compound of claim 1 or 2, wherein X is NH.

4. A compound of Formula III: 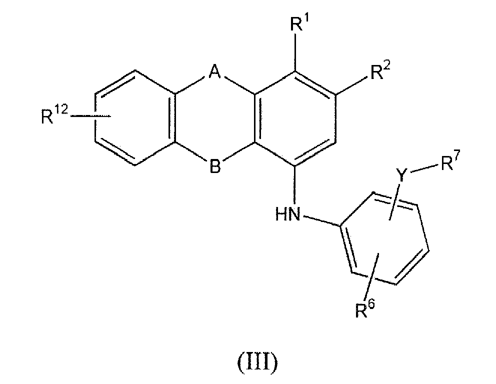 wherein:
A and B are independently selected from the group consisting of CH₂, O, S, NH, C=O, C=NH, C=S, or C=N-OH;
R¹ and R² are independently selected from the group consisting of hydrogen, unsubstituted or substituted C₁-C₁₀ alkyl, unsubstituted or substituted C₁-C₁₀ alkenyl, unsubstituted or substituted C₁-C₁₀ alkynyl, unsubstituted or substituted C₃-C₈ cycloalkyl, unsubstituted or substituted C₁-C₁₀ alkoxy, unsubstituted or substituted C₃-C₈ cycloalkoxy, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -C(O)R , -C(O)OR , - C(O)NRR', -NRR', - S(O)₂R, -S(O)₂OR, and -S(O)₂NRR';
R⁶ represents one to four substituents independently selected from the group consisting of hydrogen, halogen, unsubstituted or substituted C₁-C₄ alkyl, alkenyl or alkynyl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -NRR', -NO₂, unsubstituted or substituted C₁-C₄ alkoxy, -C(O)R, -C(O)OR , -C(O)NRR', - S(O)₂R, -S(O)₂OR, and -S(O)₂NRR';
Y is selected from the group consisting of hydrogen, halogen, NH, O, S, CH₂ , CH₂CH₂, C(O), C(O)O, S(O)₂O, or unsubstituted C₁-C₄ alkoxy, wherein when Y is hydrogen, halogen or alkoxy R⁷ is missing;
R⁷ is selected from the group consisting of hydrogen, halogen, -OR, unsubstituted or substituted C₁-C₁₀ alkyl, alkenyl or alkynyl, unsubstituted or substituted C₁-C₁₀ alkoxy, unsubstituted or substituted C₃-C₈ cycloalkoxy, unsubstituted or substituted C₃-C₈ cycloalkyl, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic wherein 1 to 3 ring atoms are independently nitrogen, oxygen or sulfur, - C(O)R, -C(O)OR , -C(O)NRR', -NRR', - S(O)₂R, -S(O)₂OR, -S(O)₂NRR' and -P(O)RR';
R¹² represents one to four subsituents independently selected from the group consisting of hydrogen, halogen, unsubstituted or substituted C₁-C₄ alkyl, alkenyl or alkynyl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -NRR', -NO₂, unsubstituted or substituted C₁-C₄ alkoxy, -C(O)R, -C(O)OR , -C(O)NRR', - S(O)₂R, -S(O)₂OR, and -S(O)₂NRR'; and
R and R' are independently selected from the group consisting of hydrogen and unsubstituted C₁-C₄ alkyl;
or a pharmaceutically acceptable salt or prodrug thereof.

5. The compound of claim 4, wherein R⁷ is selected from the group consisting of unsubstituted or substituted C₆-C₁₄ aryl or an unsubstituted or substituted 5- to 10-membered heteroaryl wherein 1 to 4 ring atoms are independently selected from nitrogen, oxygen or sulfur.

6. The compound of claim 5, wherein R⁷ is unsubstituted or substituted C₆ aryl or an unsubstituted or substituted 6-membered Heteroaryl wherein 1 to 3 ring atoms are nitrogen.

7. A compound of Formula IV: 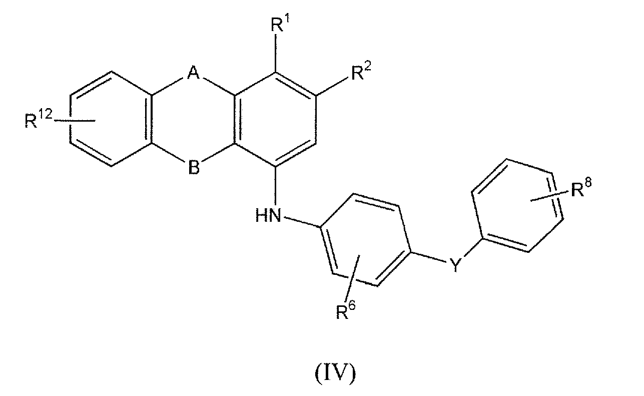 wherein:
A and B are independently selected from the group consisting of CH₂, O, S, NH, C=O, C=NH, C=S, or C=N-OH;
R¹ and R² are independently selected from the group consisting of hydrogen, unsubstituted or substituted C₁-C₁₀ alkyl, unsubstituted or substituted C₁-C₁₀ alkenyl, unsubstituted or substituted C₁-C₁₀ alkynyl, unsubstituted or substituted C₃-C₈ cycloalkyl, unsubstituted or substituted C₁-C₁₀ alkoxy, unsubstituted or substituted C₃-C₈ cycloalkoxy, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -C(O)R , -C(O)OR , - C(O)NRR', -NRR', - S(O)₂R, -S(O)₂ and -S(O)₂NRR';
R⁶ represents one to four substituents independently selected from the group consisting of hydrogen, halogen, unsubstituted or substituted C₁-C₄ alkyl, alkenyl or alkynyl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -NRR', -NO₂ unsubstituted or substituted C₁-C₄ alkoxy, -C(O)R, -C(O)OR , -C(O)NRR', - S(O)₂R, -S(O)₂OR, and -S(O)₂NRR';
Y is selected from the group consisting of NH, O, S, CH₂, CH₂CH₂, C(O), C(O)O, or S(O)₂O.
R⁸ represents one to five substituents selected from the group consisting of C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NRR' ;
R¹² represents one to four substituents independently selected from the group consisting of hydrogen, halogen, unsubstituted or substituted C₁-C₄ alkyl, alkenyl or alkynyl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -NRR', -NO₂, unsubstituted or substituted C₁-C₄ alkoxy, -C(O)R , -C(O)OR , -C(O)NRR', - S(O)₂R, -S(O)₂OR, and -S(O)₂NRR'; and
R and R' are independently selected from the group consisting of hydrogen and unsubstituted C₁-C₄ alkyl;
or a pharmaceutically acceptable salt or prodrug thereof.

8. The compound of claim 7, wherein R⁸ represents one to three substituents selected from the group consisting of halo, unsubstituted C₁-C₄ alkyl or alkoxy, carboxy, sulfonyl, amino and hydroxy.

9. The compound of any one of claims 4-8, wherein Y is NH.

10. The compound of any one of claims 4-9, wherein R⁶ represents one to four substituents selected from the group consisting of hydrogen, -S(O)₂ -C(O)OR, tetrazolyl, sulfonyl, carboxy, amino, halogen, C₁-C₄ alkoxy, C₁-C₄ alkyl, hydroxy, and nitro.

11. The compound of any one of claims 1-10, wherein R¹² represents one to four substituents selected from the group consisting of hydrogen, halo, amino, unsubstituted C₁-C₄ alkyl or alkoxy, or hydroxy.

12. The compound of any one of claims 1-11, wherein at least one of A and B is C=O.

13. The compound of any one of claims 1-11, wherein both A and B are C=O.

14. The compound of any one of claims 1-13, wherein R¹ is amino.

15. The compound of any one of claims 1-14, wherein R² is tetrazolyl, -S(O)₂OR or -C(O)OR.

16. The compound of any one of claims 1-15, wherein the compound is selected from the group consisting of 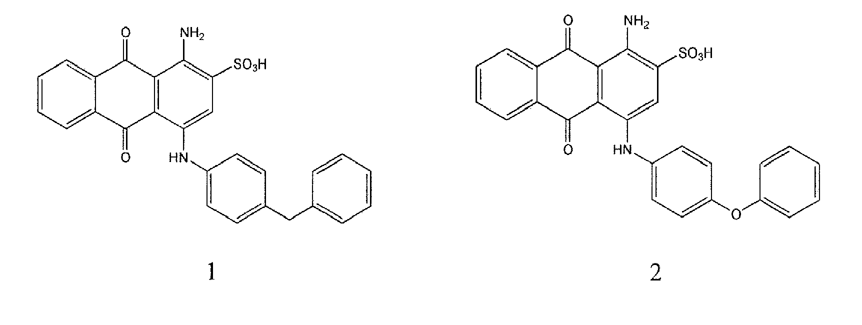 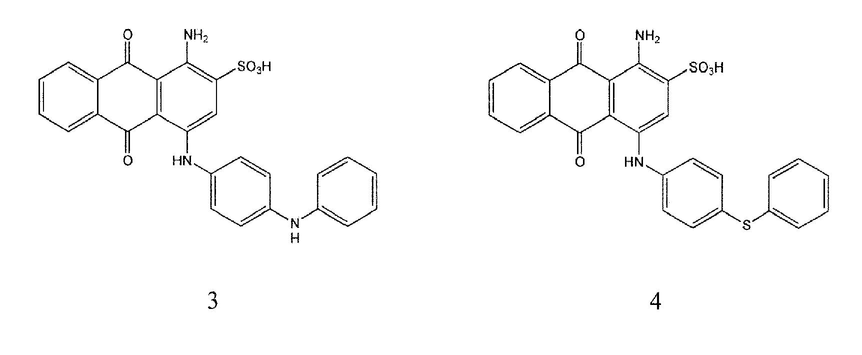 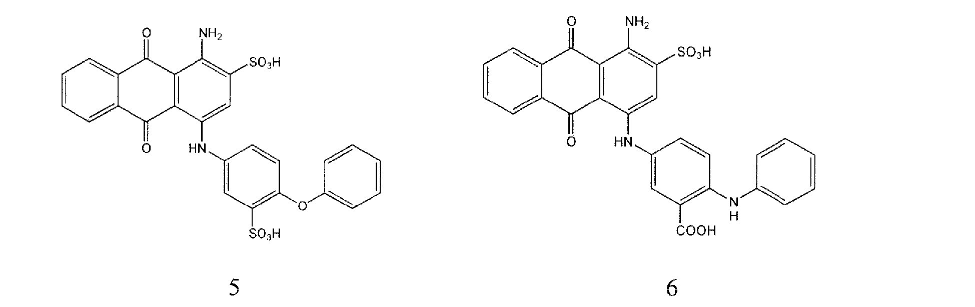 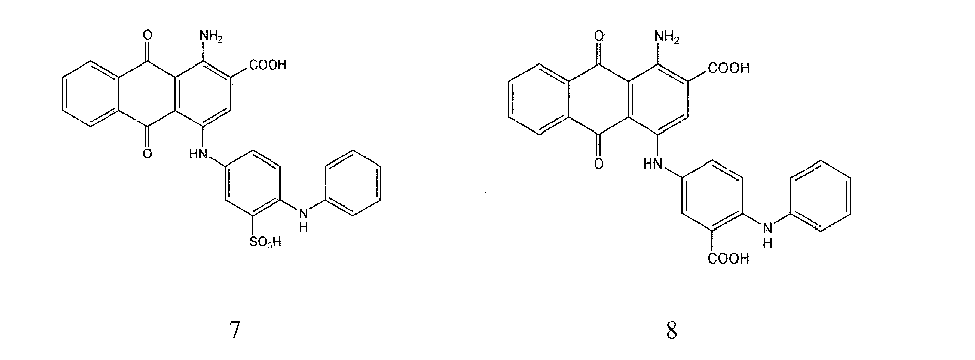 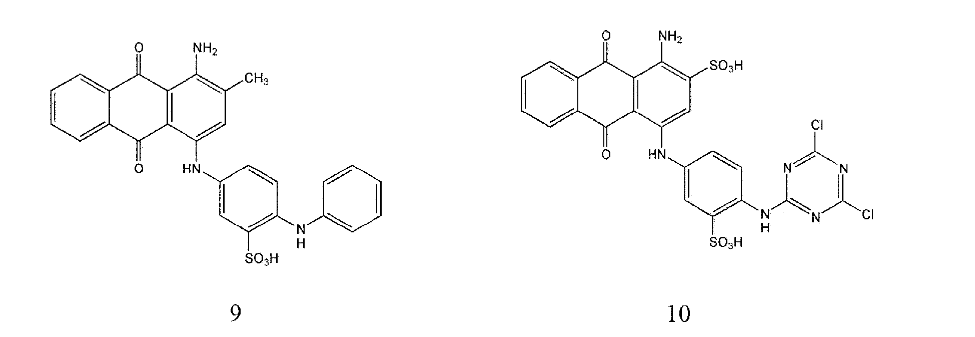 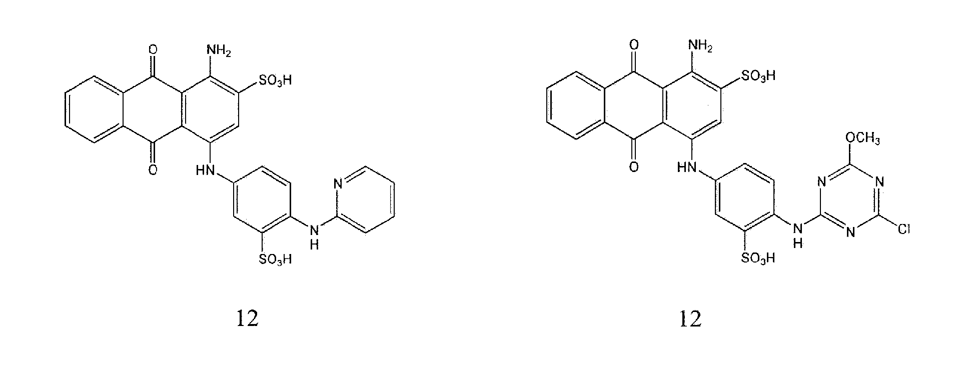 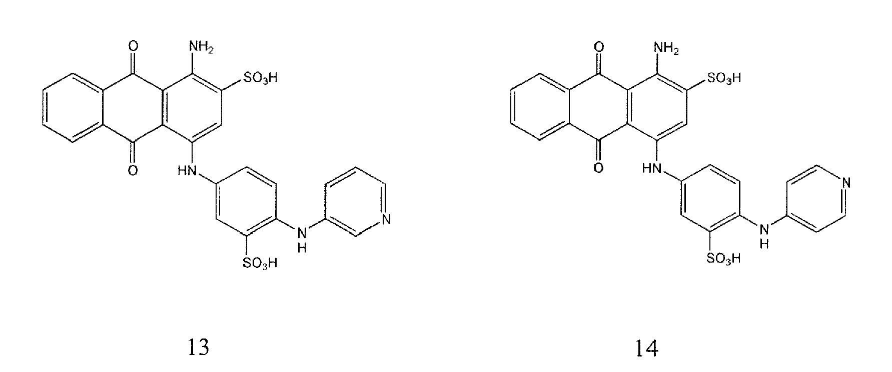 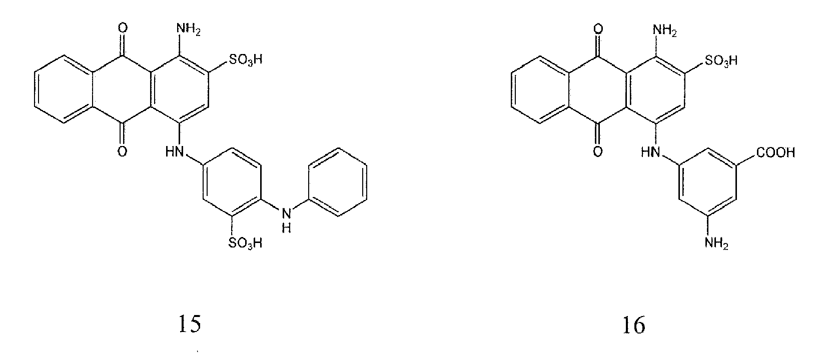 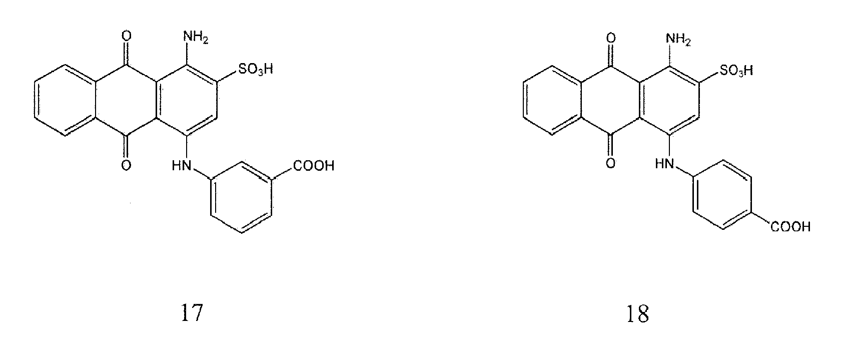 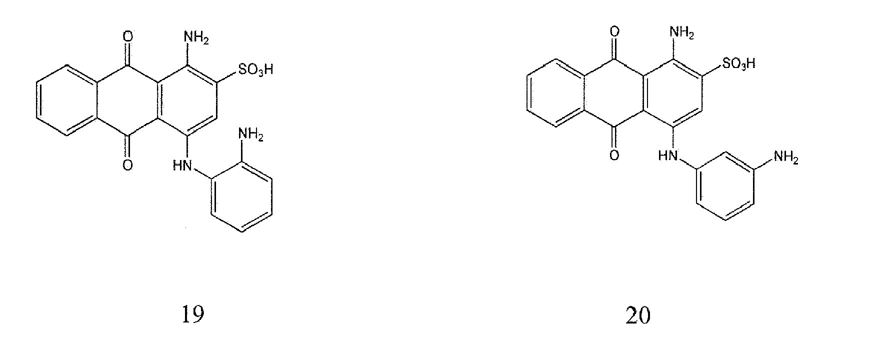 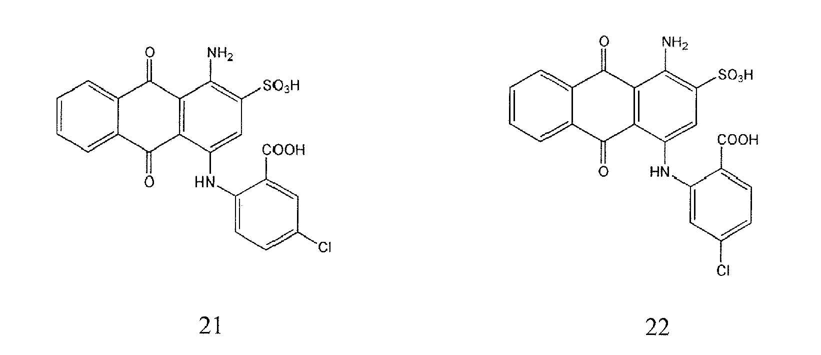 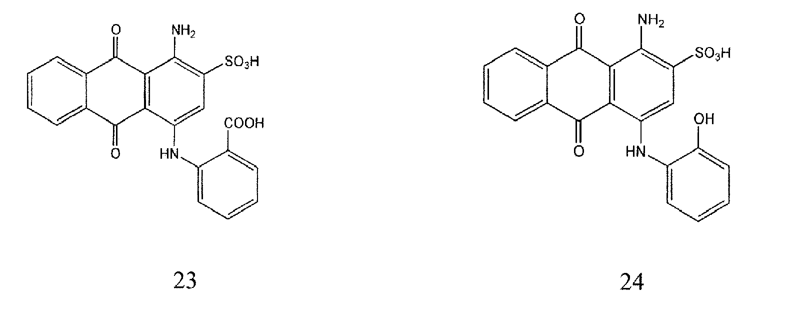 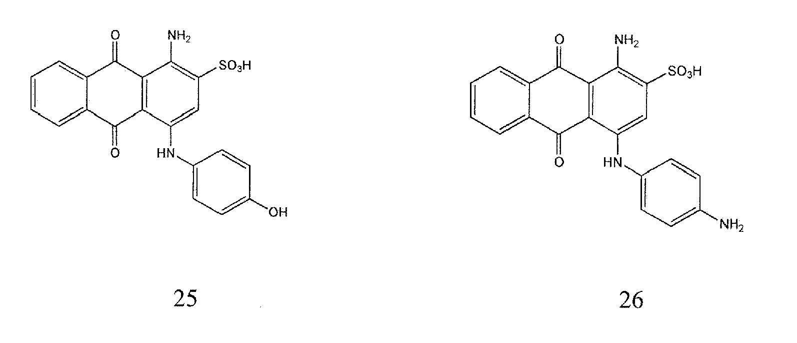 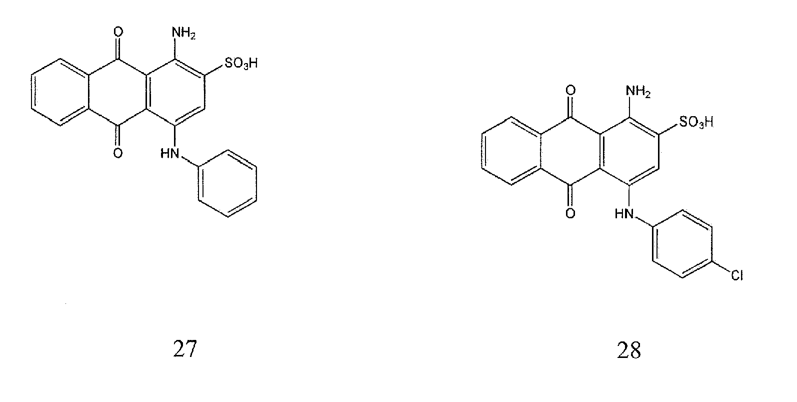 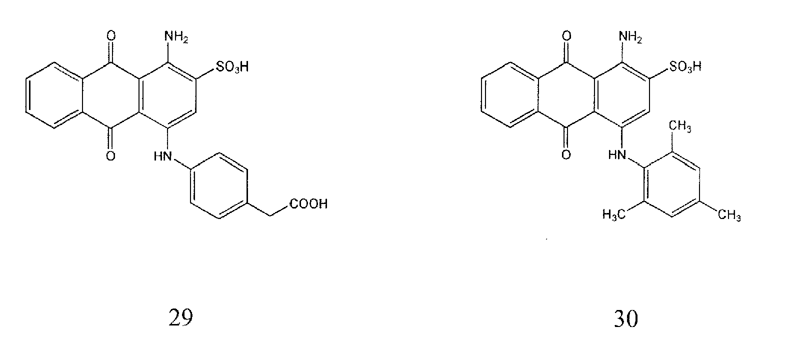 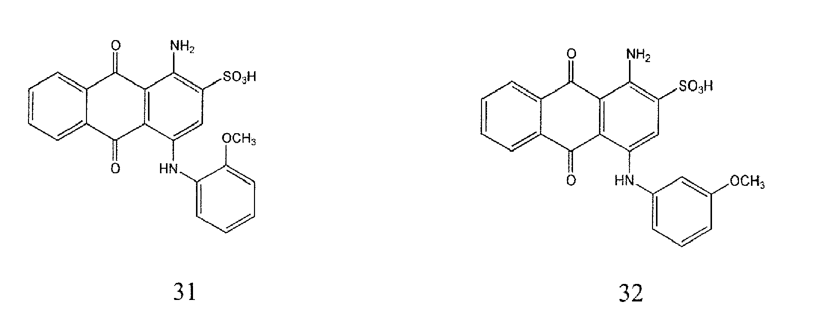 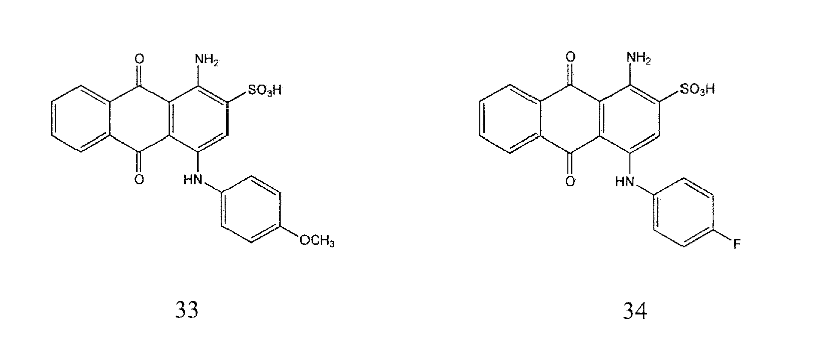 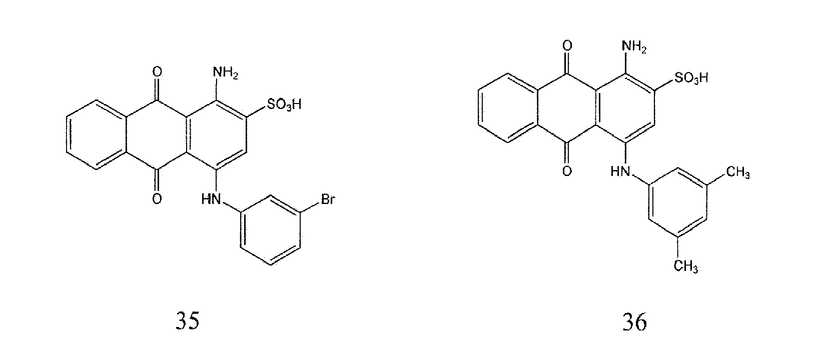 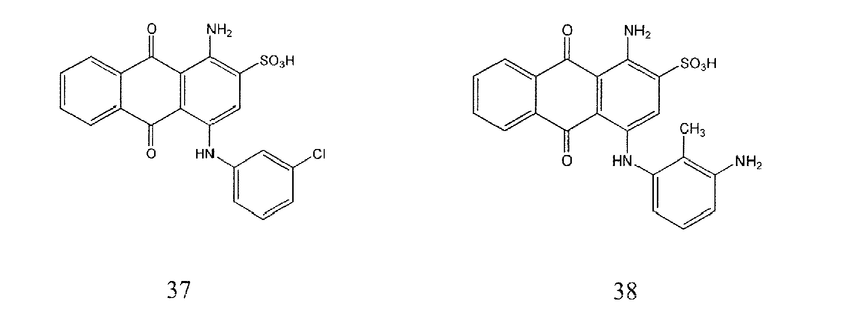 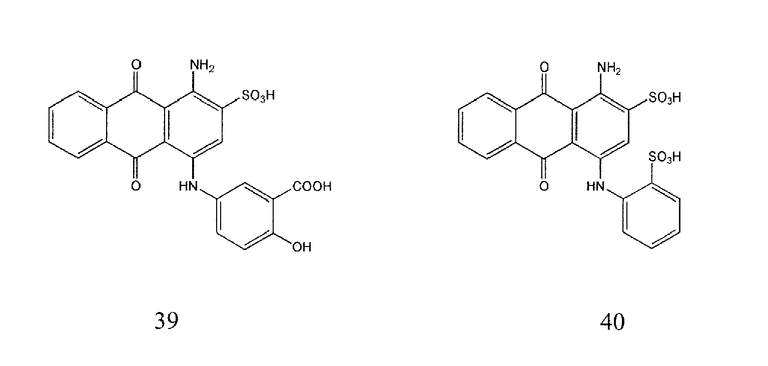 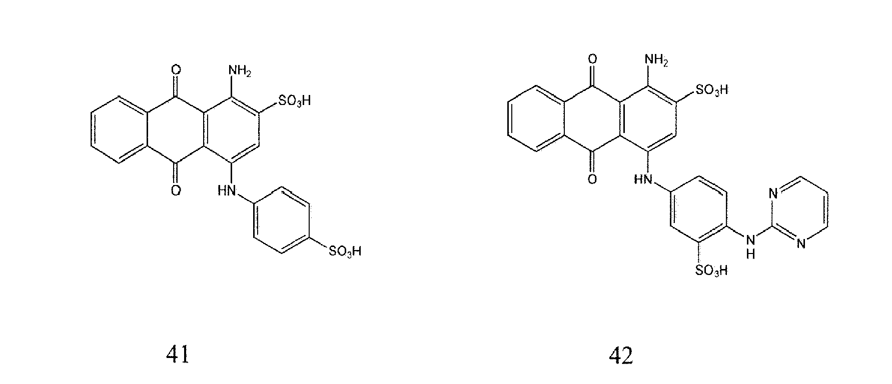 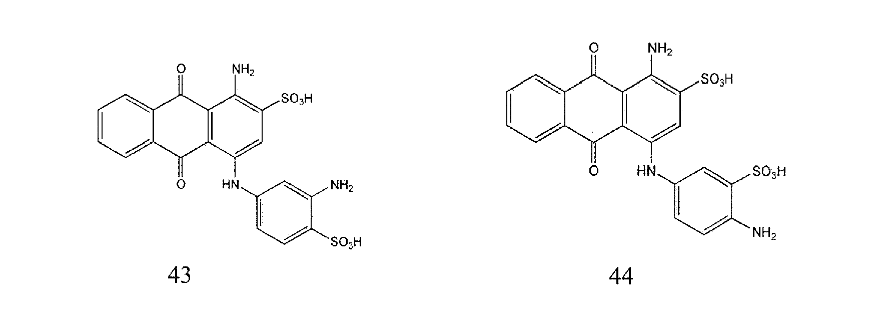 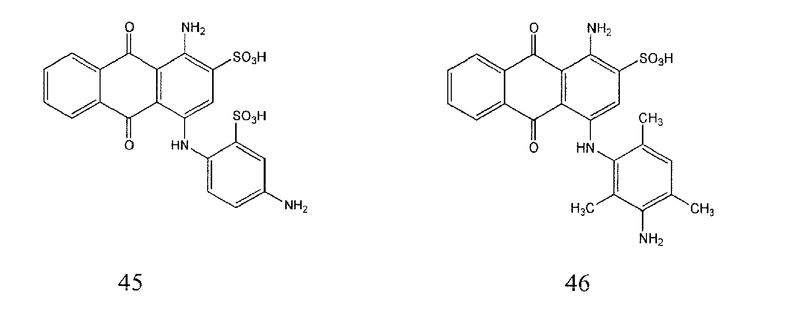 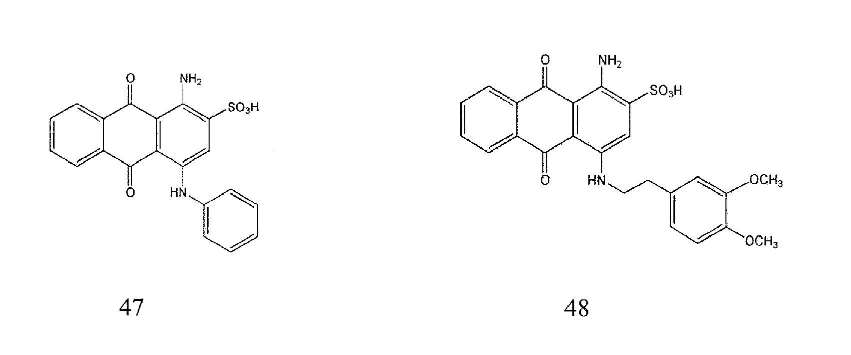 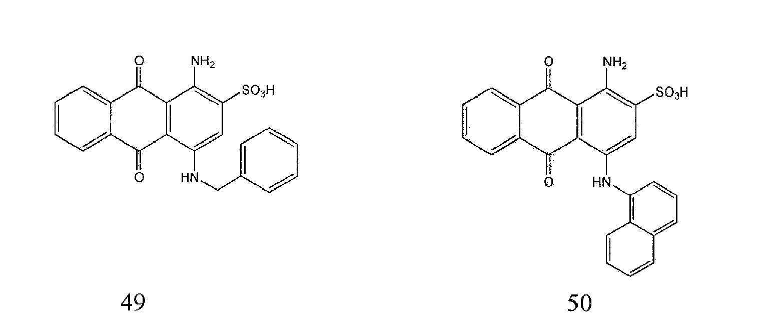 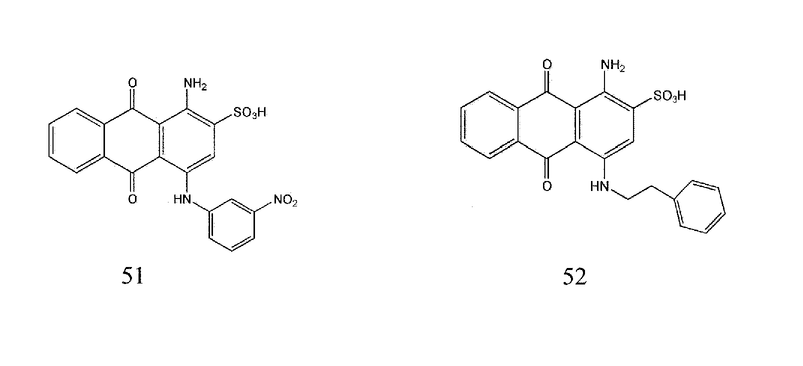 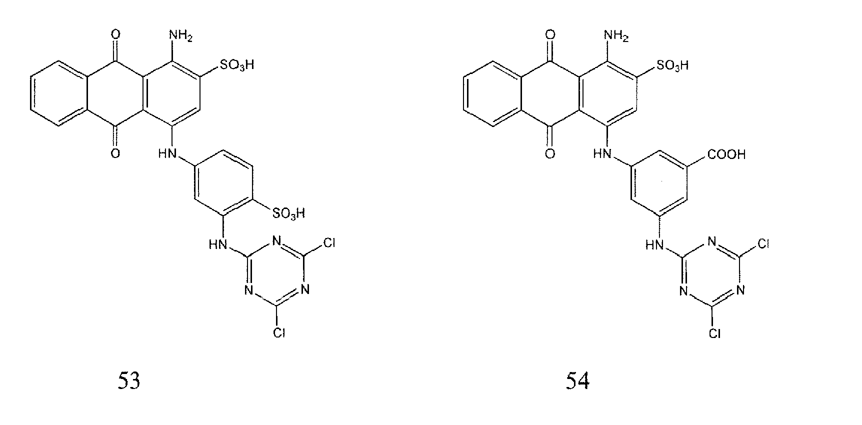 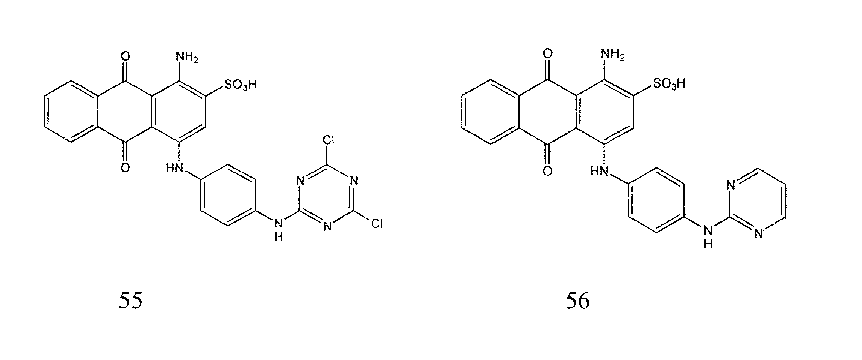 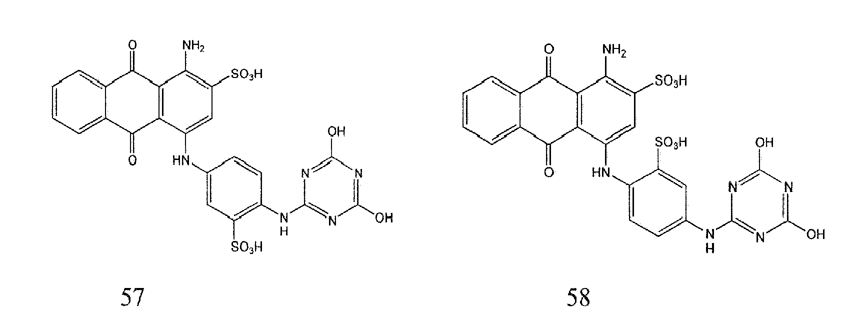 or pharmaceutically acceptable salts or prodrugs thereof.

17. A pharmaceutical composition comprising a compound or salt of any one of claims 1 to 16 and a pharmaceutically acceptable carrier or excipient.

18. The pharmaceutical composition of claim 17, further comprising an additional medicament or drug.

19. The use of a compound according to any of claims 1 to 16 for the manufacture of a pharmaceutical for inhibiting platelet aggregation.

20. The use of a compound according to any one of claims 1 to 16 for the manufacture of a pharmaceutical for the prevention, alleviation and/or treatment of a disease or disorder related to P2Y₁₂ receptor activity.

21. The use of claim 20, wherein the disease or disorder is selected from the group of stroke, myocardial infarction, myocardial ischemia, vascular diseases, coronary artery disease, peripheral artery disease, atherosclerosis, cerebrovascular disease, embolisms and CNS disorders.

22. The use of any one of claims 19-21, wherein said pharmaceutical is for the treatment of a human.

23. A method for antagonizing the P2Y₁₂ receptor function comprising the step of contacting cells expressing a P2Y₁₂ receptor with at least one compound according to any one of claims 1 to 16.

24. A method for the treatment or prevention of a P2Y₁₂ receptor related disease or disorder in a patient, comprising the administration of a pharmaceutically active amount of a compound according to any one of claims 1 to 16 to a patient in need thereof.

25. The method according to claim 24, wherein the patient is a human.

26. Method for the preparation of a compound according to any one of claims 1 to 16, wherein the method comprises reacting an aryl having the Formula VI 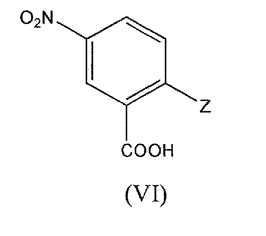 wherein Z is a leaving group selected from the group consisting of chlorine, bromine and iodine;
with an amine selected from the group of unsubstituted or substituted C₁-C₁₀ alkylamines, unsubstituted or substituted C₃-C₈ cycloalkylamines and unsubstituted or substituted C₆-C₁₄ arylamines without a solvent or catalyst under mild microwave reaction conditions to form the N-substituted 5-nitroanthranilic acid of formula V, 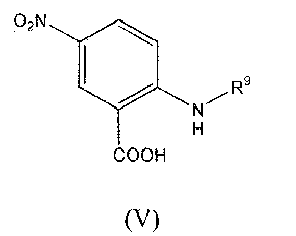 wherein R⁹ is selected from the group consisting of an unsubstituted or substituted C₁-C₁₀ alkyl, unsubstituted or substituted C₃-C₈ cycloalkyl or unsubstituted or substituted C₆-C₁₄ aryl group; and
wherein the N-substituted 5-nitroanthranilic acid of Formula V is used as an intermediate for the preparation of the compounds according to any one of claims 1 to 16.

27. Method for the preparation of a compound according to any one of claims 1 to 16, wherein the method comprises a microwave-assisted, copper-catalyzed Ullmann C-N coupling reaction according to reaction Scheme 1: 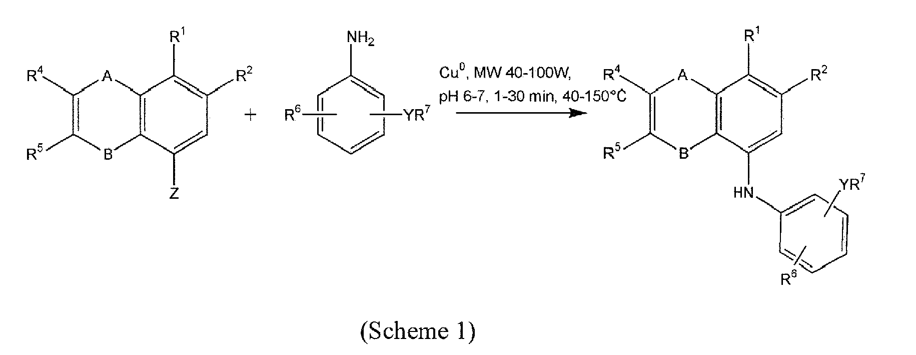 wherein
A and B are independently selected from the group consisting of CH₂, O, S, NH, C=O, C=S, C=NH or C=N-OH;
R¹ and R² are independently selected from the group consisting of hydrogen, unsubstituted or substituted C₁-C₁₀ alkyl, unsubstituted or substituted C₁-C₁₀ alkenyl, unsubstituted or substituted C₁-C₁₀ alkynyl, unsubstituted or substituted C₃-C₈ cycloalkyl, unsubstituted or substituted C₁-C₁₀ alkoxy, unsubstituted or substituted C₃-C₈ cycloalkoxy, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -C(O)R , -C(O)OR , - C(O)NRR', -NRR', - S(O)₂R, -S(O)₂OR, and -S(O0)₂NRR';
R⁴ and R⁵ are hydrogen, or, combined, R⁴ and R⁵ may form a C₃-C₈ cycloalkyl, C₆-C₁₄ aryl, 5- to 10-membered heteroaryl wherein 1 to 4 ring atoms are independently selected from nitrogen, oxygen or sulfur, or 5- to 10-membered heteroalicyclic ring wherein 1 to 3 ring atoms are independently nitrogen, oxygen or sulfur;
R⁶ represents one to four substituents independently selected from the group consisting of hydrogen, halogen, unsubstituted or substituted C₁-C₄ alkyl, alkenyl or alkynyl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -NRR', -NO₂, unsubstituted or substituted C₁-C₄ alkoxy, -C(O)R, -C(O)OR , -C(O)NRR', - S(O)₂R, -S(O)₂OR, and -S(O)₂NRR';
Y is selected from the group consisting of hydrogen, halogen, NH, O, S, CH₂ , CH₂CH₂, C(O), C(O)O, S(O)₂O, or unsubstituted C₁-C₄ alkoxy, wherein when Y is hydrogen, halogen or alkoxy R⁷ is missing;
R⁷ is selected from the group consisting of hydrogen, halogen, -OR, unsubstituted or substituted C₁-C₁₀ alkyl, alkenyl or alkynyl, unsubstituted or substituted C₁-C₁₀ alkoxy, unsubstituted or substituted C₃-C₈ cycloalkoxy, unsubstituted or substituted C₃-C₈ cycloalkyl, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic wherein 1 to 3 ring atoms are independently nitrogen, oxygen or sulfur, - C(O)R , -C(O)OR , -C(O)NRR', -NRR', - S(O)₂R, -S(O)₂OR, -S(O)₂NRR" , and -P(O)RR'; and
R and R' are independently selected from the group consisting of hydrogen and unsubstituted C₁-C₄ alkyl;
and Z is a leaving group selected from the group consisting of chlorine, bromine and iodine.
